# EUROPEAN PATENT APPLICATION

(11) **EP 4 516 796 A1**
(43) Date of publication of application: **05.03.2025**
(21) Application number: 23795322.9
(22) Date of filing: 24.04.2023
(51) Int. Cl.: C07F 5/02, A61K 31/69, A61P 35/00, A61P 37/06, A61P 7/00

(54) **BORIC ACID PROTEASOME INHIBITOR AND USE THEREOF**

(30) Priority: 29.04.2022 CN 202210468496
(71) Applicant: Artivila Biopharma, Wuxi, Jiangsu 214000 (CN)
(72) Inventor: LI, Xuke, Wuxi, Jiangsu 214000 (CN); ZHU, Zhendong, Wuxi, Jiangsu 214000 (CN); NIU, Deqiang, Wuxi, Jiangsu 214000 (CN)
(74) Representative: Zaccaro, Elisabetta
(86) International application number: PCT/CN2023/090235
(87) International publication number: WO 2023/207889

(57) **Abstract**

The present disclosure provides a boric acid proteasome inhibitor compound represented by formula I and use thereof as a proteasome inhibitor.

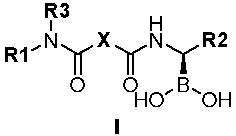

## Description

This application claims the priority of Chinese Patent Application No. 202210468496.X, filed with the China National Intellectual Property Administration on April 29, 2022, and titled with "BORIC ACID PROTEASOME INHIBITOR AND USE THEREOF", the disclosure of which is hereby incorporated by reference in its entirety.

### FIELD

The present disclosure relates to the technical field of pharmaceutical synthesis, and in particular to a boric acid proteasome inhibitor, use and preparation method thereof.

### BACKGROUND

In eukaryotic cells, protein degradation is mainly mediated by the ubiquitination pathway, wherein the protein to be degraded is attached to a polypeptide ubiquitin, and the 26S proteasome then degrades the ubiquitinated protein through the hydrolysis action of major three kinds of proteases. In cells, proteasome-mediated degradation plays a key role in many cellular functional processes such as antigen presentation by major histocompatibility complex (MHC) class I, apoptosis, growth regulation, NF-κB activation, antigen processing and pro-inflammatory signaling.

Proteasome is a multimeric protein composed of seven types of subunits, which exists in two forms in the cell, i.e., the widely expressed constitutive proteasome, and the immunoproteasome expressed mainly in the immune-inflammatory system. The two are different in subunit composition. The constitutive proteasome contains the chymotrypsin-like activity subunit (β5c), caspase-like activity subunit (β1c), and trypsin-like reactive subunit (β2c). In the immunoproteasome, the three may be induced by INF-γ to be converted into the corresponding immunological subunits (β5i/LMP7, β1i/LMP2, and β2i/MECL1, respectively). Thus, eukaryotic cells can have varying proportions of the two proteasomes.

The proteasome not only plays an important role in antigen presentation by MHC class I, but is also involved in a variety of pathological conditions, including malignant tumors, inflammatory diseases and autoimmune diseases. The prior art in this regard has demonstrated a relationship between the proteasome and the onset and development of these diseases above. In particular, the inhibition of proteasome indicates an important new strategy for cancer treatment. For example, King et al. described an important role of the ubiquitin-proteasome pathway in the regulation of the cell cycle, tumor growth and metastasis (Science, 274:1652-1659 (1996)).

Although the boric acid-based proteasome inhibitors in the prior art exhibit good activity against two subunits of β5c and β5i, they are unsatisfactory in terms of oral bioavailability. For example, the marketed proteasome inhibitor bortezomib cannot be used orally due to its low bioavailability.

Therefore, there is an urgent need for a proteasome inhibitor with good oral bioavailability. The present disclosure provides a series of novel boric acid-based proteasome inhibitors, and a number of compounds with good oral bioavailability. Such compounds are useful in the treatment of related diseases, particularly malignant and autoimmune diseases.

### SUMMARY

The present disclosure have found through extensive research that the following compounds having the structure represented by formula I or pharmaceutically acceptable salts thereof have excellent inhibitory effects on proteasome.

In view of this, in a first aspect, the present disclosure provides a compound having a structure represented by formula I or a pharmaceutically acceptable salt thereof: 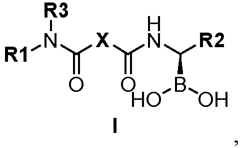 wherein,
X is selected from the group consisting of -C₁₋₄ alkyl-, -C₃₋₆ cycloalkyl-, -vinyl-, -ethynyl-, -NH-, -NH-NH-, and -heterocyclyl-, and X is optionally substituted with a substituent, wherein the substituent is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen;
R1 and R3 are each independently selected from the group consisting of hydrogen, substituted C₁₋₁₀ alkyl, C₃₋₆ cycloalkyl, aryl, heteroaryl, benzyl, heterocyclyl, and bridged ring, and the C₁₋₁₀ alkyl, C₃₋₆ cycloalkyl, aryl, heteroaryl, benzyl, heterocyclyl, and bridged ring group are optionally substituted with a substituen which is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen; and
R2 is selected from the group consisting of C₁₋₁₀ alkyl, benzyl, methyl biphenyl and heterocyclyl, and the C1-10 alkyl, benzyl, biphenylmethyl and heterocyclyl are optionally substituted with a substituent which is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen.

More specifically, in some embodiments, the compound represented by formula (I) has a structure represented by formula (II): 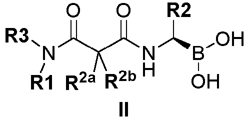
wherein, R1, R2, and R3 have the definitions as in claim 1; and
R^{2a} and R^{2b} are each independently selected from the group consisting of hydrogen, deuterium, C₁₋₁₀ alkyl, benzyl, isopropyl, cyclopropyl methyl, cyclobutyl methyl, cyclopentyl methyl, cyclohexyl methyl, heterocyclyl methyl, and halogen, or R^{2a} and R^{2b}, together with the carbon atom to which they are attached, form a 3- to 6- membered carbon ring.

In other embodiments, the compound represented by formula (I) has a structure represented by formula (III): 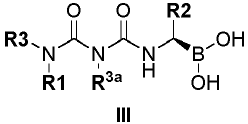
wherein, R1, R2, and R3 have the definitions as in claim 1; and
R^{3a} is selected from the group consisting of hydrogen, C₁₋₁₀ alkyl, benzyl, isopropyl, cyclopropyl methyl, cyclobutyl methyl, cyclopentyl methyl, cyclohexyl methyl, and heterocyclyl methyl.

In other embodiments, the compound represented by formula (I) has a structure represented by formula (IV): 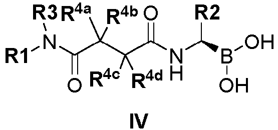
wherein, R1, R2, and R3 have the definitions as in claim 1; and
R^{4a}, R^{4b}, R^{4c} and R^{4d} are each independently selected from the group consisting of hydrogen, deuterium, C₁₋₁₀ alkyl, halogen, hydroxyl, and amino, wherein the amino is optionally substituted with C₁₋₄ alkyl or C₃₋₆ cycloalkyl.

In other embodiments, the compound represented by formula (I) has a structure represented by formula (V): 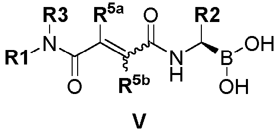
wherein, R1, R2, and R3 have the definitions as in claim 1; and
R^{5a} and R^{5b} are each independently selected from the group consisting of hydrogen, deuterium, C₁₋₄ alkyl, halogen and hydroxyl, and R^{5a} and R^{5b} are in either cis- or trans-substituted form to each other.

In other embodiments, the compound represented by formula (I) has a structure represented by formula (VI): 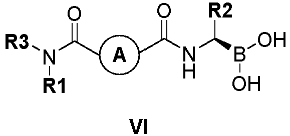
wherein, R1, R2, and R3 have the definitions as in claim 1; and
ring A is selected from the group consisting of -C₃₋₆ cycloalkyl- and -heterocyclyl-, wherein ring A is optionally substituted with a substituent which is selected from the group consisting of C₁₋₄ alkyl, C₁₋₁₀ alkoxy, C₃₋₆ cycloalkyl, cyano, hydroxyl and halogen.

In the present disclosure, for the compound represented by formula I or a compound represented by formula II-VI as described above, R1 and/or R3 can be preferably each selected from the group consisting of hydrogen,

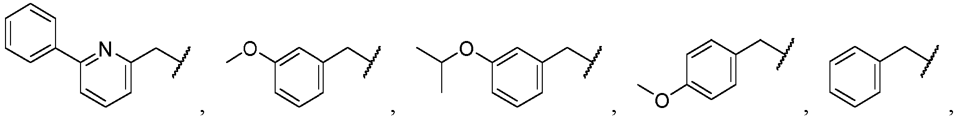

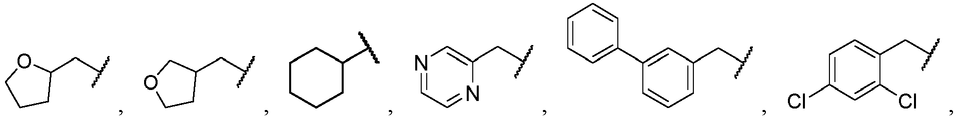

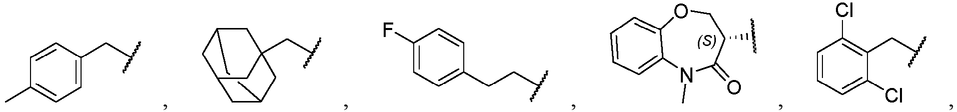

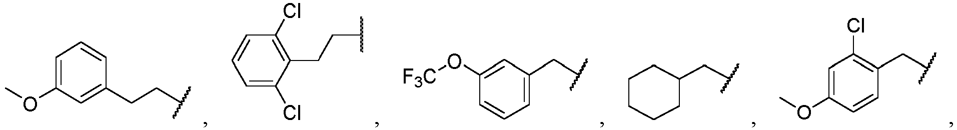

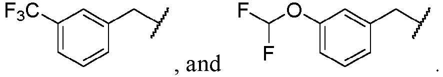

In the present disclosure, for the compound represented by formula I or a compound represented by formula II-VI as described above, R2 can be preferably selected from the group consisting of:

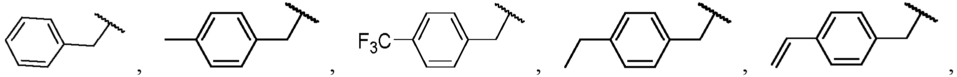

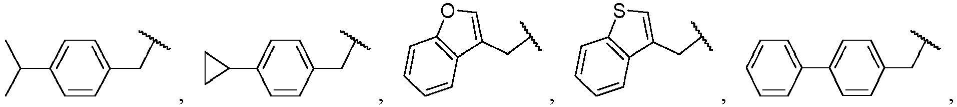

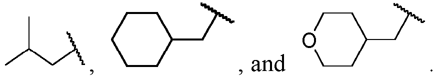

In the present disclosure, the following compounds or the pharmaceutically acceptable salts thereof are preferable:

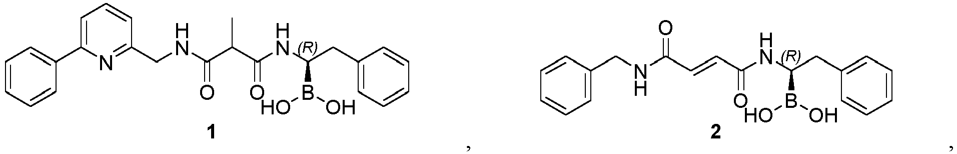

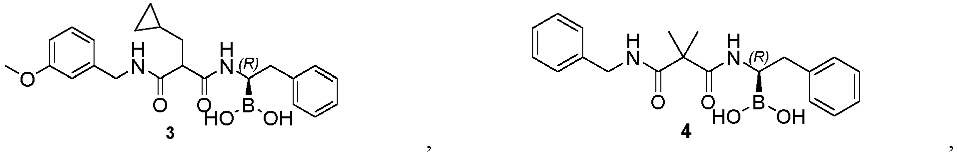

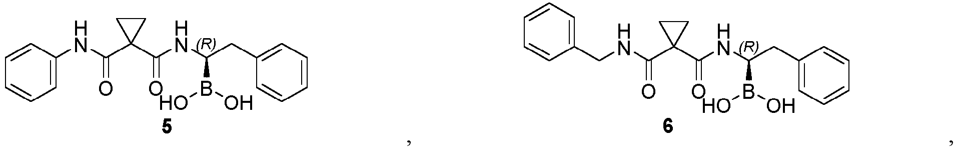

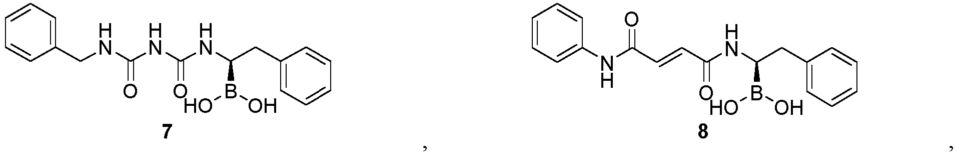

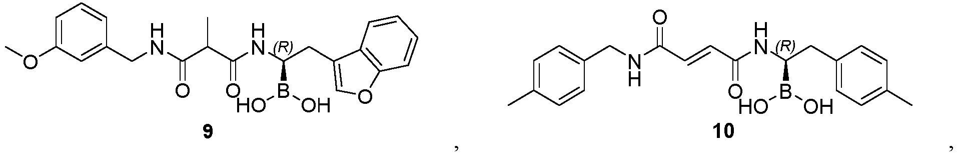

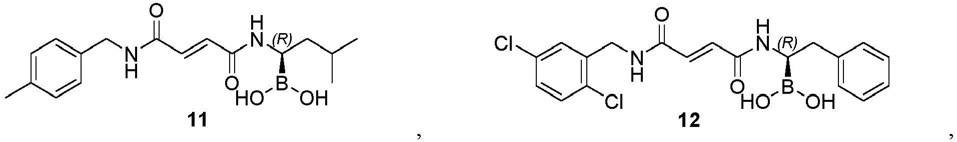

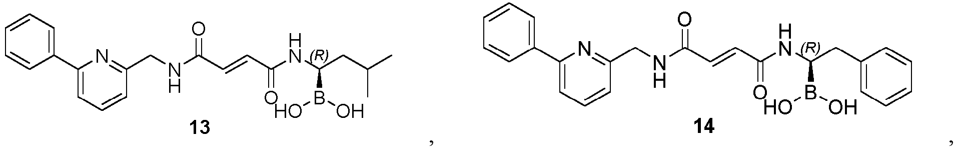

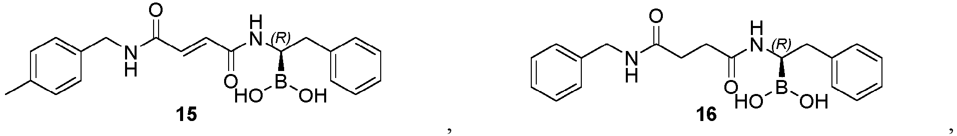

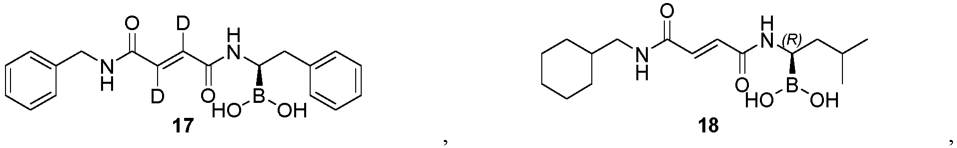

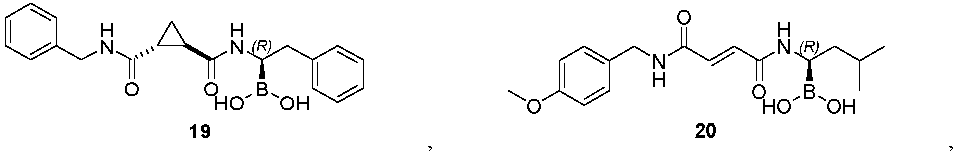

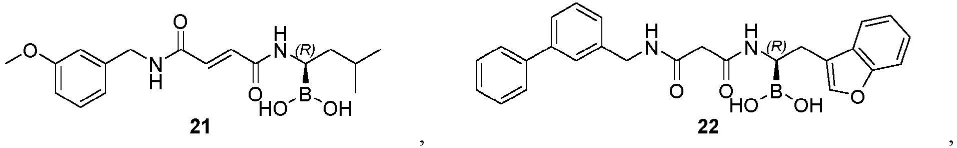

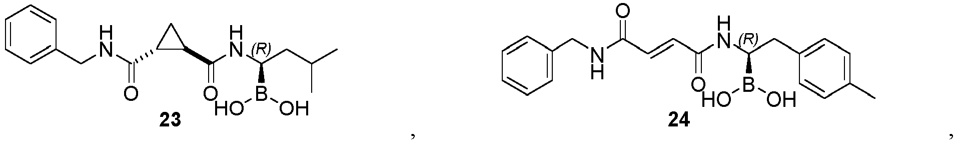

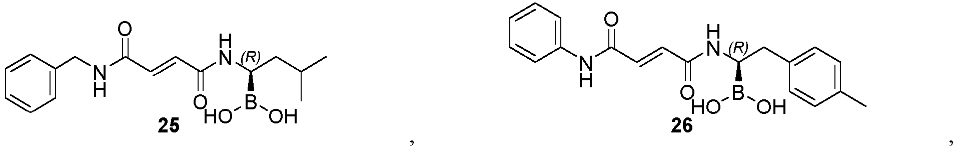

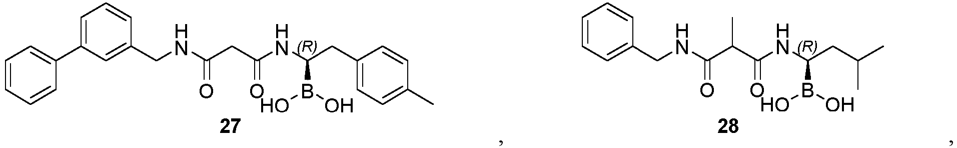

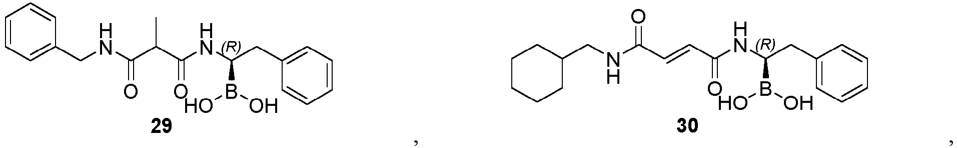

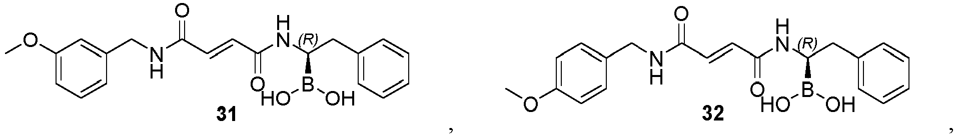

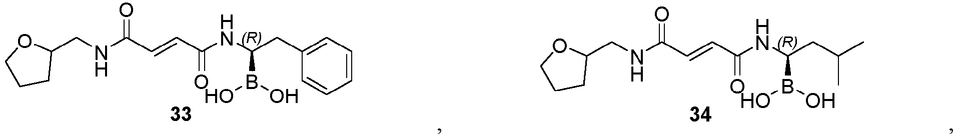

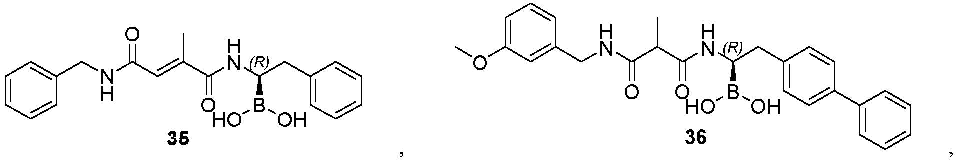

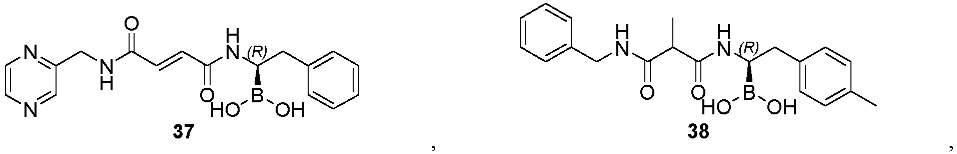

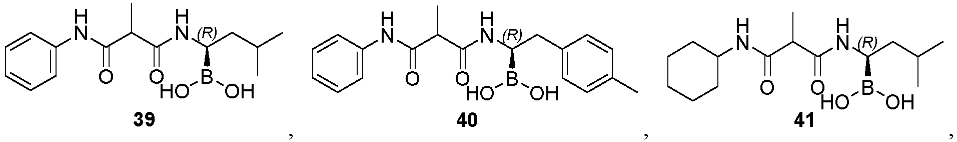

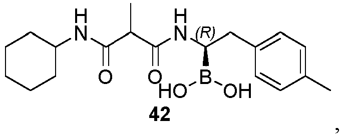

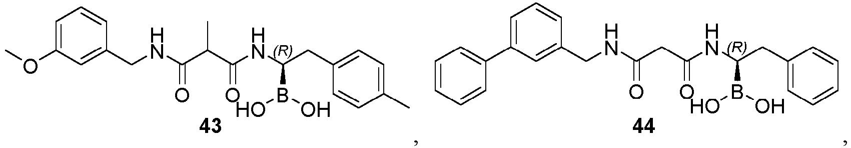

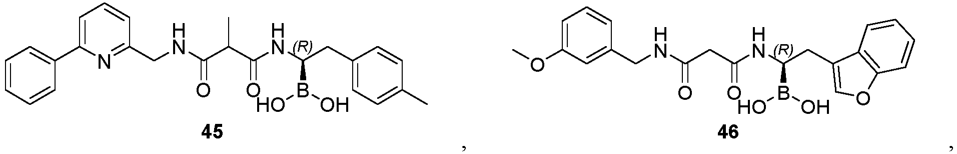

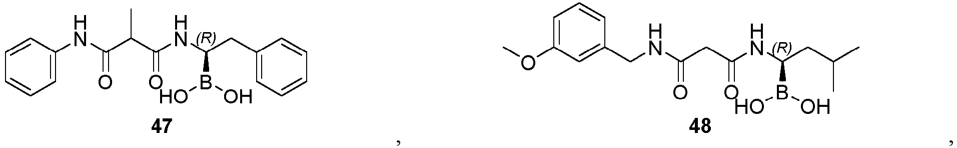

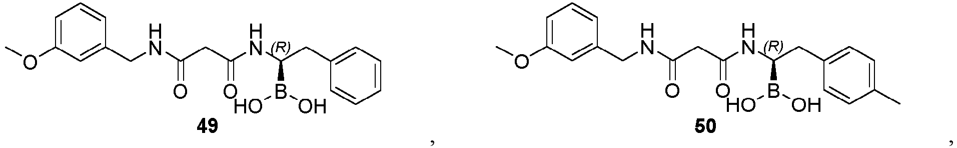

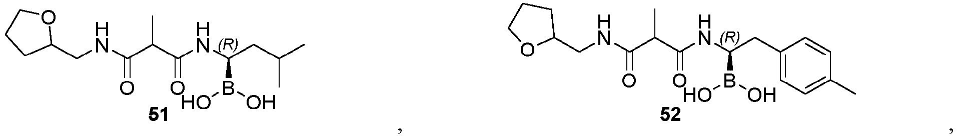

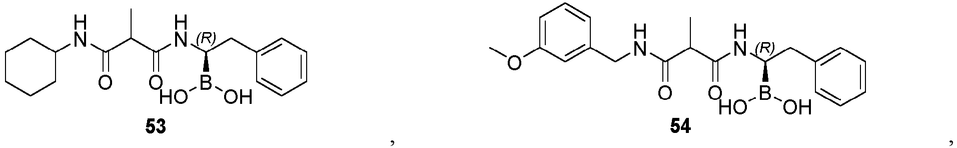

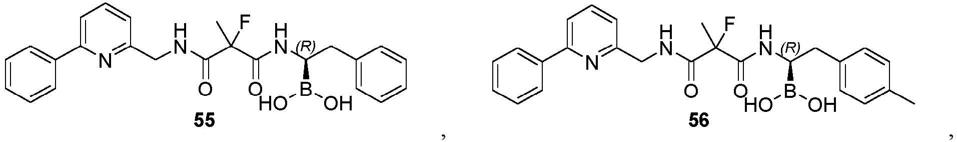

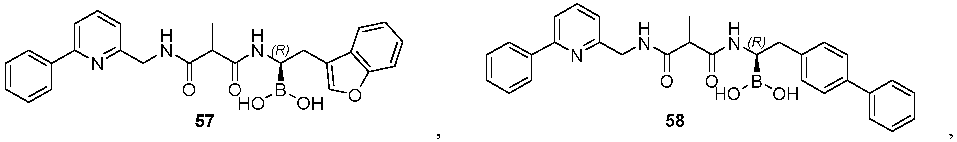

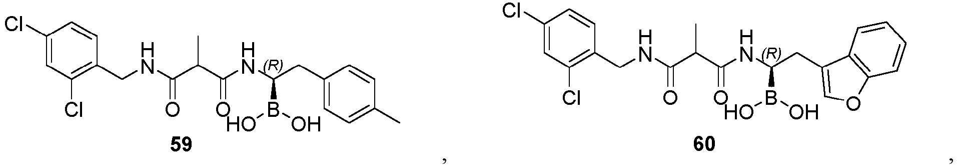

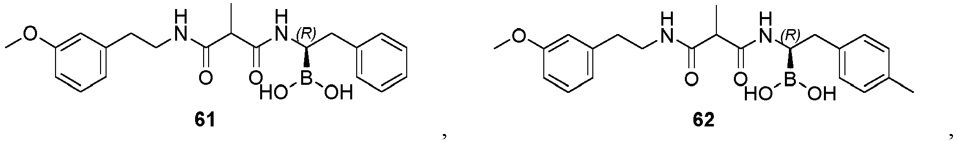

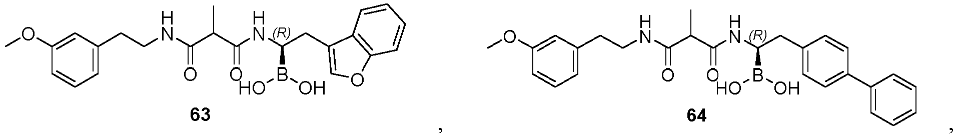

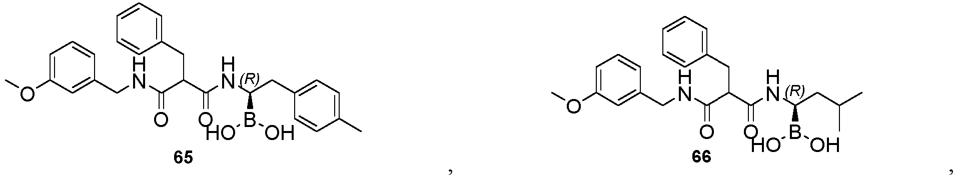

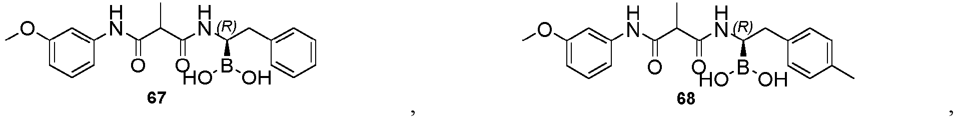

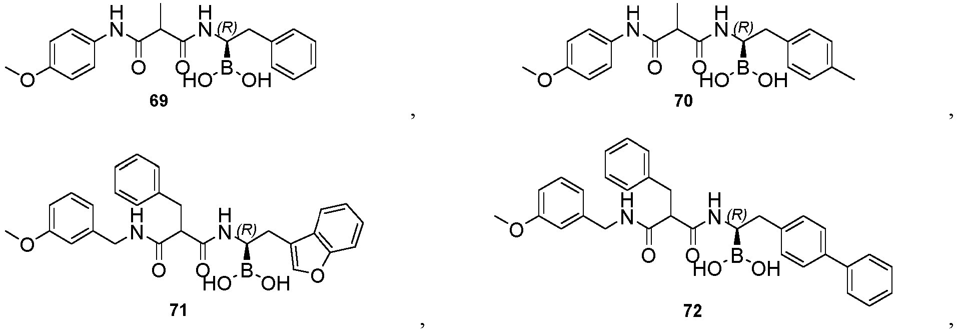

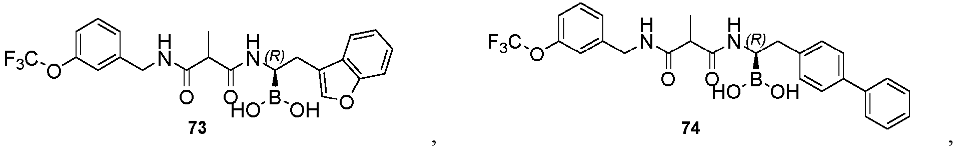

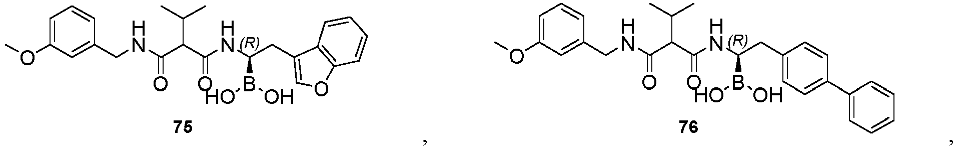

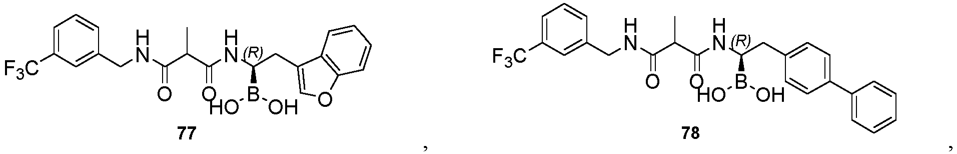

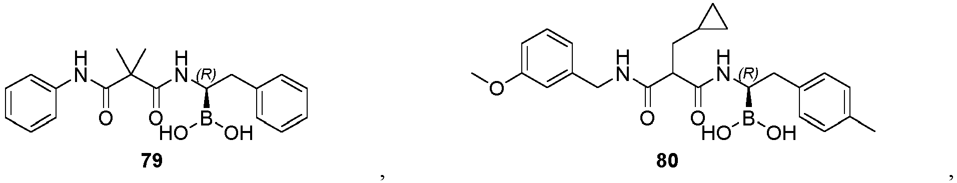

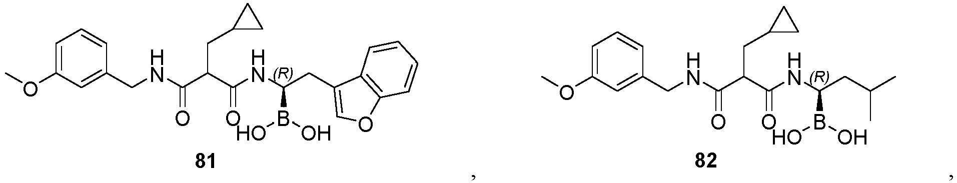

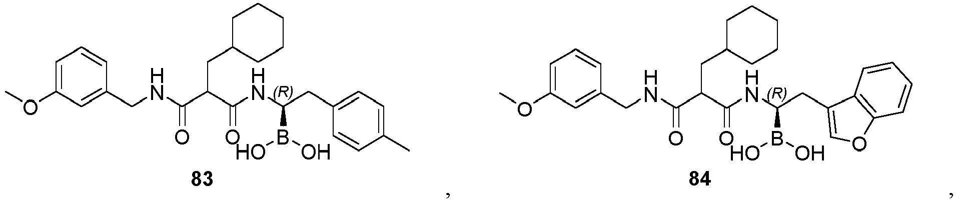

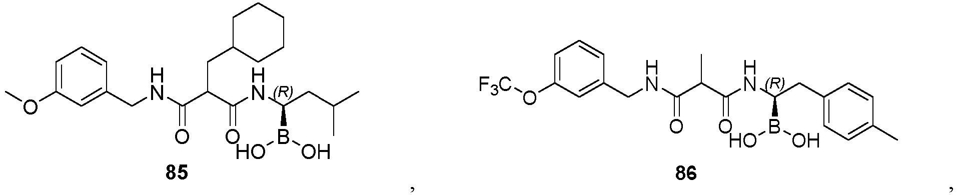

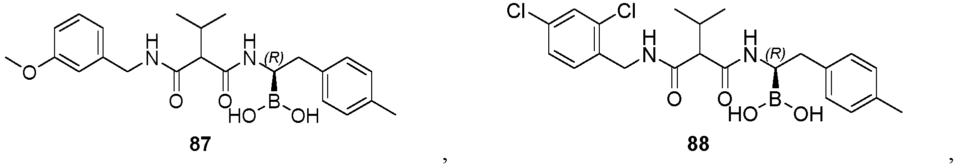

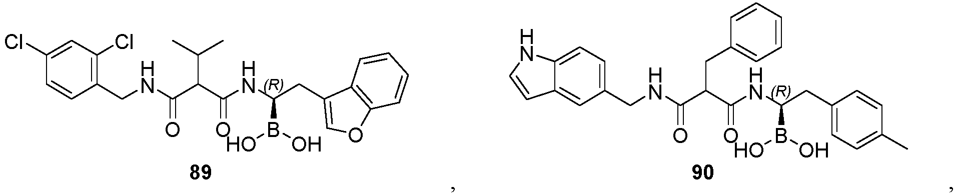

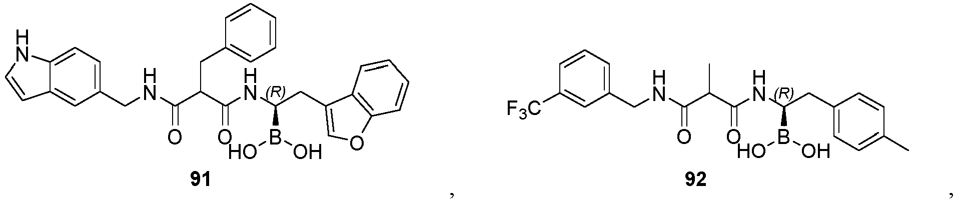

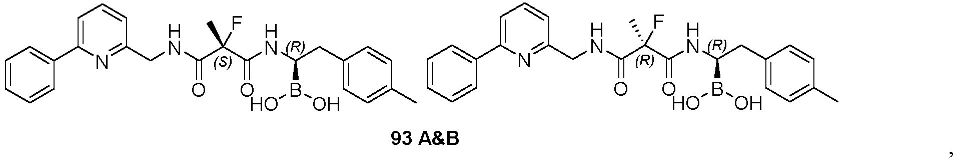

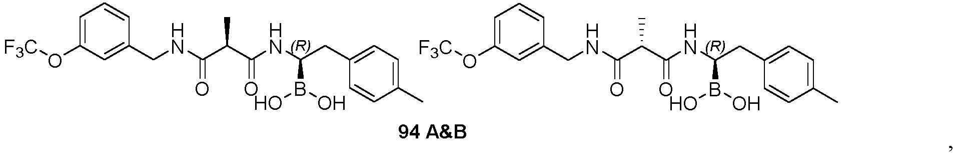

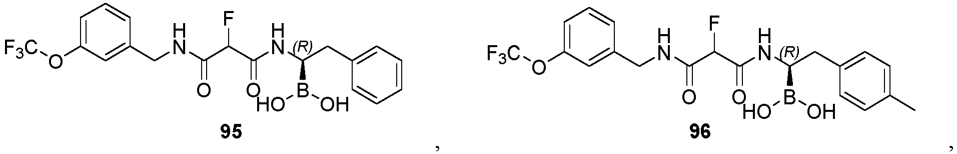

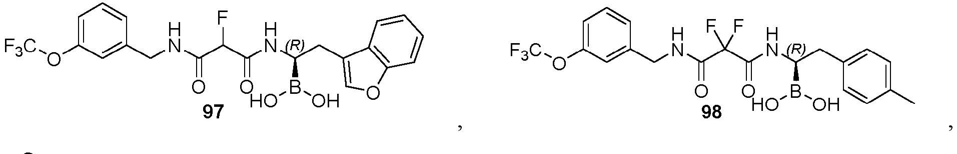

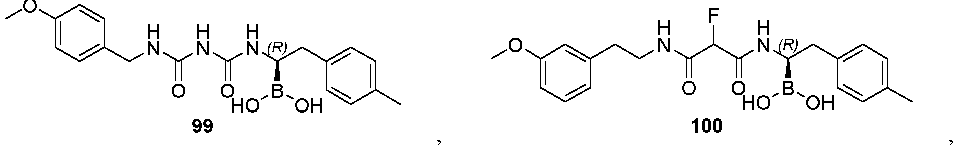

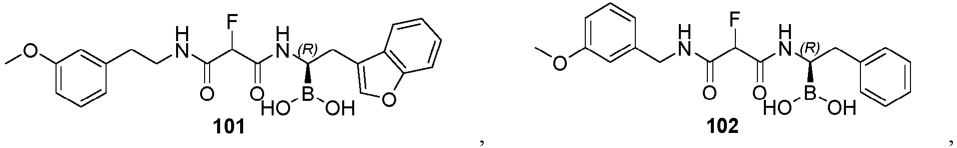

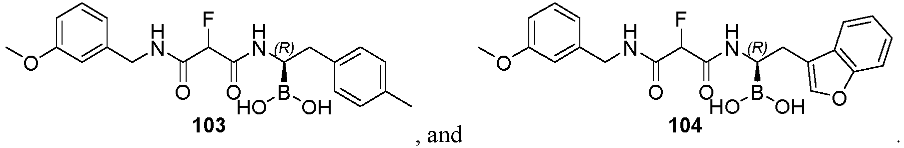

In the present disclosure, the following compounds or the pharmaceutically acceptable salts thereof are more preferable:

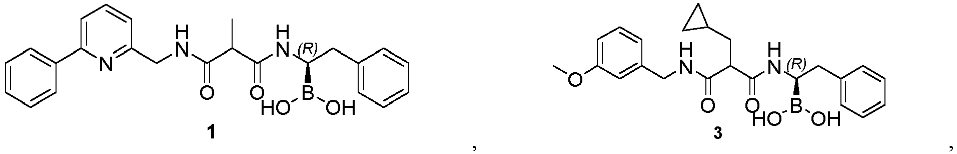

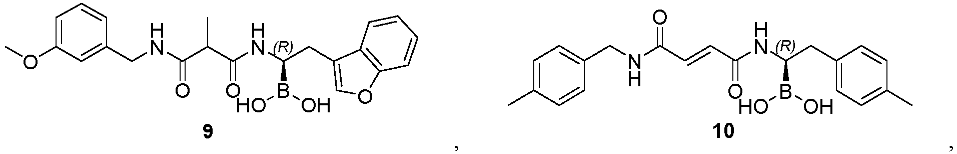

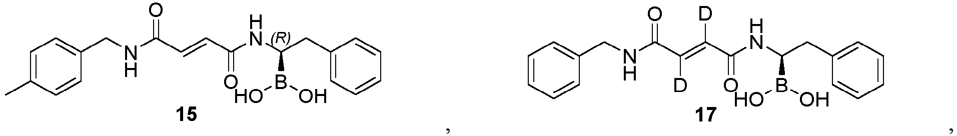

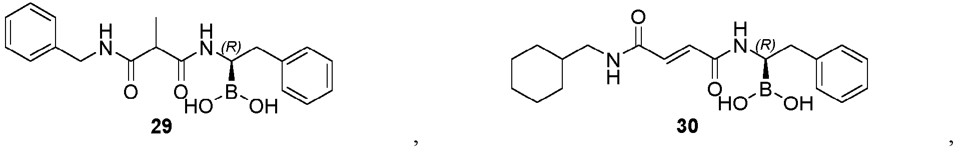

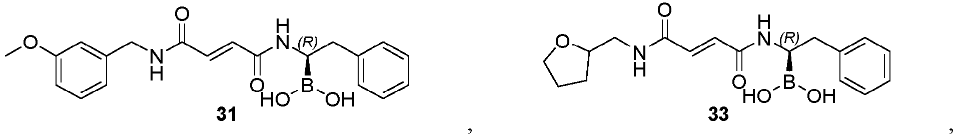

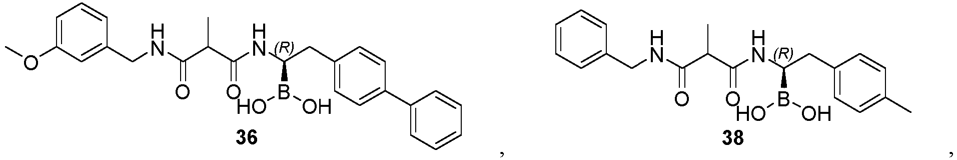

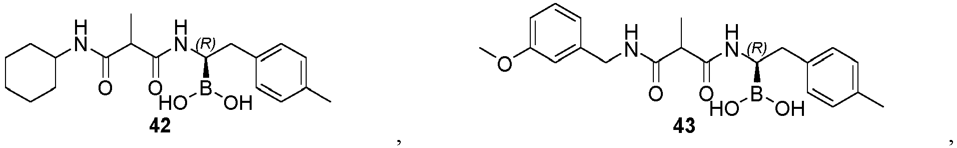

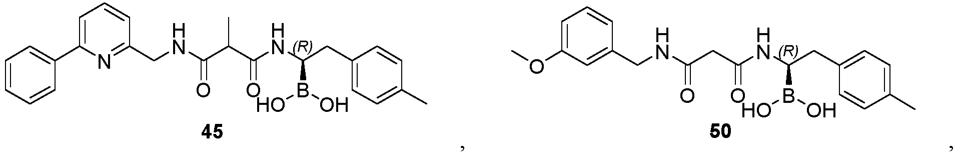

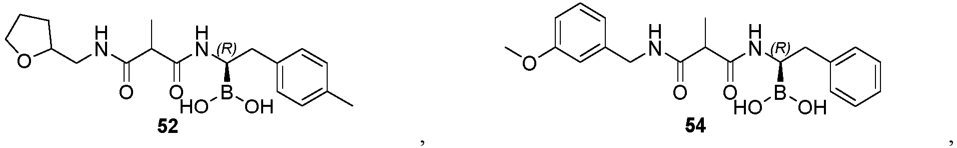

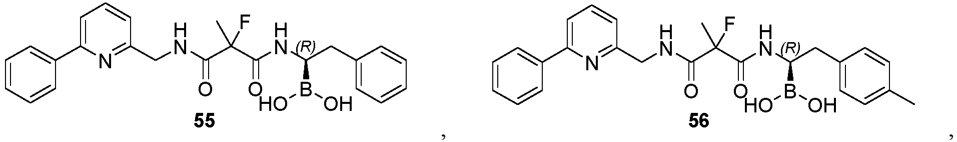

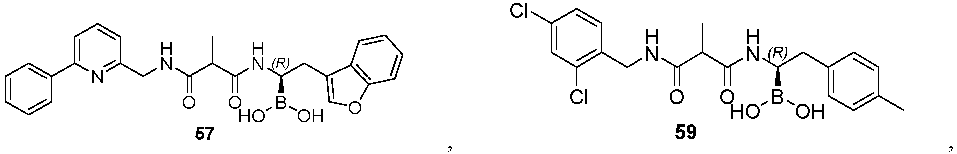

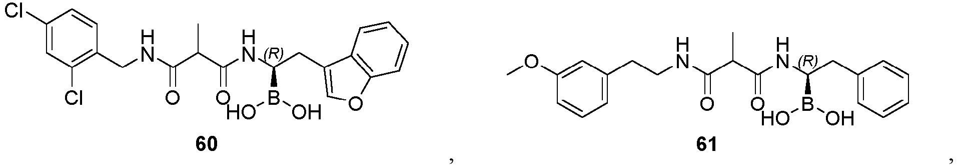

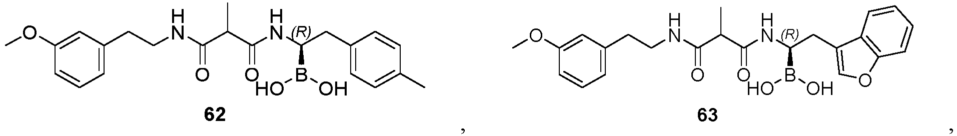

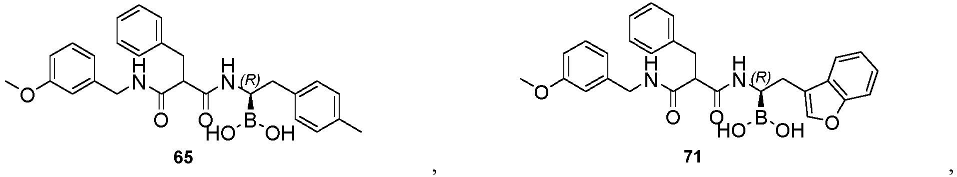

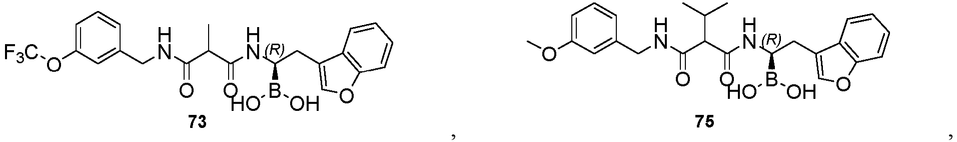

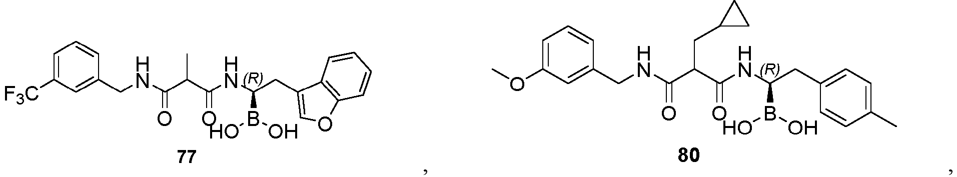

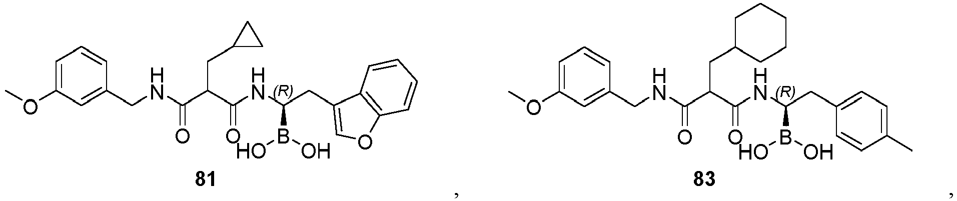

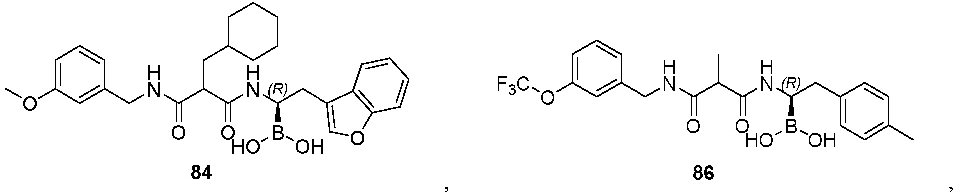

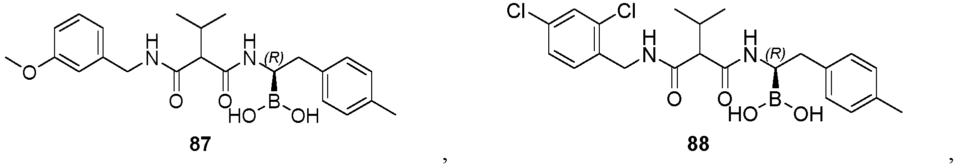

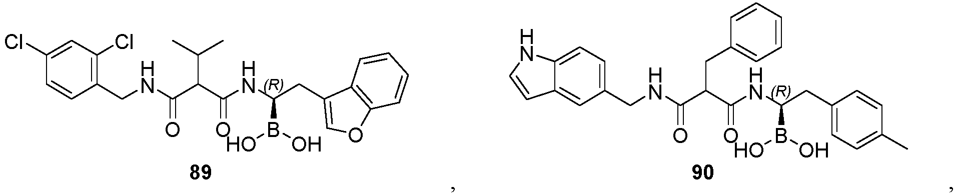

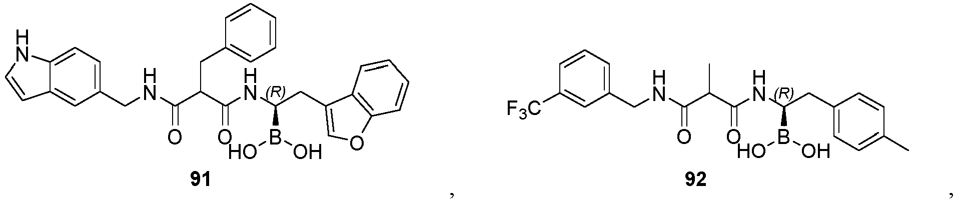

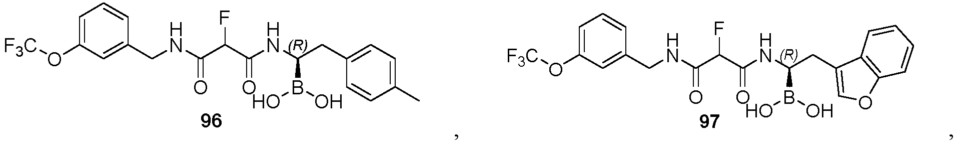

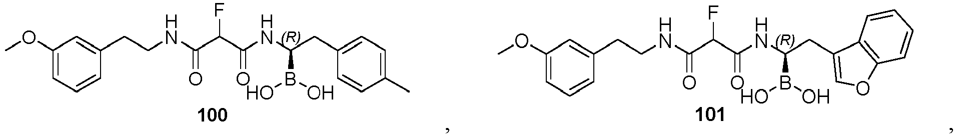

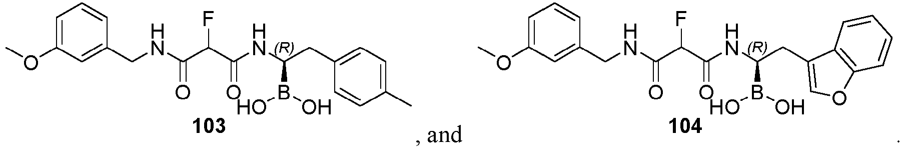

In another aspect, the present disclosure provides a pharmaceutical composition comprising the compound represented by formula I or the pharmaceutically acceptable salt thereof of the present disclosure, and an optional pharmaceutically acceptable carrier. In some embodiments, the compound represented by formula I has a structure of formula II, formula III, formula IV, formula V, or formula VI as described above. In some other embodiments, the compound represented by formula I is a specific compound described above.

In yet another aspect, the present disclosure provides use of the compound or the pharmaceutically acceptable salt thereof, or the pharmaceutical composition of the present disclosure above in the manufacture of a medicament as a proteasome inhibitor.

In yet another aspect, the present disclosure provides use of the compound or the pharmaceutically acceptable salt thereof, or the pharmaceutical composition of the present disclosure above in the manufacture of a medicament for the treatment or prevention of a disease related to proteasome.

The disease related to proteasome includes, for example, a tumor and autoimmune disease. The tumor in the present disclosure includes solid tumor and hematological tumor. The solid tumor is selected from the group consisting of non-small cell lung cancer, small cell lung cancer, lung adenocarcinoma, lung squamous cell carcinoma, pancreatic cancer, breast cancer, prostate cancer, liver cancer, skin cancer, epithelial cell cancer, astrocytoma, neuroblastoma, meningioma, gastrointestinal stromal tumor, and nasopharyngeal carcinoma, and it includes in situ tumor and metastatic tumor of tumors above. The hematological tumor is selected from the group consisting of leukemia, multiple myeloma, mantle cell lymphoma or histiocytic lymphoma, Hodgkin's lymphoma and non-Hodgkin's lymphoma. Examples of the autoimmune disease of the present disclosure include, but are not limited to, inflammatory responses such as inflammatory skin diseases including psoriasis and dermatitis (e.g., atopic dermatitis), systemic scleroderma and sclerosis, responses associated with inflammatory bowel disease (such as Crohn's disease and ulcerative colitis), respiratory distress syndrome (including adult respiratory distress syndrome(ARDS)), dermatitis, meningitis, encephalitis, uveitis, colitis, glomerulonephritis, allergic conditions such as eczema and asthma and other conditions involving infiltration of T cells and chronic inflammatory responses, atherosclerosis, leukocyte adhesion deficiency, rheumatoid arthritis, systemic lupus erythematosus (SLE), diabetes mellitus (e.g., Type I diabetes mellitus or insulin dependent diabetes mellitus), multiple sclerosis, Reynaud's syndrome, autoimmune thyroiditis, Graves' disease, allergic encephalomyelitis, Sjorgen's syndrome, juvenile onset diabetes, and immune responses associated with acute and delayed hypersensitivity mediated by cytokines and T-lymphocytes typically found in tuberculosis, sarcoidosis, polymyositis, granulomatosis and vasculitis, pernicious anemia (Addison's disease), diseases involving leukocyte diapedesis, central nervous system (CNS) inflammatory disorder, multiple organ injury syndrome, hemolytic anemia (including, but not limited to cryoglobinemia or Coombs positive anemia), myasthenia gravis, antigen-antibody complex mediated diseases, anti-glomerular basement membrane disease, immune complex nephritis, IgA nephropathy, antiphospholipid syndrome, allergic neuritis, Lambert-Eaton myasthenic syndrome, pemphigoid bullous, pemphigus, autoimmune polyendocrinopathies, Reiter's syndrome, stiff-man syndrome, Behcet's disease, giant cell arteritis, IgM polyneuropathies, immune thrombocytopenic purpura (ITP) or autoimmune thrombocytopenia, organ transplantation-induced rejection and graft-versus-host disease. In specific cases, the autoimmune disease is systemic lupus erythematosus or lupus nephritis. In some cases, the autoimmune disease is systemic vasculitis or idiopathic inflammatory myopathy.

In some embodiments, the disease is selected from the group consisting of a tumor and an autoimmune disease. In some embodiments, the disease is multiple myeloma. In some other embodiments, the disease is selected from the group consisting of systemic lupus erythematosus (SLE) and inflammatory bowel disease (IBD).

In the present disclosure, the carbon atom to which the boron atom is attached in the compound represented by formula I should maintain specific stereo configuration represented by formula I. When the other carbon atoms are chiral carbons, they can be either R-configuration or S-configuration, and the compound of the present disclosure accordingly includes either a single stereoisomer or a mixture of various proportions of isomers.

It is well known to those skilled in the art that a salt, solvate, hydrate, and boric acid polymer of a compound are alternative forms of the compound, all of which can be converted to the compound under certain conditions, and it is therefore of particular note that when a compound is referred to herein, it is generally also included in the list of its pharmaceutically acceptable salts, and consequently its solvates, hydrates, and boric acid polymers.

The pharmaceutically acceptable salt in the present disclosure can be formed using, for example, the following inorganic or organic acids. "The pharmaceutically acceptable salt" means such salt, within the scope of reasonable medical judgment, which is suitable for use in contact with tissues of humans and lower animal without undue toxicity, irritation, allergic reaction, and the like, and can be described as a reasonable benefit/risk ratio. The salt can be prepared in situ during the final isolation and purification of the compound of the present disclosure, or separately by reacting the free base or free acid with a suitable regnant, as summarized below. For example, the functional groups of the free base can react with suitable acids. In addition, when the compound of the present disclosure carries an acidic moiety, suitable pharmaceutically acceptable salts thereof may include metal salts, such as alkali metal salts (such as sodium or potassium salts) and alkaline earth metal salts (such as calcium salts or magnesium salts). Examples of pharmaceutically acceptable nontoxic acid addition salts are salts formed by amino groups with inorganic acids (e.g., hydrochloric acid, hydrobromic acid, phosphoric acid, sulfuric acid, and perchloric acid) or organic acids (e.g., acetic acid, oxalic acid, maleic acid, tartaric acid, citric acid, succinic acid or malonic acid) or by using other methods in the art such as ion exchange. Other pharmaceutically acceptable salts include adipate, sodium alginate, ascorbate, aspartate, benzenesulfonate, benzoate, bisulfate, borate, butyrate, camphorate, camphorsulfonate, citrate, cyclopentane propionate, digluconate, lauryl sulfate, ethanesulfonate, formate, fumarate, glucoheptonate, glycerophosphate, gluconate, enanthate, hexanoate, hydroiodide, 2-hydroxyethanesulfonate, lactobiate, lactate, laurate, lauryl sulfate, malate, maleate, malonate, methanesulfonate, 2-naphthalenesulfonate, nicotinate, nitrate, oleate, oxalate, palmitate, pamateate, pectate, persulfate, 3-phenylpropionate, phosphate, bitter salt, pivalate, propionate, stearate, succinate, sulfate, tartrate, thiocyanate, tosylate, undecanoate, valerate, etc. Representative alkali metal or alkaline earth metal salts include sodium salts, lithium salts, potassium salts, calcium salts, magnesium salts, and the like. Other pharmaceutically acceptable salts include, where appropriate, nontoxic ammonium salts, quaternary ammonium salts, and ammonium cations formed with counterions, e.g., halides, hydroxides, carboxylates, sulfates, phosphates, nitrates, lower alkylsulfonates and arylsulfonates.

The compound or the pharmaceutically acceptable salt thereof of the present disclosure can be administered orally or parenterally as an active ingredient, and its effective amount ranges from 0.1 to 1000 mg/kg body weight/day in mammals including humans (about 70 kg in body weight), preferably 1 to 100 mg/kg body weight/day, and administered in single or divided doses per day, or with or without a scheduled time. The dose of the active ingredient can be adjusted according to a number of relevant factors (such as the condition of the subject to be treated, the type and severity of the disease, the rate of administration and the doctor's opinion). In some cases, amounts less than the above dosages may be appropriate. If no deleterious side effects are caused, amounts greater than the above doses may be used and such amounts may be administered in divided doses daily.

In addition, the present disclosure further provides a method for preventing and/or treating a disease related to proteasome, which comprises administering a compound or a pharmaceutical composition of the present disclosure to a mammal, including a human in need thereof. In some embodiments, the disease related to proteasome is selected from the group consisting of a tumor and an autoimmune disease. In some embodiments, the disease related to proteasome is multiple myeloma. In other embodiments, the disease related to proteasome is selected from the group consisting of systemic lupus erythematosus (SLE) and inflammatory bowel disease (IBD).

In the present disclosure, "optionally substituted with......" means that the groups such as x, R1, R2 and R3 may or may not be substituted by a substituent, i.e., they are not limited to the situation where they are substituted with the listed groups, but also include the situation where they are not substituted with the listed groups. This expression is the same as the expression "R is a substituted or unsubstituted C₁₋₁₀ alkyl, C₃₋₆ cycloalkyl or heterocycloalkyl, phenyl, naphthyl, or indolyl, where the substituent is C₁₋₄ alkyl, C₁₋₄ alkoxy, cyano, hydroxyl, mercapto, amino, or halogen." But, the scope defined by term substituted or unsubstituted doesn't narrowly include C₁₋₁₀ alkyl, but also expands to all the groups mentioned. In the present disclosure, when referring to "optionally substituted with...... ", the substituent can be deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino and halogen, wherein the aryl group as a substituent can be further substituted by substituents such as deuterium, C₁₋₄ alkyl, C₁₋₁₀ alkoxy, C₃₋₆ cycloalkyl, cyano, hydroxyl, mercapto, amino and halogen.

The term "alkyl" is used to denote a linear or branched saturated hydrocarbon group. For example, C₁₋₃ alkyl means a saturated hydrocarbon group containing 1 to 3 carbon atoms, C₁₋₄ alkyl means a saturated hydrocarbon group containing 1 to 4 carbon atoms, and C₁₋₁₀ alkyl means a saturated hydrocarbon group containing 1 to 10 carbon atoms.

The term "alkoxy" refers to alkyl-O-. The term "C₁₋₆ alkoxy" is intended to include C₁, C₂, C₃, C₄, C₅, and C₆ alkoxy. Examples of alkoxy include, but are not limited to, methoxy, ethoxy, propoxy (such as n-propoxy and isopropoxy), and tert-butoxy. Similarly, "alkylthio" or "thioalkoxy" refers to alkyl as defined above having the indicated number of carbon atoms and connected through a sulfur bridge; for example, methyl-S- and ethyl-S-. Preferred alkoxy herein are C₁₋₆ alkoxy.

The term "cycloalkyl" refers to non-aromatic carbocyclic groups, including cyclized alkyl groups. Cycloalkyl can include monocyclic, bicyclic or polycyclic ring systems. Examples of cycloalkyl include cyclopropyl, cyclobutyl, cyclopentyl, cyclohexyl, and cycloheptyl, and the C₃₋₆ cycloalkyl refers to a cycloalkyl group containing 3 to 6 carbon atoms.

The term "heterocycloalkyl" or 'heterocyclyl' refers to a non-aromatic heterocycloalkyl group in which one, two or three ring-forming carbon atoms are replaced by heteroatoms such as O, N and S atoms. Preferably, the heterocycloalkyl preferably has 3, 4, 5, 6 or 7 ring-forming atoms. Preferred heterocycloalkyls herein are C₃₋₈ heterocycloalkyls.

The term "benzyl" refers to phenylmethyl, and the substituted benzyl refers to the substitution of at least one hydrogen atom on the benzene ring of the benzyl group with a non-hydrogen moiety. The substituent to the benzyl group may be halogen, -CN, -OH, -SH, -NH₂, or C₁₋₆ alkyl.

The term "C₁₋₃ alkyl heterocyclyl" refers to the substitution of an aromatic heterocyclyl group or a non-aromatic heterocyclyl group on a saturated hydrocarbon group containing 1-3 carbon atoms, including a cyclized alkyl in which one or more of the ring-forming carbon atoms is replaced by heteroatoms such as O, N and S atoms. The heterocycloalkyl preferably has 3, 4, 5, 6 or 7 ring-forming atoms.

"C₁₋₁₀ alkoxy" refers to -O-alkyl groups, and alkyl includes straight chain, branched chain and cyclic alkyl having 1 to 10 carbon atoms. Examples of alkoxy include methoxy, ethoxy, propoxy (such as n-propoxy, isopropoxy, and cyclopropoxy), and tert-butoxy.

"Aryl" refers to an aromatic carbocyclic group, including monocyclic, bicyclic, tricyclic or polycyclic aromatic hydrocarbon groups such as phenyl, naphthyl, anthryl, phenanthryl and the like. Aryl is preferably a monocyclic, bicyclic or tricyclic ring system having 5 to 12 ring members, where at least one ring in the system is aromatic and each ring in the system contains 3 to 7 ring members. "Substituted aryl" means that at least one hydrogen atom on the benzene ring of the aryl group is substituted with a non-hydrogen portion, and the substituent of the aryl may be halogen, C₁₋₁₀ alkoxy, -CN, -OH, -SH, -NH₂, or C₁₋₆ alkyl. Preferred aryl includes phenyl, biphenyl, indanyl, 1-naphthyl, 2-naphthyl and tetrahydronaphthyl. The term "aralkyl" or "arylalkyl" refers to an alkyl residue attached to an aromatic ring. Non-limiting examples include benzyl, phenylethyl, and the like. Fused aryl may be attached to another group at a suitable position on the cycloalkyl ring or aromatic ring. For example, a dotted line drawn from a ring system indicates a bond that may be attached to any suitable ring atom.

The term "heteroaryl" means a stable 5 to 12-membered aromatic monocyclic or aromatic bicyclic or aromatic polycyclic heterocyclic ring, which is fully unsaturated, partially unsaturated, and which contains carbon atoms and 1, 2, 3 or 4 heteroatoms independently selected from the group consisting of N, O, and S. It includes 5-, 6-, or 7-membered aromatic monocyclic or 8-, 9-, 10-, 11-, 12-membered aromatic bicyclic or aromatic polycyclic heterocyclic rings. Preferably, any heterocyclic ring as defined above is fused with a benzene ring. Nitrogen and sulfur atoms may be optionally oxidized. Nitrogen atom is substituted or unsubstituted (i.e. N or NR, where R is H or another substituent if defined). A heterocycle may be attached to its side group at any heteroatom or carbon atom that results in a stable structure. If the resulting compound is stable, the heterocyclyl described herein may be substituted on carbon or nitrogen atoms. The nitrogen in the heterocycle may optionally be quaternized. Preferably, in the case that the total number of S and O atoms in the heterocycle exceeds 1, these heteroatoms are not adjacent to each other. Examples of heteroaryl include, but are not limited to, acridinyl, azetidinyl, azocinyl, benzimidazolyl, benzofuryl, benzothiofuryl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolyl, dihydrofuro[2,3-b]tetrahydrofuryl, furyl, furazanyl, imidazolidinyl, imidazolinyl, imidazolyl, 1H-indazolyl, imidazopyridyl, dihydroindolyl, indolizinyl, indolyl, 3H-indolyl, isobenzofuryl, isochromanyl, isoindazolyl, isodihydroindolyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridyl, oxazolidinyl, perimidinyl, hydroxyindolyl, pyrimidinyl, phenanthridinyl, phenanthrolinyl, phenazinyl, phenothiazinyl, phenoxathiyl, phenoxazinyl, phthalazinyl, piperazinyl, piperidinyl, piperidinonyl, 4-piperidinonyl, piperonyl, pteridinyl, purinyl, pyranyl, pyrazinyl, pyrazolidinyl, pyrazolinyl, pyrazolopyridyl, pyrazolyl, pyridazinyl, pyridoxazolyl, pyridimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolinyl, 4H-quinazinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthryl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl, xanthenyl, quinolinyl, isoquinolyl, phthalazinyl, quinazolinyl, indolyl, 1H-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuryl, 1,2,3,4-tetrahydroquinoxalinyl and 1,2,3,4-tetrahydroquinazolinyl. The term "heteroaryl" may also include a biaryl structure formed by an "aryl" as defined above with a monocyclic "heteroaryl", for example, but not limited to, the following -phenyl-pyridyl-, -phenyl-pyrimidinyl, -pyridyl-phenyl, -pyridyl-pyrimidinyl, and-pyrimidinyl-phenyl-.

"Aryloxy" refers to -O-aryl, and the concept of aryl is as described above, the most preferred example of an aryloxy is phenoxyl.

"Halogen" includes fluorine, chlorine, bromine and iodine.

The term "bridged cycloalkyl" as used herein refers to polycyclic compounds sharing two or more carbon atoms, including bicyclic bridged hydrocarbons and polycyclic bridged hydrocarbons. The former consists of two aliphatic rings sharing more than two carbon atoms; the latter is a cyclic bridged hydrocarbon consisting of three or more rings. Preferred bridged cycloalkyl herein is selected from adamantyl and adamantan-1-yl-methyl.

### DETAILED DESCRIPTION

### Part I: Synthesis of compounds.

### Synthesis Example:

### Key intermediate BB1: (R)-2-phenyl-1-(4,4,5,5-tetramethyl-1,3,2-dioxaborolan-2-yl)ethan-1-amine hydrochloride

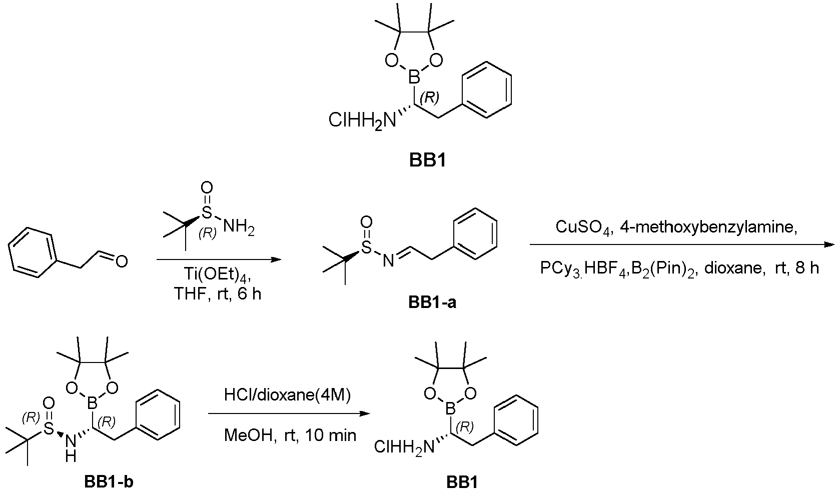

### Implementation steps:

### Step 1: (R,E)-2-methyl-N-(2-phenylethylidene)propane-2-sulfinamide

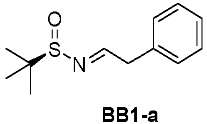

Phenylacetaldehyde (4.40 g, 20.0 mmol) and (R)-2-methylpropane-2-sulfinamide (2.43 g, 20.0 mmol) were dissolved in dried tetrahydrofuran (40 mL) followed by slow dropwise addition of tetraethyl titanate (7.7 mL, 40.0 mmol). This reaction solution was stirred at room temperature for 26 h of reaction. After completion of reaction, the reaction solution was added with water (60 mL), continued to be stirred for 30 min, filtered to remove an insoluble material, and the filtrate was extracted with ethyl acetate (60 ml×2). The organic phases were combined, washed with saturated sodium chloride solution (40 mL), dried over anhydrous sodium sulfate, filtered and concentrated. The residue was separated and purified by column chromatography (an eluent of 10% ethyl acetate in petroleum ether solution) to obtain a colorless liquid (3.50 g, 78%).

LC-MS m/z: 224.0 [M+1]⁺.

¹HNMR (400 MHz, CDCl₃): δ 8.13 (t, *J* = 5.2 Hz, 1H), 7.35 - 7.22 (m, 5H), 3.88 - 3.78 (m, 2H), 1.19 (s, 9H).

### Step 2: (R)-2-methyl-N-((R)-2-phenyl-1-(4,4,5,5-tetramethyl-1,3,2-dioxaborolan-2-yl)ethyl)propane-2-su lfinamide

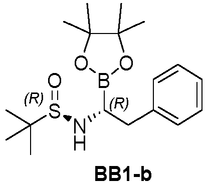

(*R*,*E*)-2-methyl-*N*-(2-phenylethylidene)propane-2-sulfinamide (3.50 g, 15.7 mmol) was dissolved in 1,4-dioxane (35 mL), and then added with 4-methoxybenzylamine (107 mg, 0.78 mmol), tricyclohexylphosphonium tetrafluoroborate (69 mg, 0.19 mmol), and an aqueous solution of copper sulfate (6.3 mL, 0.03 M, 0.19 mmol). This reaction solution was cooled to 0°C and added in portions with bis(pinacolato)diboron (5.97 g, 23.5 mmol). The reaction solution was slowly heated to room temperature and stirred for 15 h of reaction under nitrogen protection. After completion of reaction, the reaction solution was poured into water (40 mL) and extracted with ethyl acetate (30 ml×2). The organic phases were combined, then washed with saturated sodium chloride solution (30 mL), dried over anhydrous sodium sulfate, filtered and then concentrated. The residue was separated and purified by column chromatography (an eluent of 35% dichloromethane in petroleum ether solution) to obtain a compound as a colorless liquid (3.30 g, 60%).

¹HNMR (400 MHz, DMSO-d6): δ 7.28 - 7.16 (m, 5H), 4.79 (d, *J* = 5.6 Hz, 1H), 3.09 - 3.03 (m, 1H), 2.97 - 2.92 (m, 1H), 2.82 - 2.77 (m, 1H), 1.11 (s, 6H), 1.08 (s, 9H), 1.03 (s, 6H).

### Step 3: (R)-2-phenyl-1-(4,4,5,5-tetramethyl-1,3,2-dioxaborolan-2-yl)ethan-1-amine hydrochloride

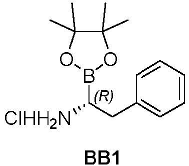

(*R*)-2-methyl-*N*-((*R*)-2-phenyl-1-(4,4,5,5-tetramethyl-1,3,2-dioxaborolan-2-yl)ethyl)pro pane-2-sulfinamide (250 mg, 0.72 mmol) was dissolved in methanol (5 mL), and then added with a solution of hydrogen chloride in dioxane (0.9 mL, 4 M, 3.55 mmol). The reaction solution was stirred at room temperature for 30 min of reaction. After completion of reaction, the reaction solution was concentrated under reduced pressure and the resulting crude product was pulped in tert-butyl methyl ether (2 mL) to obatin a yellow solid (210 mg, 99%).

LC-MS m/z: 248.1 [M+1]⁺.

¹HNMR (400 MHz, DMSO-d6): δ 8.00 (brs, 3H). 7.34 - 7.30 (m, 2H), 7.26 - 7.23 (m, 3H), 3.03 - 2.96 (m, 2H), 2.90 - 2.83 (m, 1H), 1.18 (s, 6H), 1.14 (s, 6H).

### Key intermediate BB2: (R)-1-(4,4,5,5-tetramethyl-1,3,2-dioxaborolan-2-yl)-2-(p-tolyl)ethan-1-amine hydrochloride

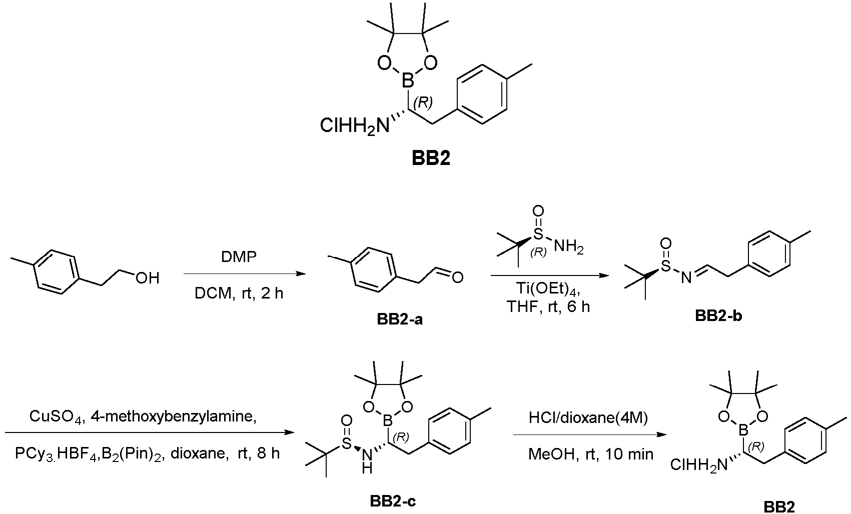

### Implementation steps:

### Step 1: 2-(p-tolyl)acetaldehyde

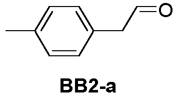

2-(p-tolyl)ethan-1-ol (1.36 g, 10.0 mmol) was dissolved in dichloromethane (25 mL) and then added with Dess-Martin reagent (4.67 g, 11.0 mmol) in 3 portions at 0°C. After completion of reaction, the reaction solution was quenched by the addition of saturated sodium thiosulfate solution (30 mL) and stirred for 30 min. The organic phase was sequentially washed with saturated sodium bicarbonate solution (30 ml×2), and saturated sodium chloride solution (30 mL), then dried over anhydrous sodium sulfate, filtered and concentrated under reduced pressure to obtain a colorless liquid (1.10 g, 82%).

¹HNMR (400 MHz, CDCl₃): δ 9.73 (t, *J* = 2.4 Hz, 1H), 7.19 - 7.17 (m, 2H), 7.11 - 7.09 (m, 2H), 3.64 (d, *J* = 2.4 Hz, 2H), 2.35 (s, 3H).

### Step 2: (R,E)-2-methyl-N-(2-(p-tolyl)ethylidene)propane-2-sulfinamide

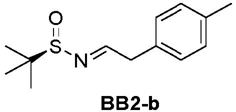

2-(p-tolyl)acetaldehyde (1.10 g, 8.2 mmol) and (R)-2-methylpropane-2-sulfinamide (1.20 g, 9.8 mmol) were dissolved in dried tetrahydrofuran (20 mL) followed by slow dropwise addition of tetraethyl titanate (3.4 mL, 16.4 mmol). The reaction solution was stirred at room temperature for 6 h of reaction. After completion of reaction, the reaction solution was added with water (50 mL), continued to be stirred for 30 min, and filtered to remove an insoluble material. The filtrate was extracted with ethyl acetate (50 ml×2). The organic phases were combined, washed with saturated sodium chloride solution (30 mL), dried over anhydrous sodium sulfate, filtered and concentrated. The residue was separated and purified by column chromatography (an eluent of 10% ethyl acetate in petroleum ether solution) to obtain a yellow liquid (1.10 g, 57%).

LC-MS m/z: 238.3 [M+1]⁺.

¹HNMR (400 MHz, CDCl₃): δ 8.11 (t, *J* = 5.2 Hz, 1H), 7.15 - 7.10 (m, 4H), 3.83 - 3.74 (m, 2H), 2.33 (s, 3H), 1.19 (s, 9H).

### Step 3: (R)-2-methyl-N-((R)-1-(4,4,5,5-tetramethyl-1,3,2-dioxaborolan-2-yl)-2-(p-tolyl)ethyl)propane-2-s ulfinamide

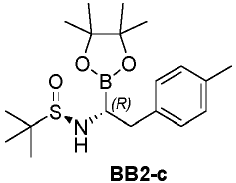

(*R*,*E*)-2-methyl-*N*-(2-(p-tolyl)ethylidene)propane-2-sulfinamide (1.10 g, 3.01 mmol) was dissolved in 1,4-dioxane (20 mL), and then 4-methoxybenzylamine (32 mg, 0.23 mmol), tricyclohexylphosphonium tetrafluoroborate (21 mg, 0.056 mmol), and an aqueous solution of copper sulfate (1.9 mL, 0.3 M, 0.056 mmol) were added. This reaction solution was cooled to 0°C and bis(pinacolato)diboron (1.76 g, 6.94 mmol) was added in portions. The reaction solution was slowly heated to room temperature and stirred for 6 h of reaction under nitrogen protection. After completion of reaction, the reaction solution was poured into water (50 mL) and extracted with ethyl acetate (50 ml×2), The organic phases were combined, washed with saturated sodium chloride solution (30 mL), dried over anhydrous sodium sulfate, filtered and concentrated. The residue was separated and purified by column chromatography (an eluent of 20% ethyl acetate in petroleum ether solution) to obtain a colorless liquid (1.35 g, 80%).

LC-MS m/z: 366.1 [M+1]⁺.

¹HNMR (400 MHz, CDCl₃): δ 7.15 (d, *J* = 8.0 Hz, 2H), 7.08 (d, *J* = 8.0 Hz, 2H), 3.34 - 3.29 (m, 1H), 3.24 - 3.22 (m, 1H), 3.00 - 2.91 (m, 2H), 2.30 (s, 3H), 1.24 (s, 9H), 1.20 (s, 6H), 1.18 (s, 6H).

### Step 4: (R)-1-(4,4,5,5-tetramethyl-1,3,2-dioxaborolan-2-yl)-2-(p-tolyl)ethan-1-amine hydrochloride

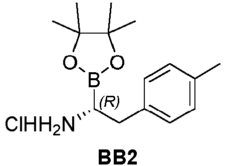

(*R*)-2-methyl-*N*-((R)-1-(4,4,5,5-tetramethyl-1,3,2-dioxaborolan-2-yl)-2-(p-tolyl)ethyl)pr opane-2-sulfinamide (250 mg, 0.71 mmol) was dissolved in methanol (5 mL), and then added with a solution of hydrogen chloride in dioxane (0.9 mL, 4 M, 3.55 mmol). The reaction solution was stirred at room temperature for 30 min of reaction. After completion of reaction, the reaction solution was concentrated under reduced pressure and the resulting crude product was pulped in tert-butyl methyl ether (2 mL) to obtain a yellow solid (210 mg, 99%).

LC-MS m/z: 239.3 [M-17]⁺.

¹HNMR (400 MHz, DMSO-d6): δ 7.89 (brs, 3H), 7.13 (s, 4H), 2.98 - 2.90 (m, 2H), 2.86 - 2.80 (m, 1H), 2.27 (s, 3H), 1.18 (d, *J* = 13.2 Hz, 12H).

### Key intermediate BB3: (R)-2-(benzofuran-3-yl)-1-(4,4,5,5-tetramethyl-1,3,2-dioxaborolan-2-yl)ethan-1-amine hydrochloride

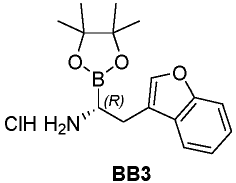

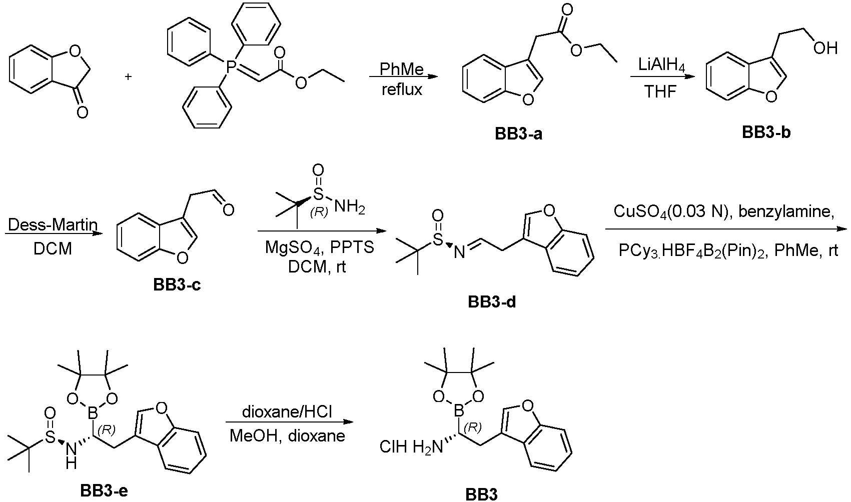

### Implementation steps:

### Step 1: Ethyl 2-(benzofuran-3-yl)acetate

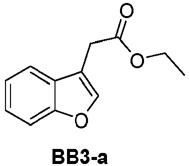

Benzofuran-3(2H)-one (4 g, 29.8 mmol) was dissolved in toluene (200 mL), and then added with (methoxycarbonylmethylene)triphenylphosphorane (15.6 g, 44.7 mmol). The reaction solution was stirred and reacted for 48 h under nitrogen protection. After completion of reaction, the reaction solution was cooled to room temperature, concentrated under reduced pressure, and the resulting crude product was separated and purified by column chromatography (an eluent of 2%-4% ethyl acetate in petroleum ether solution) to obtain a yellow liquid (3.5 g, 57%).

LC-MS m/z: 204.8 [M+1]⁺.

¹HNMR (400 MHz, CDCl₃): δ 7.63 (s, 1H), 7.58 - 7.56 (m, 1H), 7.47 (d, *J* = 8.0 Hz, 1H), 7.32 - 7.28 (m, 1H), 7.27-7.23 (m, 1H), 4.22 - 4.16 (m, 2H), 3.70 (d, *J* = 1.2 Hz, 2H), 1.27 (t, *J* = 7.2 Hz, 3H).

### Step 2: 2-(Benzofuran-3-yl)ethan-1-ol

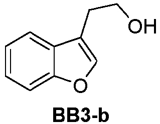

Ethyl 2-(benzofuran-3-yl)acetate (3.5 g, 17.1 mmol) was dissolved in tetrahydrofuran (20 mL) and then slowly added dropwise to a tetrahydrofuran suspension (50 mL) of lithium aluminium hydride (1.3 g, 34.2 mmol) in an ice-water bath under nitrogen protection. The reaction solution was stirred at room temperature for 4 h of reaction. After completion of reaction, water (1.3 mL), an aqueous solution of sodium hydroxide (1.3 mL, 15%) and water (3.9 mL) were added sequentially to the reaction solution. The reaction solution was stirred again for 30 minutes, and filtered to remove the solid. The filtrate was washed with saturated sodium chloride solution (30 mL), dried over anhydrous sodium sulfate, filtered and concentrated. The residue was separated and purified by column chromatography (an eluent of 30% ethyl acetate in petroleum ether solution) to obtain a yellow liquid (2.3 g, 83%).

LC-MS m/z: 162.8 [M+1]⁺.

¹HNMR (400 MHz, CDCl₃): δ 7.58 - 7.56 (m, 1H), 7.52 (brs, 1H), 7.49 - 7.47 (m, 1H), 7.32 - 7.23 (m, 2H), 3.96 - 3.91 (m, 2H), 2.97 - 2.94 (m, 2H), 1.55 (t, *J* = 4.8 Hz, 1H).

### Step 3: 2-(Benzofuran-3-yl)acetaldehyde

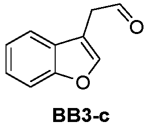

2-(Benzofuran-3-yl)ethan-1-ol (2.3 g, 14.2 mmol) was dissolved in dichloromethane (110 mL), and then added with Dess-Martin reagent (7.2 g, 17 mmol) in 3 portions at 0°C. The reaction solution was stirred at room temperature for 16 h of reaction. After completion of reaction, the reaction solution was quenched by the addition of saturated sodium thiosulfate solution (30 mL) and stirred for 30 min. The organic phase was washed with saturated sodium bicarbonate solution (30 mL×2) and saturated sodium chloride solution (30 mL), dried over anhydrous sodium sulfate and concentrated under reduced pressure to obtain a colorless liquid (2.23 g, 98%).

¹HNMR (400 MHz, CDCl₃): δ 9.82 (t, *J* = 2.0 Hz, 1H), 7.65 (brs, 1H), 7.52 - 7.48 (m, 2H), 7.36 - 7.31 (m, 1H), 7.29 - 7.25 (m, 1H), 3.78 - 3.77 (m, 2H).

### Step 4: (R,E)-N-(benzofuran-3-yl)ethylidene)-2-methylpropane-2-sulfinamide

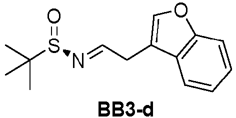

2-(Benzofuran-3-yl)acetaldehyde (2.23 g, 13.9 mmol), and (*R*)-2-methylpropane-2-sulfinamide (2.03 g, 16.7 mmol), and pyridinium 4-toluenesulfonate (175 mg, 0.7 mmol) were dissolved in dried dichloromethane (60 mL), and then added with anhydrous magnesium sulfate (8.4 g, 69.7 mmol ). The reaction solution was stirred and reacted under nitrogen protection at room temperature for 24 h. After completion of reaction, the reaction solution was filtered and concentrated. The residue was separated and purified by column chromatography (an eluent of 10% ethyl acetate in petroleum ether solution) to obtain a yellow liquid (2.3 g, 62%).

LC-MS m/z: 263.9 [M+1]⁺.

¹HNMR (400 MHz, CDCl₃): δ 8.19 (t, *J* = 4.8 Hz, 1H), 7.59 (brs, 1H), 7.54 - 7.48 (m, 2H), 7.34 - 7.30 (m, 1H), 7.27 - 7.23 (m, 1H), 3.91 - 3.90 (m, 2H), 1.18 (s, 9H).

### Step 5: (R)-N-((R)-2-(benzofuran-3-yl)-1-(4,4,5,5-tetramethyl-1,3,2-dioxaborolan-2-yl)ethyl)-2-methylpr opane-2-sulfinamide

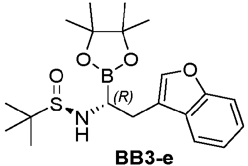

(*R,E*)-*N*-(benzofuran-3-yl)ethylidene)-2-methylpropane-2-sulfinamide (2.4 g, 9.1 mmol) was dissolved in toluene (30 mL), and then added with benzylamine (49 mg, 0.45 mmol), tricyclohexylphosphonium tetrafluoroborate (41 mg, 0.11 mmol), and an aqueous solution of copper sulfate (0.03 N, 3.7 mL). The reaction solution was cooled to 0°C, and added with bis(pinacolato)diboron (3.46 g, 13.7 mmol) in portions. The reaction solution was slowly heated to room temperature under nitrogen protection and stirred for 6 h of reaction. After completion of reaction, the reaction solution was poured into water (50 mL) and extracted with ethyl acetate (50 mL×2). The organic phases were combined, washed with saturated sodium chloride solution (30 mL), dried over anhydrous sodium sulfate, filtered and concentrated. The residue was separated and purified by column chromatography (an eluent of 20% ethyl acetate in petroleum ether solution) to obtain a colorless liquid (1.3 g, 36%).

LC-MS m/z: 391.9 [M+1]⁺.

¹HNMR (400 MHz, DMSO-d6): δ 7.77 (s, 1H), 7.66 - 7.64 (m, 1H), 7.53 (d, *J* = 7.6 Hz, 1H), 7.32 - 7.24 (m, 2H), 5.02 (d, *J* = 5.6 Hz, 1H), 3.24 - 3.18 (m, 1H), 3.02 -2.91 (m, 2H), 1.16 (s, 6H), 1.11 (s, 9H), 1.02 (s, 6H).

### Step 6: (R)-2-(benzofuran-3-yl)-1-(4,4,5,5-tetramethyl-1,3,2-dioxaborolan-2-yl)ethan-1-amine hydrochloride

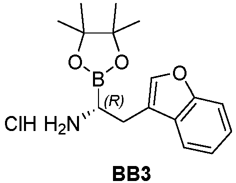

(*R*)-*N*-((*R*)-2-(Benzofuran-3-yl)-1-(4,4,5,5-tetramethyl-1,3,2-dioxaborolan-2-yl)ethyl)-2 -methylpropane-2-sulfinamide (1.3 g, 3.32 mmol) was dissolved in methanol (5 mL), and then added with a solution of hydrogen chloride in dioxane (4 N, 1 mL). The reaction solution was stirred at room temperature for 1 h of reaction. After completion of reaction, the reaction solution was concentrated under reduced pressure and the resulting crude product was pulped in tert-butyl methyl ether (15 mL) to obtain a yellow solid (403 mg, 37%).

LC-MS m/z: 288.0 [M+1]⁺.

¹HNMR (400 MHz, DMSO-d6): δ 8.07 (brs, 3H), 7.85 (s, 1H), 7.73 (d, *J* = 7.6 Hz, 1H), 7.56 (d, *J* = 8.0 Hz, 1H), 7.35 -7.26 (m, 2H), 3.11 - 2.98 (m, 3H), 1.17 (s, 6H), 1.15 (s, 6H).

### Key intermediate BB4: (R)-2-([1,1'-biphenyl]-4-yl)-1-(4,4,5,5-tetramethyl-1,3,2-dioxaborolan-2-yl)ethan-1-amine hydrochloride

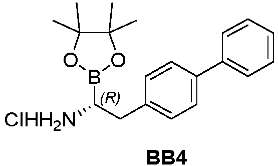

The procedure was carried out as in fragment BB2, where the corresponding 2-(p-tolyl)ethan-1-ol was replaced with 2-([1,1'-biphenyl]-4-yl)ethan-1-ol to obtain the title compound.

LC-MS m/z: 324.2 [M+1]⁺.

¹HNMR (400 MHz, DMSO-d6) δ 7.97 (s, 3H), 7.69 - 7.58 (m, 4H), 7.51 - 7.43 (m, 2H), 7.41 - 7.29 (m, 3H), 3.10 - 2.85 (m, 3H), 1.19 (d, *J* = 13.0 Hz, 12H).

### Key intermediate BB5: (R)-(1-Amino-3-methylbutyl)boronic acid pinacol ester hydrochloride

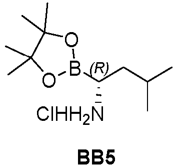

(*R*)-(1-Amino-3-methylbutyl)boronic acid pinacol ester hydrochloride (BB5) is a commercially available fragment, which was purchased from Shanghai Bide Pharmatech Co., Ltd.

### Key intermediate BB6: (R)-1-amino-3-methylbutyl pinanediol boric acid ester trifluoroacetate

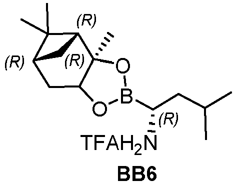

(R)-1-Amino-3-methylbutyl pinanediol boric acid ester trifluoroacetate (BB6) is a commercially available fragment, which was purchased from Shanghai Bide Pharmatech Co., Ltd.

### Example 1 (Implementation Method I): ((1R)-1-(2-methyl-3-oxo-3-(((6-phenylpyridin-2-yl)methyl)amino)propionylamino)-2-phenylethy l)boric acid

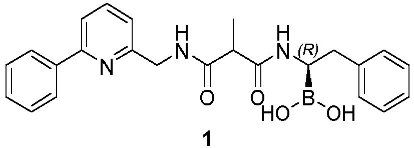

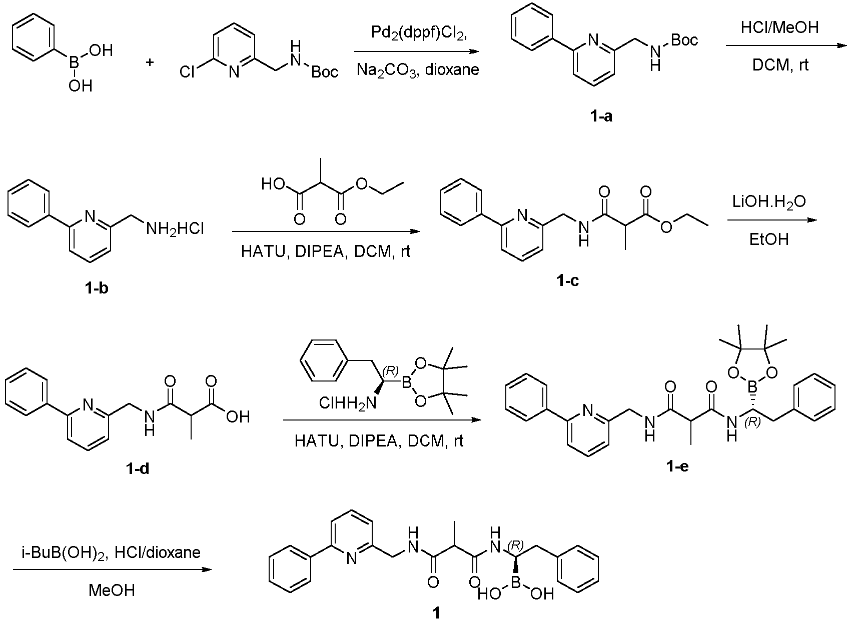

### Implementation Method I: Implementation steps:

### Step 1: tert-butyl ((6-phenylpyridin-2-yl)methyl)carbamate

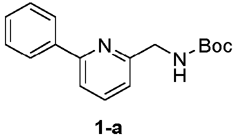

To a solution of tert-butyl ((6-chloropyridin-2-yl)methyl) carbamate (800 mg, 3.30 mmol), phenyl boric acid (600 mg, 4.92 mmol), and sodium carbonate (870 mg, 8.20 mmol) in 1,4-dioxane (8 mL) and water (1 mL) was added bis(ferrocene) dichloride palladium (241 mg, 0.33 mmol). The atmosphere was replaced with nitrogen three times and the reaction system was heated to 80°C for 18 h of reaction. After completion of reaction, the reaction solution was extracted by the addition of water (60 mL) and ethyl acetate (60 mL). The organic phases were combined, dried over anhydrous sodium sulfate, filtered and concentrated under reduced pressure. The resulting crude product was separated and purified by preparative thin-layer chromatography (an eluent of 50% ethyl acetate in petroleum ether solution) to obtain a brown solid (800 mg, 85%).

LC-MS m/z: 285.0 [M+1]⁺.

### Step 2: (6-phenylpyridin-2-yl)methylamine hydrochloride

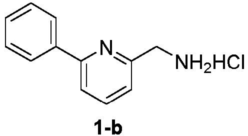

To a solution of tert-butyl ((6-phenylpyridin-2-yl)methyl)carbamate (800 mg, 2.81 mmol) in methanol (8 mL) was added a solution of hydrogen chloride in dioxane (6 mL, 4.0 M). The reaction solution was stirred and reacted at room temperature for 4 h. After completion of reaction, the reaction solution was concentrated under reduced pressure to obtain a yellow solid (600 mg). The resulting crude product was directly used in the next step of the reaction without further purification.

LC-MS m/z: 185.1 [M+1]⁺.

### Step 3: Ethyl 2-methyl-3-oxo-3-(((6-phenylpyridin-2-yl)methyl)amino)propanoate

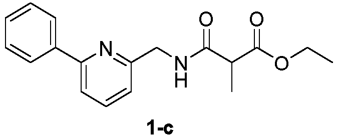

Diisopropylethylamine (795 mg, 6.16 mmol) was added dropwise to a solution of (6-phenylpyridin-2-yl)methylamine hydrochloride (300 mg, 2.05 mmol), monoethyl 2-methylmalonate (415 mg, 2.26 mmol) and HATU (860 mg, 2.26 mmol) in dichloromethane (5 mL). The reaction mixture was stirred under an ice bath for 1 h, then slowly heated to room temperature and stirred overnight. After completion of reaction, the reaction solution was diluted with water (10 mL) and extracted with dichloromethane (5 mL×2). The combined organic layers were washed with a saturated aqueous solution of sodium chloride (10 mL), dried over anhydrous sodium sulfate, filtered and concentrated to obtain a crude product as yellow oil (800 mg).

### Step 4: 2-methyl-3-oxo-3-(((6-phenylpyridin-2-yl)methyl)amino)propanoic acid

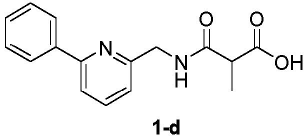

To a solution of ethyl 2-methyl-3-oxo-3-(((6-phenylpyridin-2-yl)methyl)amino)propanoate (600 mg, 1.92 mmol) in ethanol (10 mL) was added lithium hydroxide monohydrate (242 mg, 5.77 mmol). The reaction mixture was stirred and reacted at room temperature for 3 h. After completion of reaction, it was concentrated under reduced pressure, and dissolved in water (10 mL). After pH was adjusted with dilute hydrochloric acid (1 N) to approximate 5-6, the reaction mixture was extracted with dichloromethane (5 mL×2). The organic phases were combined, then washed with a saturated aqueous solution of sodium chloride (10 mL), dried over anhydrous sodium sulfate, filtered and concentrated to obtain a white solid (300 mg, 55%).

### Step 5: 2-methyl-N¹-((R)-2-phenyl-1-(4,4,5,5-tetramethyl-1,3,2-dioxaborolan-2-yl)ethyl)-N³-((6-phenylp yridin-2-yl)methyl)propanediamide

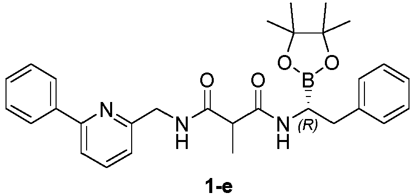

To a solution of 2-methyl-3-oxo-3-(((6-phenylpyridin-2-yl)methyl)amino)propanoic acid (50 mg, 0.18 mmol), (*R*)-2-phenyl-1-(4,4,5,5-tetramethyl-1,3,2-dioxaborolan-2-yl)ethan-1-amine hydrochloride (55 mg, 0.19 mmol) (BB1 fragment) and HATU (2-(7-azobenzotriazole)-N,N,N',N'-tetramethyluronium hexafluorophosphate (80 mg, 0.21 mmol) in dichloromethane (1 mL) was added diisopropylethylamine (57 mg, 0.44 mmol). The reaction mixture was stirred and reacted under an ice bath for 2 h, and then slowly heated to room temperature overnight. After completion of reaction, the reaction solution was diluted with water (3 mL) and extracted with dichloromethane (3 mL×2). The combined organic layers were washed with a saturated aqueous solution of sodium chloride (3 mL), dried over anhydrous sodium sulfate, filtered and concentrated to obtain a crude product as yellow oil (90 mg).

LC-MS m/z: 514.0 [M+1]⁺.

### Step 6: ((1R)-1-(2-methyl-3-oxo-3-(((6-phenylpyridin-2-yl)methyl)amino) propionylamino)-2-phenylethyl)boric acid

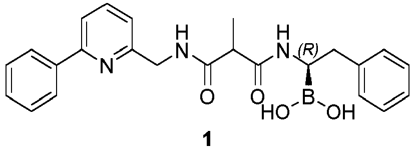

2-Methyl-*N*¹-((*R*)-2-phenyl-1-(4,4,5,5-tetramethyl-1,3,2-dioxaborolan-2-yl)ethyl)-*N*³-((6 -phenylpyridin-2- yl)methyl)malonamide (90 mg, 0.18 mmol) and isobutyl boric acid (53.6 mg, 0.53 mmol) were dissolved in methanol (1 mL). The reaction solution was then added with a solution of hydrogen chloride in methanol (0.13 mL, 4 M, 0.35 mmol), stirred and reacted for 3 h. After completion of reaction, the reaction solution was washed with petroleum ether (2 mL×2). The methanol layer was concentrated, and the resulting crude product was separated and purified by preparative thin-layer chromatography (a chromatography eluent of 9% methanol in dichloromethane) to obtain a white solid (12 mg, 16%).

LC-MS m/z: 413.8 [M-17]⁺.

### Example 2 (Implementation Method II): (R,E)-(1-(4-(benzylamino)-4-oxobut-2-enamido)-2-phenylethyl)boric acid

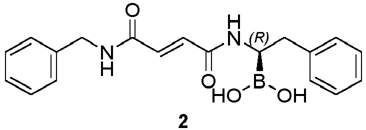

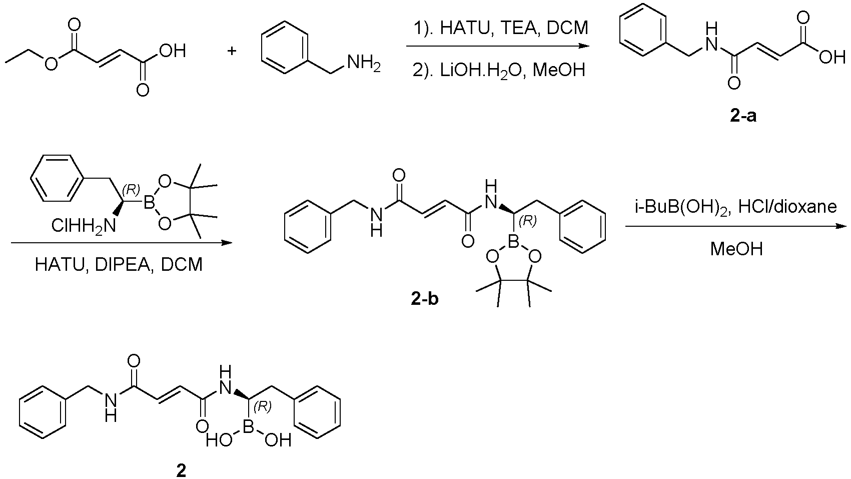

### Implementation method II: Implementation steps:

### Step 1: N-benzyl fumaric acid monoamide

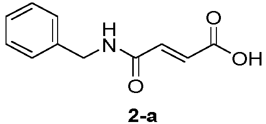

To a solution of monoethyl fumarate (2.12 g, 14.7 mmol), benzylamine (1.50 g, 14.0 mmol) and diisopropylethylamine (5.43 g, 42.0 mmol) in dichloromethane (45 mL) was added HATU (5.59 g, 14.7 mmol). The reaction mixture was stirred and reacted at room temperature for 9 h. After completion of reaction, the reaction solution was diluted with water (100 mL) and extracted with dichloromethane (80 mL×2). The combined organic layers were washed with a saturated aqueous solution of sodium chloride (60 mL), dried over anhydrous sodium sulfate, filtered and concentrated. The residue was separated and purified by column chromatography (an eluent of 25% ethyl acetate in petroleum ether) to obtain a white solid (3.05 g, 94%). This white solid (3.05 g, 13.1 mmol) was then dissolved in ethanol (30 mL) and added with lithium hydroxide monohydrate (1.65 g, 39.3 mmol). The reaction solution was stirred and reacted at room temperature for 11 h. After completion of reaction, the reaction solution was concentrated under reduced pressure. The residue was dissolved in water (30 mL), then added with a solution of hydrogen chloride in dioxane (12 mL, 4 M, 48.0 mmol) under an ice bath, and stirred for 10 min to produce a white solid. The resulting white solid was filtered, washed with water (50 mL) and dried under reduced pressure to obtain a white solid (2.71 g, 94%).

LC-MS m/z: 206.1 [M+1]⁺.

### Step 2: (R)-N¹-benzyl-N⁴-(2-phenyl-1-(4,4,5,5-tetramethyl-1,3,2-dioxaborolan-2-yl)ethyl)fumaramide

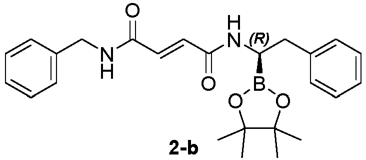

To a solution of *N*-benzyl fumaric acid monoamide (90 mg, 440 µmol), (*R*)-2-phenyl-1-(4,4,5,5-tetramethyl-1,3,2-dioxaborolan-2-yl)ethan-1-amine hydrochloride (boric acid fragment BB1) (126 mg, 440 µmol), and diisopropylethylamine (113 mg , 880 µmol) in dichloromethane (10 mL) was added HATU (200 mg, 530 µmol). The reaction mixture was stirred and reacted under an ice bath for 9h. After completion of reaction, the reaction solution was diluted with water (20 mL) and extracted with dichloromethane (20 mL×2). The combined organic layers were washed with a saturated aqueous solution of sodium chloride (20 mL), dried over anhydrous sodium sulfate, filtered and concentrated to obtain a brown solid (110 mg, 57%).

### Step 3: (R,E)-(1-(4-(benzylamino)-4-oxobut-2-enamido)-2-phenylethyl)boric acid

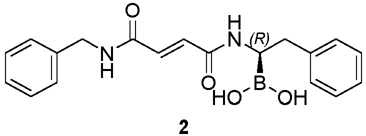

(*R*)-*N*¹-benzyl-*N*⁴-(2-phenyl-1-(4,4,5,5-tetramethyl-1,3,2-dioxaborolan-2-yl)ethyl)fumar amide (110 mg, 250 µmol) and isobutyl boric acid (77 mg, 750 µmol) were dissolved in methanol (3 mL). The reaction solution was then added with a solution of hydrogen chloride in dioxane (0.31 mL, 4 M, 1.24 mmol), stirred and reacted at room temperature for 3 h. After completion of reaction, the reaction solution was concentrated under reduced pressure, then diluted with water (20 mL) and extracted with dichloromethane (20 mL×2). The combined organic layers were washed with a saturated aqueous solution of sodium chloride (20 mL), dried over anhydrous sodium sulfate, filtered and concentrated. The resulting crude product was separated and purified by preparative thin-layer chromatography (a chromatography eluent of 9% methanol in dichloromethane) to obtain a white solid (40 mg, 45%).

LC-MS m/z: 335.0 [M-17]⁺.

¹HNMR (400 MHz, DMSO-d6) δ 9.32 (s, 1H), 8.95 (t, *J* = 6.0 Hz, 1H), 7.34 - 7.11 (m, 10H), 6.97 (q, *J* = 15.3 Hz, 2H), 4.37 (d, *J* = 5.9 Hz, 2H), 2.96 - 2.87 (m, 1H), 2.81 (dd, *J* = 14.3, 5.1 Hz, 1H), 2.59 - 2.52 (m, 1H).

### Example 3 (Implementation Method III): ((1R)-1-(2-(cyclopropylmethyl)-3-((3-methoxybenzyl)amino)-3-oxopropionylamimo)-2-phenylet hyl)boric acid

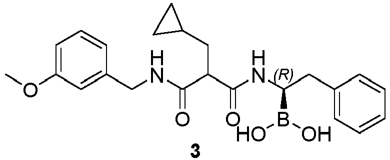

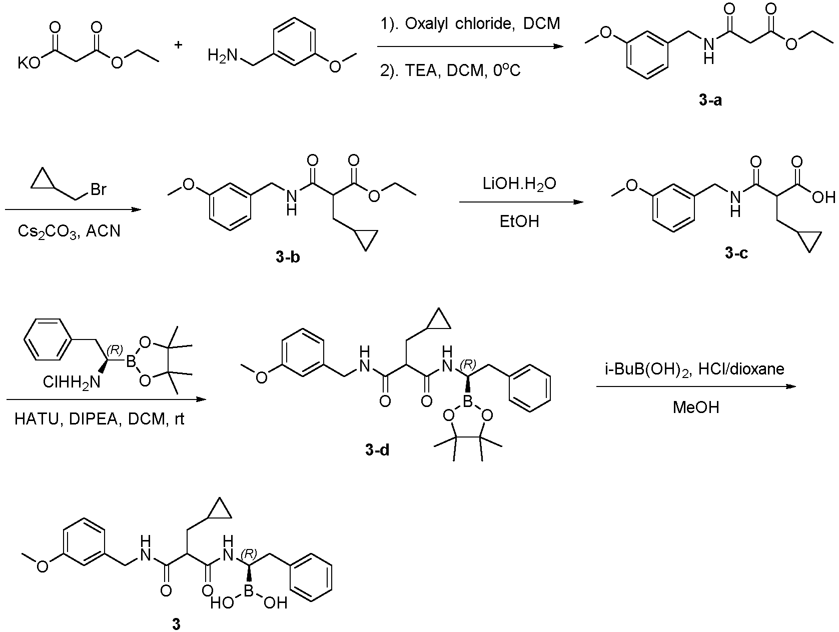

### Implementation Method 3: Implementation steps:

### Step 1: Ethyl 3-((3-methoxybenzyl) amino)-3-oxopropionate

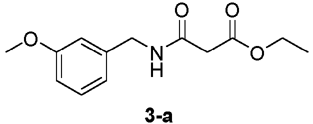

To a solution of monoethyl malonate potassium salt (3.00 g, 17.6 mmol) in dichloromethane (30 mL) was dropwise added ethyl oxalyl monochloride (2.70 g, 53.7 mmol) and a drop of N,N-dimethylformamide under a condition of ice bath. The reaction mixture was stirred and reacted at room temperature for 1 h. The reaction solution was filtered. The filtrate was concentrated under reduced pressure, and then diluted with dichloromethane (30 mL). 3-methoxybenzylamine (2.42 g, 17.6 mmol) and triethylamine (3.7 mL, 26.5 mmol) were added dropwise to the reaction solution under an ice bath. The reaction mixture was stirred and reacted under an ice bath for 1 h. After completion of reaction, the reaction solution was diluted with water (200 mL) and extracted with dichloromethane (150 mL×2). The combined organic layers were washed with a saturated aqueous solution of sodium chloride (160 mL), dried over anhydrous sodium sulfate, filtered and concentrated. The crude product was separated and purified by column chromatography (an eluent of 25% ethyl acetate in petroleum ether solution) to obtain a colorless liquid (2.73 g, 62%).

LC-MS m/z: 252.1 [M+1]⁺.

### Step 2: Ethyl 2-(cyclopropylmethyl)-3-((3-methoxybenzyl)amino)-3-oxopropanoate

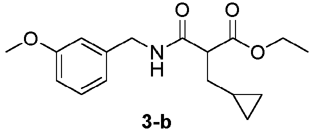

To a solution of ethyl 3-((3-methoxybenzyl)amino)-3-oxopropanoate (700 mg, 2.79 mmol) and bromomethylcyclopropane (409 mg, 3.01 mmol) in acetonitrile (10 mL) was added cesium carbonate (1.82 g, 5.57 mmol). The reaction mixture was stirred and reacted at room temperature for 16 h. After completion of reaction, the reaction solution was diluted with water (20 mL) and extracted with dichloromethane (20 mL×2). The combined organic layers were washed with a saturated aqueous solution of sodium chloride (20 mL), dried over anhydrous sodium sulfate, filtered and concentrated. The resulting crude product was separated and purified by column chromatography (an eluent of 25% ethyl acetate in petroleum ether solution) to obtain a colorless liquid (530 mg, 62%).

LC-MS m/z: 306.1 [M+1]⁺.

### Step 3: 2-(cyclopropylmethyl)-3-((3-methoxybenzyl)amino)-3-oxopropanoic acid

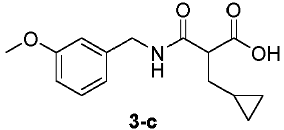

To a solution of ethyl 2-(cyclopropylmethyl)-3-((3-methoxybenzyl)amino)-3-oxopropanoate (530 mg, 1.74 mmol) in ethanol (10 mL) was added lithium hydroxide monohydrate (240 mg, 5.75 mmol). The reaction mixture was stirred and reacted at room temperature for 3 h. After completion of reaction, it was concentrated under reduced pressure, dissolved in water (10 mL) and its pH was adjusted to approximate 4-5 with dilute hydrochloric acid (1 N). The reaction mixture was extracted with dichloromethane (5 mL×2). The organic phases were combined, washed with a saturated aqueous solution of sodium chloride (10 mL), dried over anhydrous sodium sulfate, filtered, and concentrated to obtain a white solid (450 mg, 93%).

### Step 4: 2-(cyclopropylmethyl)-N¹-(3-methoxybenzyl)-N³-((R)-2-phenyl-1-(4,4,5,5-tetramethyl-1,3,2-diox aborolan-2-yl)ethyl)malonamide

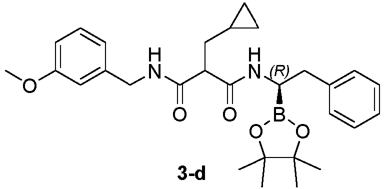

To a solution of 2-(cyclopropylmethyl)-3-((3-methoxybenzyl)amino)-3-oxopropanoic acid (100 mg, 0.38 mmol), (*R*)-2-phenyl-1-(4,4,5,5-tetramethyl-1,3,2-dioxaborolan-2-yl)ethan-1-amine hydrochloride (94 mg, 0.38 mmol) (fragment BB1) and HATU (159 mg , 0.42 mmol) in dichloromethane (5 mL) was added diisopropylethylamine (147 mg, 1.14 mmol). The reaction mixture was stirred and reacted under an ice bath for 2 h and then slowly heated to room temperature overnight. After completion of reaction, the reaction solution was diluted with water (10 mL) and extracted with dichloromethane (10 mL×2). The combined organic layers were washed with a saturated aqueous solution of sodium chloride (10 mL), dried over anhydrous sodium sulfate, filtered and concentrated to obtain a crude product as a yellow oil (150 mg, 62%).

LC-MS m/z: 507.2 [M+1]⁺.

### Step 5: ((1R)-1-(2-(cyclopropylmethyl)-3-((3-methoxybenzyl)amino)-3-oxopropionamido)-2-phenylethyl )boric acid

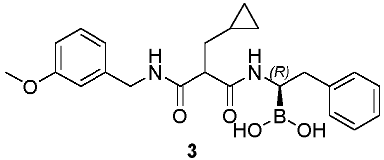

2-(Cyclopropylmethyl)-*N*¹-(3-methoxybenzyl)-*N*³-((*R*)-2-phenyl-1-(4,4,5,5-tetramethyl-1,3,2-dioxaborolan-2-yl)ethyl)malonamide (150 mg, 256 µmol) and isobutyl boric acid (78 mg, 768 µmol) were dissolved in methanol (3 mL), then a solution of hydrogen chloride in dioxane (0.26 mL, 4 M, 1.0 mmol) was added. The reaction solution was stirred and reacted at room temperature for 3 h. After completion of reaction, the reaction solution was concentrated under reduced pressure, then diluted with water (20 mL) and extracted with dichloromethane (20 mL×2). The combined organic layers were washed with a saturated aqueous solution of sodium chloride (20 mL), dried over anhydrous sodium sulfate, filtered and concentrated. The crude product was separated and purified by preparative reversed-phase chromatography (a mobile phase: 60% aqueous solution of acetonitrile) to obtain a white solid (39 mg, 31%).

LC-MS m/z: 407.2 [M-17]⁺.

### Example 4: (R)-(1-(3-(benzylamino)-2,2-dimethyl-3-oxopropionamido)-2-phenylethyl)boric acid

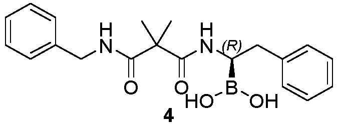

The implementation method was the same as method I, where (6-phenylpyridine-2-yl) methylamine hydrochloride was replaced by corresponding benzylamine as a starting material, and monoethyl methyl malonate was replaced by corresponding monoethyl dimethyl malonate to obtain the title compound.

LC-MS m/z: 351.0 [M-17]⁺.

¹HNMR (400 MHz, DMSO-d6) δ 8.50 (d, *J* = 2.8 Hz, 1H), 8.18 (t, *J* = 5.9 Hz, 1H), 7.29 - 7.24 (m, 2H), 7.21 - 7.09 (m, 8H), 4.24 (d, *J* = 5.8 Hz, 2H), 2.81 (dd, *J* = 13.2, 6.4 Hz, 1H), 2.71 (d, *J* = 3.0 Hz, 1H), 2.59 (dd, *J* = 13.2, 6.3 Hz, 1H), 1.31 (d, *J* = 8.4 Hz, 6H).

### Example 5: (R)-(2-phenyl-1-(1-(phenylcarbamoyl)cyclopropane-1-formamido)ethyl)boric acid

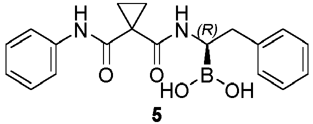

The implementation method was the same as method I, where (6-phenylpyridin-2-yl)methanamine hydrochloride was replaced by the corresponding aniline as a starting material, and monoethyl methyl malonate was replaced by monomethyl 1,1-cyclopropyl diformate to obtain the title compound.

LC-MS m/z: 335.0 [M-17]⁺.

### Example 6: (R)-(1-(1-(benzylcarbamoyl)cyclopropane-1-formamido)-2-phenylethyl) boric acid

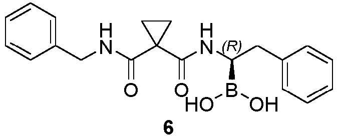

The implementation method was the same as method I, where (6-phenylpyridin-2-yl)methanamine hydrochloride was replaced by the corresponding benzylamine as a starting material, and monoethyl methyl malonate was replaced by monomethyl 1,1-cyclopropyl diformate to obtain the title compound.

LC-MS m/z: 349.0 [M-17]⁺.

¹HNMR (500 MHz, DMSO-d6) δ 9.06 (s, 1H), 8.41 (t, *J* = 5.7 Hz, 1H), 7.27 (dd, *J* = 13.7, 6.5 Hz, 2H), 7.22 - 7.13 (m, 8H), 4.28 - 4.21 (m, 2H), 2.75 (d, *J* = 10.0 Hz, 2H), 2.62 - 2.54 (m, 1H), 1.46 - 1.27 (m, 4H).

### Example 7: (R)-(1-(3-(benzylcarbamoyl)ureido)-2-phenylethyl)boric acid

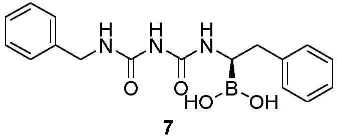

### Implementation Method:

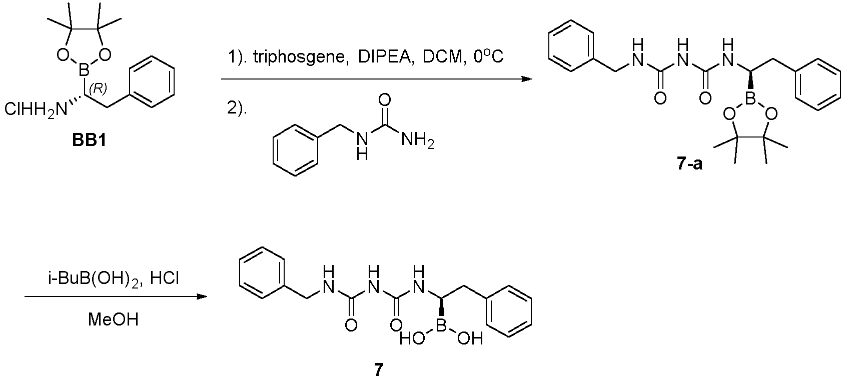

### Implementation steps:

### Step 1: (R)-N¹-(benzylcarbamoyl)-N³-2-phenyl-1-(4,4,5,5-tetramethyl-1,3,2-dioxaborolan-2-yl)ethylurea

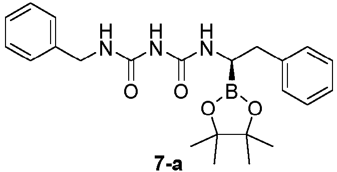

To a solution of (*R*)-2-phenyl-1-(4,4,5,5-tetramethyl-1,3,2-dioxaborolan-2-yl)ethan-1-amine hydrochloride (80 mg, 282 µmol) (fragment BB1) and triphosgene (98 mg, 330 µmol) in dichloromethane (10 mL) was dropwise added diisopropylethylamine (7.30 g, 53.7 mmol) under a condition of ice bath. The reaction mixture was stirred and reacted under an ice bath for half an hour, and then the reaction solution was concentrated under reduced pressure. The residue was dissolved in dichloromethane (10 mL) and benzylurea (47 mg, 310 µmol) was added. The reaction solution was stirred at room temperature for 2 h. After completion of reaction, the reaction solution was diluted with water (15 mL) and extracted with dichloromethane (10 mL×2). The combined organic layers were washed with a saturated aqueous solution of sodium chloride (20 mL), dried over anhydrous sodium sulfate, filtered and concentrated to obtain a yellow oil (80 mg, 67%).

LC-MS m/z: 424.1 [M+1]⁺.

### Step 2: (R)-(1-(3-(benzylcarbamoyl)ureido)-2-phenylethyl)boric acid

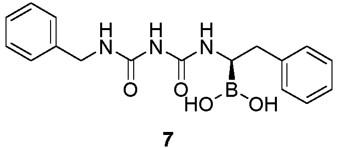

(*R*)-*N*¹-(benzylcarbamoyl)-*N*³-2-phenyl-1-(4,4,5,5-tetramethyl-1,3,2-dioxaborolan-2-yl) ethyl urea (85 mg, 189 µmol) and isobutyl boric acid (57 mg, 560 µmol) were dissolved in methanol (3 mL), and then a solution of hydrogen chloride in dioxane (0.23 mL, 4 M, 933 µmol) was added to the reaction solution. The reaction solution was stirred and reacted at room temperature for 2 h. After completion of reaction, the reaction solution was concentrated under reduced pressure and then diluted with n-hexane (10 mL) and methanol (10 mL). The methanol phase was concentrated and the crude product was separated and purified by preparative thin-layer chromatography (a chromatographic eluent of 9% methanol in dichloromethane) to obtain a white solid (3 mg, 5%).

LC-MS m/z: 324.0 [M-17]⁺.

### Example 8: (R, E)-(1-(4-oxo-4-(phenylamino))butyl-2-enamido)-2-phenylethyl)boric acid

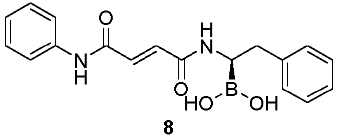

The implementation method was the same as method II, where benzylamine was replaced by the corresponding aniline as a starting material to obtain the title compound.

LC-MS m/z: 321.0 [M-17]⁺.

¹HNMR (500 MHz, DMSO-d6) δ 10.54 (s, 1H), 9.43 (s, 1H), 7.68 (d, *J* = 7.2 Hz, 2H), 7.24 (tdd, *J* = 36.5, 14.3, 7.3 Hz, 10H), 7.11 - 6.96 (m, 2H), 2.99 (s, 1H), 2.86 (dd, *J* = 14.3, 4.7 Hz, 1H), 2.60 (dd, *J* = 14.2, 9.6 Hz, 1H).

### Example 9: ((1R)-2-(benzofuran-3-yl)-1-(3-(3-methoxybenzyl)amino)-2-methyl-3-oxopropionamido)ethyl)bo ric acid

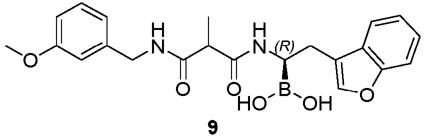

The implementation method was the same as method I, where (6-phenylpyridine-2-yl)methylamine hydrochloride was replaced by the m-methoxybenzylamine as a starting material, and fragment BB1 was replaced by fragment BB3 to obtain the title compound.

LC-MS m/z: 407.1 [M-17]⁺.

### Example 10: (R,E)-(1-(4-((4-methylbenzyl)amino))-4-oxobut-2-enamido)-2-(p-tolyl)ethyl)boric acid

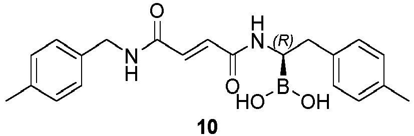

The implementation method was the same as method II, where benzylamine was replaced by the corresponding p-methylbenzylamine as a starting material, and fragment BB1 is replaced by fragment BB2 to obtain the title compound.

LC-MS m/z: 363.1 [M-17]⁺.

¹HNMR (400 MHz, DMSO-d6) δ 9.27 (s, 1H), 8.89 (t, *J* = 5.4 Hz, 1H), 7.12 (s, 5H), 7.04 (dd, *J* = 15.6, 8.2 Hz, 5H), 6.95 (d, *J* = 13.1 Hz, 2H), 4.31 (d, *J* = 5.4 Hz, 2H), 2.87 (s, 1H), 2.74 (d, *J* = 14.3 Hz, 2H), 2.27 (s, 3H), 2.22 (s, 3H).

### Example 11: (R,E)-(3-methyl-1-(4-((4-methylbenzyl)amino))-4-oxobut-2-enamido)butyl)boric acid

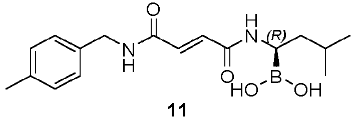

The implementation method was the same as method II, where benzylamine was replaced by the corresponding p-methylbenzylamine as a starting material, and fragment BB1 was replaced by fragment BB5 to obtain the title compound.

LC-MS m/z: 315.1 [M-17]⁺.

¹HNMR (400 MHz, DMSO-d6) δ 9.30 (s, 1H), 8.88 (s, 1H), 7.13 (s, 4H), 6.93 (d, *J* = 13.6 Hz, 2H), 4.32 (s, 2H), 2.70 (s, 1H), 2.27 (s, 3H), 1.59 (s, 1H), 1.24 (s, 2H), 0.83 (s, 6H).

### Example 12: (R,E)-(1-(4-((2,5-dichlorobenzyl)amino))-4-oxobut-2-enamido)-2-phenylethyl)boric acid

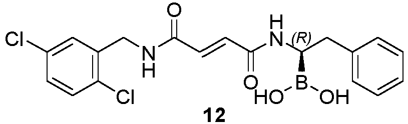

The implementation method was the same as method II, where benzylamine was replaced by the corresponding 2,4-dichlorobenzylamine as a starting material to obtain the title compound.

LC-MS m/z: 402.9 [M-17]⁺.

### Example 13: (R,E)-(3-methyl-1-(4-oxo-4-(((6-phenylpyridin-2-yl)methyl)amino)but-2-enamido)butyl)boric acid

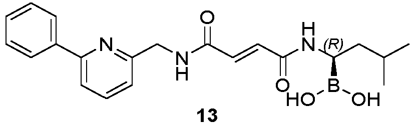

The implementation method was the same as method II, where benzylamine was replaced by the corresponding (6-phenylpyridin-2-yl)methylamine hydrochloride as a starting material, and fragment BB1 was replaced by fragment BB5 to obtain the title compound.

LC-MS m/z: 378.0 [M-17]⁺.

### Example 14: (R,E)-(1-(4-oxo-4-(((6-phenylpyridin-2-yl)methyl)amino)but-2-enamido)-2-phenylethyl)boric acid

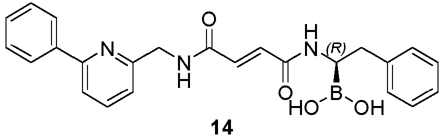

The implementation method was the same as method II, where benzylamine was replaced by the corresponding (6-phenylpyridin-2-yl)methylamine hydrochloride as a starting material to obtain the title compound.

LC-MS m/z: 412.1 [M-17]⁺.

¹HNMR (400 MHz, DMSO-d6) δ 9.33 (s, 1H), 9.08 (s, 1H), 8.06 (d, *J* = 7.2 Hz, 2H), 7.80 (d, *J* = 3.9 Hz, 2H), 7.52 - 7.31 (m, 3H), 7.21 (s, 1H), 7.03 (d, *J* = 18.0 Hz, 1H), 6.92 (d, *J* = 15.1 Hz, 1H), 4.52 (d, *J* = 5.4 Hz, 2H), 2.70 (s, 1H), 1.59 (s, 1H), 1.29 (d, *J* = 18.9 Hz, 2H), 0.81 (s, 6H).

### Example 15: (R,E)-(1-(4-((4-methylbenzyl)amino))-4-oxobut-2-enamido)-2-phenylethyl)boric acid

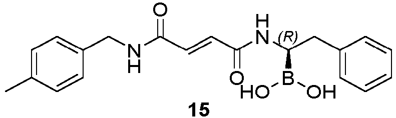

The implementation method was the same as method II, where benzylamine was replaced by the corresponding p-methylbenzylamine as a starting material to obtain the title compound.

LC-MS m/z: 349.0 [M-17]⁺.

### Example 16: (R)-(1-(4-(benzylamino)-4-oxobutanoylamino)-2-phenylethyl)boric acid

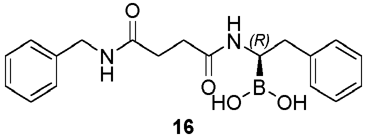

The implementation method was the same as method II, where monoethyl fumarate was replaced by the corresponding monoethyl p-succinate as a starting material to obtain the title compound.

LC-MS m/z: 337.0 [M-17]⁺.

¹HNMR (500 MHz, DMSO-d6) δ 8.60 (d, *J* = 3.4 Hz, 1H), 8.37 (t, *J* = 5.9 Hz, 1H), 7.33 - 7.08 (m, 10H), 4.24 (t, *J* = 6.6 Hz, 2H), 2.83 - 2.69 (m, 2H), 2.52 (d, *J* = 9.8 Hz, 1H), 2.49 - 2.32 (m, 4H).

### Example 17: (R,E)-(1-(4-(benzylamino)-4-oxobut-2-enamido-2,3-D2)-2-phenylethyl)boric acid

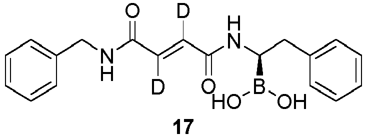

The implementation method was the same as method II, where monoethyl fumarate was replaced by the corresponding deuterated malonic acid as a starting material to obtain the title compound.

LC-MS m/z: 337.1 [M-17]⁺.

### Example 18: (R,E)-(1-(4-((cyclohexylmethyl)amino)-4-oxobut-2-enamido)-3-methylbutyl)boric acid

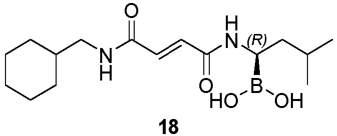

The implementation method was the same as method II, where benzylamine was replaced by the corresponding cyclohexylmethylamine as a starting material, and fragment BB1 was replaced by the corresponding fragment BB6 to obtain the title compound.

LC-MS m/z: 307.1 [M-17]+.

### Example 19: ((R)-1-((1R,2R)-2-(benzylcarbamoyl)cyclopropyl)-1-formamido)-2-phenylethyl)boric acid

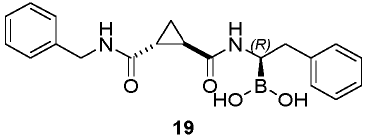

The implementation method was the same as method II, where monoethyl fumarate was replaced by the corresponding monoethyl (1R,2R)-cyclopropane-1,2-dicarboxylate as a starting material to obtain the title compound.

LC-MS m/z: 349.1 [M-17]⁺.

### Example 20: (R,E)-(1-(4-((4-methoxybenzyl)amino)-4-oxobut-2-enamido)-3-methylbutyl)boric acid

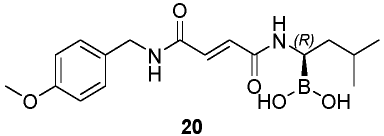

The implementation method was the same as method II, where benzylamine was replaced by the corresponding p-methoxybenzylamine as a starting material, and fragment BB1 was replaced by the corresponding fragment BB6 to obtain the title compound.

LC-MS m/z: 331.1 [M-17]⁺.

### Example 21: (R,E)-(1-(4-((3-methoxybenzyl)amino)-4-oxobut-2-enamido)-3-methylbutyl)boric acid

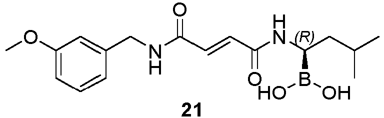

The implementation method was the same as method II, where benzylamine was replaced by the corresponding m-methoxybenzylamine as a starting material, and fragment BB1 was replaced by the corresponding fragment BB6 to obtain the title compound.

LC-MS m/z: 331.1 [M-17]⁺.

### Example 22: (R)-(1-(3-(([1,1'-biphenyl]-3-ylmethyl)amino)-3-oxopropionamido)-2-(benzofuran-3-yl)ethyl)bor ic acid

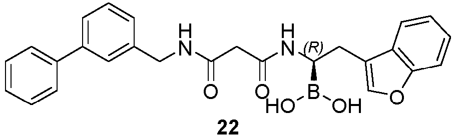

The implementation method was the same as method I, where monoethyl 2-methylmalonate was replaced by the corresponding monoethyl malonate as a starting material, (6-phenylpyridin-2-yl)methylamine hydrochloride was replaced by [1,1'-biphenyl]-3-ylmethylamine, and fragment BB1 was replaced by the fragment BB3 to obtain the title compound.

LC-MS m/z: 439.1 [M-17]⁺.

### Example 23: ((R)-1-((1R,2R)-2-(benzylcarbamoyl)cyclopropane-1-formamido)-3-methylbutyl)boric acid

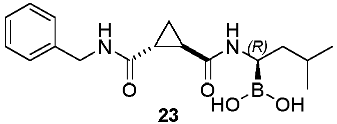

The implementation method was the same as method II, where monoethyl fumarate was replaced by the corresponding monoethyl (1*R*,2*R*)-cyclopropane-1,2-dicarboxylate as a starting material, and fragment BB1 was replaced by the corresponding fragment BB6 to obtain the title compound.

LC-MS m/z: 315.1 [M-17]⁺.

### Example 24: (R,E)-(1-(4-(benzylamino)-4-oxobut-2-enamido)-2-(p-tolyl)ethyl)boronic acid

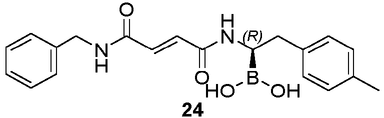

The implementation method was the same as method II, where fragment BB1 was replaced by the corresponding fragment BB2 to obtain the title compound.

LC-MS m/z: 349.1 [M-17]⁺.

### Example 25: (R,E)-(1-(4-(benzylamino)-4-oxobut-2-enamido)-3-methylbutyl)boric acid

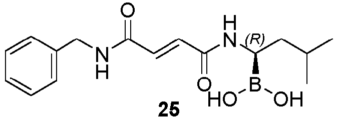

The implementation method was the same as method II, where fragment BB1 was replaced by the corresponding fragment BB6 to obtain the title compound.

LC-MS m/z: 301.1 [M-17]⁺.

### Example 26: (R,E)-(1-(4-oxo-4-(phenylamino)but-2-enamido)-2-(p-tolyl)ethyl)boric acid

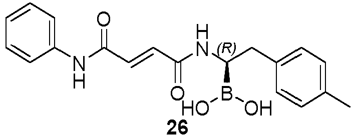

The implementation method was the same as method II, where benzylamine was replaced by the corresponding aniline as a starting material, and fragment BB1 was replaced by the corresponding fragment BB2 to obtain the title compound.

LC-MS m/z: 335.1 [M-17]⁺.

### Example 27: (R)-(1-(3-(([1,1'-biphenyl]-3-ylmethyl)amino)-3-oxopropionamido)-2-(p-tolyl)ethyl)boric acid

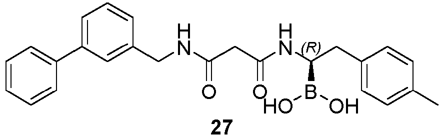

The implementation method was the same as method I, where monoethyl 2-methylmalonate was replaced by the corresponding monoethyl malonate as a starting material, (6-phenylpyridin-2-yl)methylamine hydrochloride was replaced by [1,1'-biphenyl]-3-ylmethylamine, and fragment BB1 was replaced by the fragment BB2 to obtain the title compound.

LC-MS m/z: 413.1 [M-17]+.

### Example 28: ((1R)-1-(3-(benzylamino)-2-methyl-3-oxopropionamido)-3-methylbutyl)boric acid

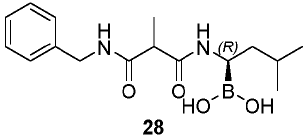

The implementation method was the same as method I, where (6-phenylpyridin-2-yl)methanamine hydrochloride was replaced by the corresponding benzylamine as a starting material, and fragment BB1 was replaced by fragment BB6 to obtain the title compound.

LC-MS m/z: 303.1 [M-17]⁺.

### Example 29: ((1R)-1-(3-(benzylamino)-2-methyl-3-oxopropionamido)-2-phenylethyl)boric acid

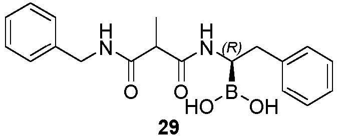

The implementation method was the same as method I, where (6-phenylpyridin-2-yl)methanamine hydrochloride was replaced by the corresponding benzylamine as a starting material to obtain the title compound.

LC-MS m/z: 337.1 [M-17]+.

### Example 30: (R,E)-(1-(4-((cyclohexylmethyl)amino)-4-oxobut-2-enamido)-2-phenylethyl)boric acid

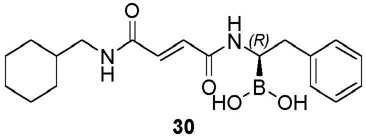

The implementation method was the same as method II, where benzylamine was replaced by the corresponding cyclohexylmethylamine as a starting material to obtain the title compound.

LC-MS m/z: 341.1 [M-17]⁺.

### Example 31: (R,E)-(1-(4-((3-methoxybenzyl)amino)-4-oxobut-2-enamido)-2-phenylethyl)boric acid

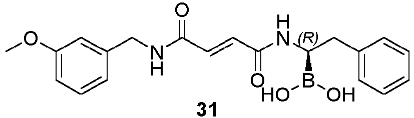

The implementation method was the same as method II, where benzylamine was replaced by the corresponding m-methoxybenzylamine as a starting material to obtain the title compound.

LC-MS m/z: 365.1 [M-17]⁺.

### Example 32: (R,E)-(1-(4-((4-methoxybenzyl)amino)-4-oxobut-2-enamido)-2-phenylethyl)boric acid

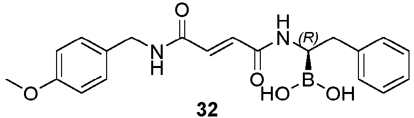

The implementation method was the same as method II, where benzylamine was replaced by the corresponding p-methoxybenzylamine as a starting material to obtain the title compound.

LC-MS m/z: 365.1 [M-17]⁺.

### Example 33: ((1R)-1-((E)-4-oxo-4-(((tetrahydrofuran-2-yl)methyl)amino)but-2-enamido)-2-phenylethyl)boric acid

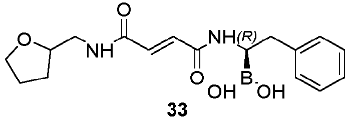

The implementation method was the same as method II, where benzylamine was replaced by the corresponding tetrahydrofurfurylamine as a starting material to obtain the title compound.

LC-MS m/z: 329.1 [M-17]+.

### Example 34: ((1R)-3-methyl-1-((E)-4-oxo-4-(((tetrahydrofuran-2-yl)methyl)amino)but-2-enamido)butyl)boric acid

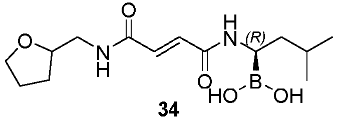

The implementation method was the same as method II, where benzylamine was replaced by the corresponding tetrahydrofurfurylamine as a starting material, and fragment BB1 was replaced by fragment BB5 to obtain the title compound.

LC-MS m/z: 295.1 [M-17]⁺.

### Example 35: (R,E)-(1-(4-(benzylamino)-2-methyl)-4-oxobut-2-enamido)-2-phenylethyl)boric acid

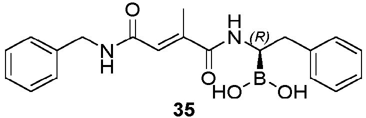

The implementation method was the same as method II, where monoethyl fumarate was replaced by the corresponding furonic acid as a starting material to obtain the title compound.

LC-MS m/z: 349.1 [M-17]+.

### Example 36: ((1R)-2-([1,1'-biphenyl]-4-yl)-1-(3-((3-methoxybenzyl)amino)-2-methyl-3-oxopropionamido)eth yl)boric acid

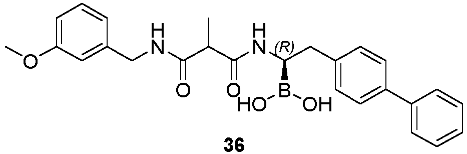

The implementation method was the same as method I, where (6-phenylpyridin-2-yl)methylamine hydrochloride was replaced by the corresponding m-methoxybenzylamine as a starting material, and fragment BB1 was replaced by fragment BB4 to obtain the title compound.

LC-MS m/z: 443.2 [M-17]⁺.

### Example 37: (R,E)-(1-(4-oxo-4-((pyrazin-2-ylmethyl)amino)but-2-enamido)-2-phenylethyl)boric acid

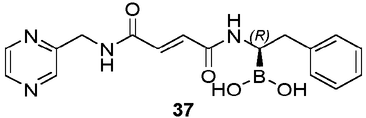

The implementation method was the same as method II, where benzylamine was replaced by the corresponding pyrazine-2-methylamine as a starting material to obtain the title compound.

LC-MS m/z: 337.1 [M-17]+.

### Example 38: ((1R)-1-(3-(benzylamino)-2-methyl-3-oxopropionamido)-2-(p-tolyl)ethyl)boric acid

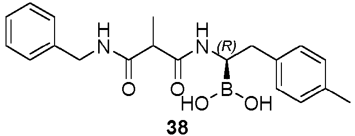

The implementation method was the same as method I, where (6-phenylpyridin-2-yl)methylamine hydrochloride was replaced by the corresponding benzylamine as a starting material, and fragment BB1 was replaced by fragment BB2 to obtain the title compound.

LC-MS m/z: 351.1 [M-17]⁺.

### Example 39: ((1R)-3-methyl-1-(2-methyl-3-oxo-3-(phenylamino)propionamido)butyl)boric acid

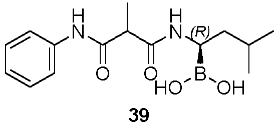

The implementation method was the same as method I, where (6-phenylpyridin-2-yl)methylamine hydrochloride was replaced by the corresponding aniline as a starting material, and fragment BB1 was replaced by fragment BB5 to obtain the title compound.

LC-MS m/z: 289.1 [M-17]+.

### Example 40: ((1R)-1-(2-methyl-3-oxo-3-(phenylamino) propionamido)-2-(p-tolyl)ethyl)boric acid

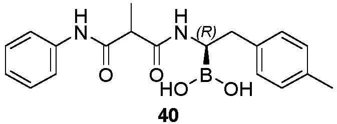

The implementation method was the same as method I, where (6-phenylpyridin-2-yl)methylamine hydrochloride was replaced by the corresponding aniline as a starting material, and fragment BB1 was replaced by fragment BB2 to obtain the title compound.

LC-MS m/z: 337.1 [M-17]⁺.

### Example 41: ((1R)-1-(3-(cyclohexylamino)-2-methyl-3-oxopropionamido)-3-methylbutyl)boric acid

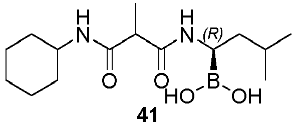

The implementation method was the same as method I, where (6-phenylpyridin-2-yl)methylamine hydrochloride was replaced by the corresponding cyclohexylamine as a starting material, and fragment BB1 was replaced by fragment BB5 to obtain the title compound.

LC-MS m/z: 295.1 [M-17]+.

### Example 42: ((1R)-1-(3-(cyclohexylamino)-2-methyl-3-oxopropionamido)-2-(p-tolyl)ethyl)boric acid

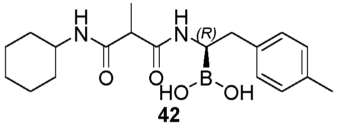

The implementation method was the same as method I, where (6-phenylpyridin-2-yl)methylamine hydrochloride was replaced by the corresponding cyclohexylamine as a starting material, and fragment BB1 was replaced by fragment BB2 to obtain the title compound.

LC-MS m/z: 343.1 [M-17]⁺.

¹HNMR (400 MHz, DMSO-d6) δ 8.10 - 7.86 (m, 1H), 7.28 (t, *J* = 5.6 Hz, 4H), 3.51 (ddd, *J* = 7.8, 5.2, 2.7 Hz, 1H), 3.31 (q, *J* = 7.1 Hz, 1H), 3.05 (dt, *J* = 13.7, 5.5 Hz, 1H), 2.93 (td, *J* = 13.2, 8.0 Hz, 1H), 2.79 - 2.75 (m, 3H), 2.08 - 1.70 (m, 5H), 1.50 (d, *J* = 9.6 Hz, 3H), 1.43 - 1.26 (m, 5H).

### Example 43: ((1R)-1-(3-((3-methoxybenzyl)amino)-2-methyl-3-oxopropionamido)-2-(p-tolyl)ethyl)boric acid

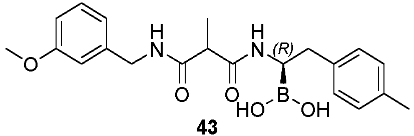

The implementation method was the same as method I, where (6-phenylpyridin-2-yl)methylamine hydrochloride was replaced by the corresponding m-methoxybenzylamine as a starting material, and fragment BB1 was replaced by fragment BB2 to obtain the title compound.

LC-MS m/z: 381.1 [M-17]⁺.

¹HNMR (400 MHz, DMSO-d6 + D₂O) δ 7.21 (t, *J* = 8.1 Hz, 1H), 6.99 (d, *J* = 4.4 Hz, 4H), 6.78 (d, *J* = 6.5 Hz, 3H), 4.21 (t, *J* = 6.1 Hz, 2H), 3.70 (s, 3H), 3.22 (d, *J* = 7.2 Hz, 1H), 3.16 (q, *J* = 7.0 Hz, 1H), 2.76 (d, *J* = 8.3 Hz, 1H), 2.67 (t, *J =* 8.4 Hz, 1H), 2.22 (s, 3H), 1.16 (d, *J =* 8.5 Hz, 3H).

### Example 44: (R)-(1-(3-(([1,1'-biphenyl]-3-ylmethyl)amino)-3-oxopropionamido)-2-phenylethyl)boronic acid

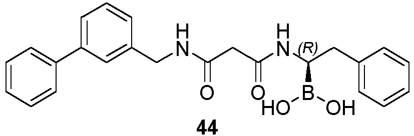

The implementation method was the same as method I, where monoethyl 2-methylmalonate was replaced by the corresponding monoethyl malonate as a starting material, and (6-phenylpyridin-2-yl)methylamine hydrochloride was replaced by [1,1'-biphenyl]-3-ylmethylamine to obtain the title compound.

LC-MS m/z: 399.1 [M-17]⁺.

### Example 45: ((1R)-1-(2-methyl-3-oxo-3-(((6-phenylpyridin-2-yl)methyl)amino)propionamide)-2-(p-tolyl)ethyl )boric acid

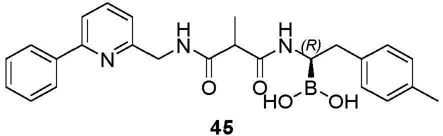

The implementation method was the same as method I, where fragment BB1 was replaced by fragment BB2 to obtain the title compound.

LC-MS m/z: 428.1 [M-17]+.

### Example 46: (R)-(2-(benzofuran-3-yl)-1-(3-((3-methoxybenzyl)amino)-3-oxopropionamido)ethyl)boric acid

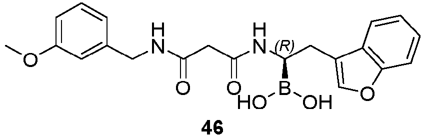

The implementation method was the same as method I, where monoethyl 2-methylmalonate was replaced by the corresponding monoethyl malonate as a starting material, (6-phenylpyridin-2-yl)methylamine hydrochloride was replaced by m-methoxybenzylamine, and fragment BB1 was replaced by fragment BB3 to obtain the title compound.

LC-MS m/z: 393.1 [M-17]⁺.

### Example 47: ((1R)-1-(2-methyl-3-oxo-3-(phenylamino)propionylamino)-2-phenylethyl)boronic acid

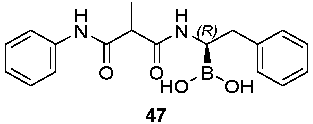

The implementation method was the same as method I, where (6-phenylpyridin-2-yl)methylamine hydrochloride was replaced by the corresponding aniline as a starting material to obtain the title compound.

LC-MS m/z: 323.1 [M-17]+.

1HNMR (400 MHz, DMSO-d6 + D2O) 9.87 (d, J = 9.3 Hz, 1H), 7.70 - 7.39 (m, 3H), 7.31 (td, J = 7.8, 5.9 Hz, 2H), 7.20 - 6.94 (m, 5H), 3.31 (s, 2H), 2.83 (ddd, J = 16.0, 13.7, 5.2 Hz, 1H), 2.78 - 2.65 (m, 1H), 1.39 - 1.12 (m, 3H).

### Example 48: (R)-(1-(3-((3-methoxybenzyl)amino)-3-oxopropionamido)-3-methylbutyl)boric acid

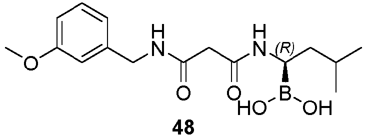

The implementation method was the same as method I, where (6-phenylpyridin-2-yl)methylamine hydrochloride was replaced by the corresponding m-methoxybenzylamine as a starting material, monoethyl 2-methylmalonate was replaced by the corresponding monoethyl malonate, and fragment BB1 was replaced by fragment BB5 to obtain the title compound.

LC-MS m/z: 319.2 [M-17]+.

### Example 49: (R)-(1-(3-((3-methoxybenzyl)amino)-3-oxopropionylamino)-2-phenylethyl)boric acid

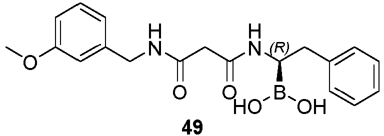

The implementation method was the same as method I, where (6-phenylpyridin-2-yl)methylamine hydrochloride was replaced by the corresponding m-methoxybenzylamine as a starting material, and monoethyl 2-methylmalonate was replaced by monoethyl malonate to obtain the title compound.

LC-MS m/z: 353.2 [M-17]+.

### Example 50: (R)-(1-(3-((3-methoxybenzyl)amino)-3-oxopropionylamino)-2-(p-tolyl)ethyl)boric acid

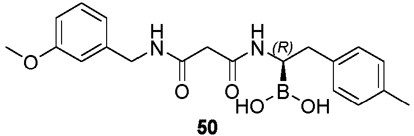

The implementation method was the same as method I, where (6-phenylpyridin-2-yl)methylamine hydrochloride was replaced by the corresponding m-methoxybenzylamine as a starting material, monoethyl 2-methylmalonate was replaced by monoethyl malonate, and fragment BB1 was replaced by fragment BB2 to obtain the title compound.

LC-MS m/z: 367.2 [M-17]⁺.

¹HNMR (400 MHz, DMSO-d6) δ 7.69 (d, *J* = 3.4 Hz, 1H), 7.22 (t, *J* = 7.8 Hz, 1H), 7.02 (d, *J* = 1.8 Hz, 4H), 6.88 - 6.75 (m, 3H), 4.24 (d, *J* = 4.2 Hz, 2H), 3.72 (s, 3H), 3.23 (dd, *J* = 8.2, 5.1 Hz, 1H), 3.16 (d, *J* = 6.4 Hz, 1H), 3.06 (d, *J* = 4.0 Hz, 1H), 2.78 (dd, *J* = 13.7, 5.4 Hz, 1H), 2.65 (dd, *J* = 13.8, 8.2 Hz, 1H), 2.22 (s, 3H).

### Example 51: ((1R)-3-methyl-1-(2-methyl-3-oxo-3-(((tetrahydrofuran-2-yl)methyl)amino)propionamido)butyl) boric acid

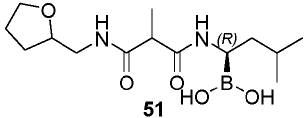

The implementation method was the same as method I, where (6-phenylpyridin-2-yl)methylamine hydrochloride was replaced by the corresponding tetrahydrofurfurylamine as a starting material, and fragment BB1 was replaced by fragment BB5 to obtain the title compound.

LC-MS m/z: 297.2 [M-17]+.

### Example 52: ((1R)-1-(2-methyl-3-oxo-3-(((tetrahydrofuran-2-yl)methyl)amino)propionamido)-2-(p-tolyl)ethyl )boric acid

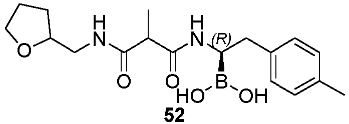

The implementation method was the same as method I, where (6-phenylpyridin-2-yl)methylamine hydrochloride was replaced by the corresponding tetrahydrofurfurylamine as a starting material, and fragment BB1 was replaced by fragment BB2 to obtain the title compound.

LC-MS m/z: 345.2 [M-17]⁺.

### Example 53: ((1R)-1-(3-(cyclohexylamino)-2-methyl-3-oxopropionamido)-2-phenylethyl)boric acid

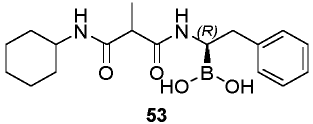

The implementation method was the same as method I, where (6-phenylpyridin-2-yl)methylamine hydrochloride was replaced by the corresponding cyclohexylamine as a starting material to obtain the title compound.

LC-MS m/z: 329.2 [M-17]+.

### Example 54: ((1R)-1-(3-((3-methoxybenzyl)amino)-2-methyl-3-oxopropionamido)-2-phenylethyl)boric acid

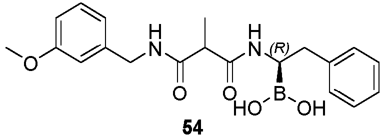

The implementation method was the same as method I, where (6-phenylpyridin-2-yl)methylamine hydrochloride was replaced by the corresponding m-methoxybenzylamine as a starting material to obtain the title compound.

LC-MS m/z: 367.2 [M-17]⁺.

### Example 55: ((1R)-1-(2-fluoro-2-methyl-3-oxo-3-(((6-phenylpyridin-2-yl)methyl)amino)propionylamino)-2-p henylethyl)boric acid

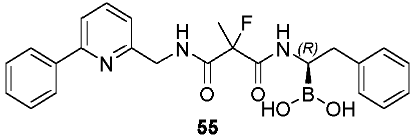

The implementation method was the same as method I, where monoethyl 2-methylmalonate was replaced by the corresponding 3-ethoxy-2-fluoro-2-methyl-3-oxopropanoic acid as a starting material to obtain the title compound.

LC-MS m/z: 432.1 [M-17]+.

In the method, 3-ethoxy-2-fluoro-2-methyl-3-oxopropionic acid was obtained by the implementation method below:

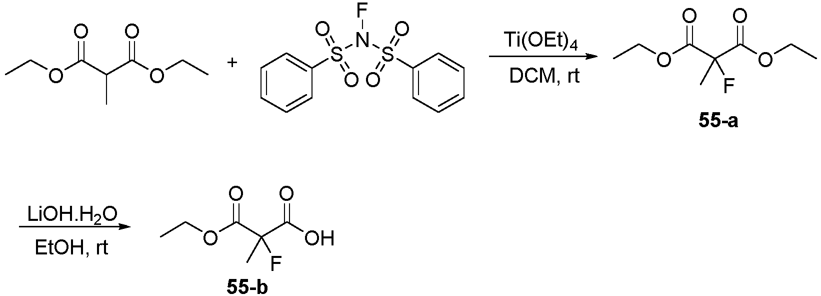

### Implementation steps:

### Step 1: Diethyl 2-fluoro-2-methylmalonate

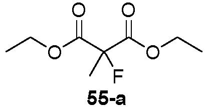

To a solution of diethyl 2-methylmalonate (1.0 g, 5.74 mmol) and N-fluorobenzenesulfonamide (3.63 g, 11.5 mmol) in dichloromethane (10 mL) was added tetraethyl titanate (800 mg, 3.5 mmol) under nitrogen protection. The reaction system was stirred under nitrogen protection at room temperature for 18 h. After completion of reaction, the reaction solution was extracted by the addition of water (60 mL) and dichloromethane (30 ml×3). The organic phases were combined, dried over anhydrous sodium sulfate, and concentrated under reduced pressure. The resulting crude product was separate and purified by preparative thin-layer chromatography (an eluent of 10% ethyl acetate in petroleum ether solution) to obtain a light yellow oil (541 mg, 49%).

¹H NMR (400 MHz, CDCl₃) δ 4.29 (q, *J* = 7.1 Hz, 4H), 1.78 (d, *J* = 22.0 Hz, 3H), 1.30 (t, *J* = 7.1 Hz, 6H).

### Step 2: 3-Ethoxy-2-fluoro-2-methyl-3-oxopropanoic acid

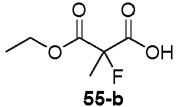

To a solution of the compound diethyl 2-fluoro-2-methylmalonate (540 mg, 2.8 mmol) in ethanol (10 mL), lithium hydroxide monohydrate (124 mg, 2.95 mmol) was added, and the reaction system was stirred and reacted at room temperature for 2 h. After reaction the reaction solution was concentrated under reduced pressure, diluted with water (40 mL) and its pH was adjusted to approximate 3-4 with dilute hydrochloric acid (3 N) followed by extraction with dichloromethane (20 mL×2). The combined organic layers were washed with a saturated aqueous solution of sodium chloride (30 mL), dried over anhydrous sodium sulfate, filtered and concentrated to obtain a compound as a colorless liquid (169 mg, 37%).

LC-MS m/z: 163.1 [M-1]⁻.

### Example 56: ((1R)-1-(2-fluoro-2-methyl-3-oxo-3-(((6-phenylpyridin-2-yl)methyl)amino)propionylamino)-2-(p -tolyl)ethyl)boric acid

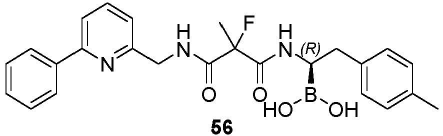

The implementation method was the same as in method I, where monoethyl 2-methylmalonate was replaced by the corresponding 3-ethoxy-2-fluoro-2-methyl-3-oxopropanoic acid as a starting material, and fragment BB1 was replaced by fragment BB2 to obtain the title compound.

LC-MS m/z: 446.1 [M-17]+.

### Example 57: ((1R)-2-(benzofuran-3-yl)-1-(2-methyl-3-oxo-3-(((6-phenylpyridin-2-yl)methyl)amino)propiona mido)ethyl)boric acid

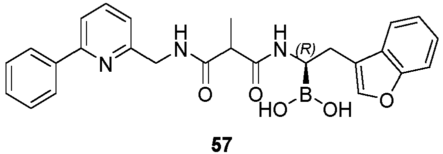

The implementation method was the same as in method I, where (6-phenylpyridin-2-yl)methanamine hydrochloride was replaced by the corresponding (6-phenylpyridin-2-yl)methanamine hydrochloride as a starting material, and fragment BB1 was replaced by fragment BB3 to obtain the title compound.

LC-MS m/z: 454.2 [M-17]+.

### Example 58: ((1R)-2-([1,1'-biphenyl]-4-yl)-1-(2-methyl-3-oxo-3-((((6-phenylpyridin-2-yl)methyl)amino)propionamido)ethyl)boric acid

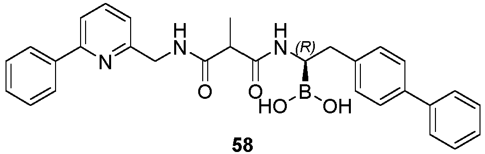

The implementation method was the same as in method I, where (6-phenylpyridin-2-yl)methanamine hydrochloride was replaced by the corresponding (6-phenylpyridin-2-yl)methanamine hydrochloride as a starting material, and fragment BB1 was replaced by fragment BB4 to obtain the title compound.

LC-MS m/z: 490.2 [M-17]⁺.

### Example 59: ((1R)-1-(3-((2,4-dichlorobenzyl)amino)-2-methyl-3-oxopropionamido)-2-(p-tolyl)ethyl)boric acid

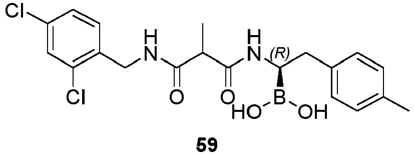

The implementation method was the same as in method I, where (6-phenylpyridin-2-yl)methanamine hydrochloride was replaced by the corresponding 2,4-dichlorobenzylamine hydrochloride as a starting material, and fragment BB1 was replaced by fragment BB2 to obtain the title compound.

LC-MS m/z: 419.1 [M-17]+.

### Example 60: ((1R)-2-(benzofuran-3-yl)-1-(3-((2,4-dichlorobenzyl)amino)-2-methyl-3-oxopropionamido)ethyl) boric acid

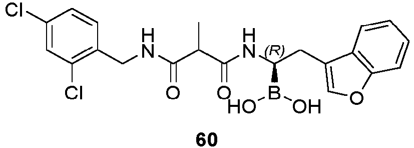

The implementation method was the same as in method I, where (6-phenylpyridin-2-yl)methanamine hydrochloride was replaced by the corresponding 2,4-dichlorobenzylamine hydrochloride as a starting material, and fragment BB1 was replaced by fragment BB3 to obtain the title compound.

LC-MS m/z: 445.0 [M-17]+.

### Example 61: ((1R)-1-(3-((3-methoxyphenylethyl)amino)-2-methyl-3-oxopropionamido)-2-phenylethyl)boric acid

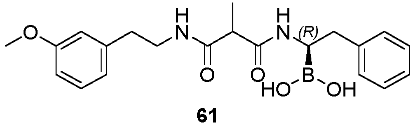

The implementation method was the same as in method I, where (6-phenylpyridin-2-yl)methanamine hydrochloride was replaced by the corresponding 2-(3-methoxyphenyl)ethan-1-amine as a starting material to obtain the title compound.

LC-MS m/z: 381.1 [M-17]+.

### Example 62: ((1R)-1-(3-((3-methoxyphenylethyl)amino)-2-methyl-3-oxopropionamido)-2-(p-tolyl)ethyl)boric acid

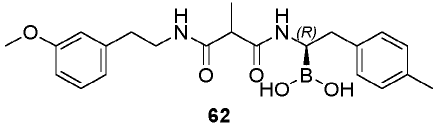

The implementation method was the same as in method I, where (6-phenylpyridin-2-yl)methanamine hydrochloride was replaced by the corresponding 2-(3-methoxyphenyl)ethan-1-amine as a starting material, and fragment BB1 was replaced by fragment BB2 to obtain the title compound.

LC-MS m/z: 395.2 [M-17]⁺.

### Example 63: ((1R)-2-(benzofuran-3-yl)-1-(3-((3-methoxyphenylethyl)amino)-2-methyl-3-oxopropionamido)et hyl)boric acid

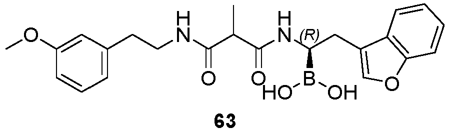

The implementation method was the same as in method I, where (6-phenylpyridin-2-yl)methanamine hydrochloride was replaced by the corresponding 2-(3-methoxyphenyl)ethan-1-amine as a starting material, and fragment BB1 was replaced by fragment BB3 to obtain the title compound.

LC-MS m/z: 421.1 [M-17]+.

### Example 64: ((1R)-2-([1,1'-biphenyl]-4-yl)-1-(3-((3-methoxyphenylethyl)amino)-2-methyl-3-oxopropionamid o)ethyl)boric acid

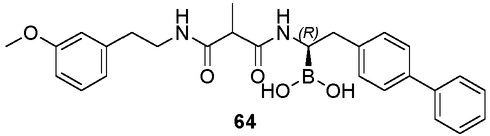

The implementation method was the same as in method I, where (6-phenylpyridin-2-yl)methanamine hydrochloride was replaced by the corresponding 2-(3-methoxyphenyl)ethan-1-amine as a starting material, and fragment BB1 was replaced by fragment BB4 to obtain the title compound.

LC-MS m/z: 457.2 [M-17]⁺.

### Example 65: ((1R)-1-(2-benzyl-3-((3-methoxybenzyl)amino)-3-oxopropionamido)-2-(p-tolyl)ethyl)boric acid

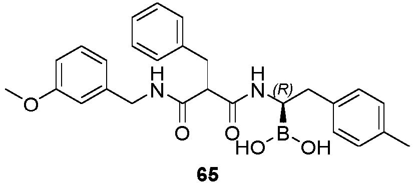

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-benzylmalonate, and fragment BB1 was replaced by fragment BB2 to obtain the title compound.

LC-MS m/z: 457.2 [M-17]+

### Example 66: ((1R)-1-(2-benzyl-3-((3-methoxybenzyl)amino)-3-oxopropionamido)-3-methylbutyl)boronic acid

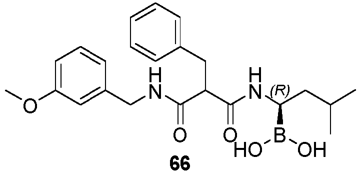

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-benzylmalonate, and fragment BB1 was replaced by fragment BB6 to obtain the title compound.

LC-MS m/z: 409.2 [M-17]⁺.

### Example 67: ((1R)-1-(3-((3-methoxyphenyl)amino)-2-methyl-3-oxopropionamido)-2-phenylethyl)boric acid

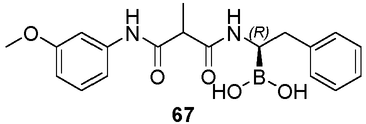

The implementation method was the same as in method I, where (6-phenylpyridin-2-yl)methanamine hydrochloride was replaced by the corresponding 4-methoxyaniline as a starting material to obtain the title compound.

LC-MS m/z: 353.1 [M-17]+.

### Example 68: ((1R)-1-(3-((3-methoxyphenyl)amino)-2-methyl-3-oxopropionamido)-2-(p-tolyl)ethyl)boric acid

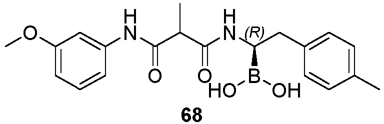

The implementation method was the same as in method I, where (6-phenylpyridin-2-yl)methanamine hydrochloride was replaced by the corresponding 3-methoxyaniline as a starting material, and fragment BB1 was replaced by fragment BB2 to obtain the title compound.

LC-MS m/z: 367.1 [M-17]⁺.

### Example 69: ((1R)-1-(3-((4-methoxyphenyl)amino)-2-methyl-3-oxopropionamido)-2-phenylethyl)boric acid

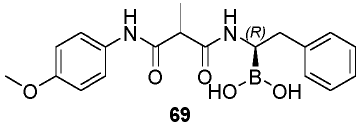

The implementation method was the same as in method I, where (6-phenylpyridin-2-yl)methanamine hydrochloride was replaced by the corresponding 4-methoxyaniline as a starting material to obtain the title compound.

LC-MS m/z: 353.1 [M-17]+.

### Example 70: ((1R)-1-(3-((4-methoxyphenyl)amino)-2-methyl-3-oxopropionamido)-2-(p-tolyl)ethyl)boric acid

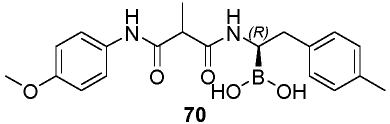

The implementation method was the same as in method I, where (6-phenylpyridin-2-yl)methanamine hydrochloride was replaced by the corresponding 4-methoxyaniline as a starting material, and fragment BB1 was replaced by fragment BB2 to obtain the title compound.

LC-MS m/z: 367.1 [M-17]⁺.

### Example 71: ((1R)-2-(benzofuran-3-yl)-1-(2-benzyl-3-((3-methoxybenzyl)amino)-3-oxopropionamido)ethyl)b oric acid

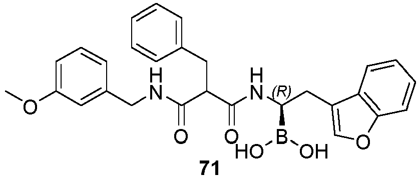

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-benzylmalonate, and fragment BB1 was replaced by fragment BB3 to obtain the title compound.

LC-MS m/z: 483.2 [M-17]+.

### Example 72: ((1R)-2-([1,1'-biphenyl]-4-yl)-1-(2-benzyl-3-((3-methoxybenzyl)amino)-3-oxopropionamido)ethy l)boric acid

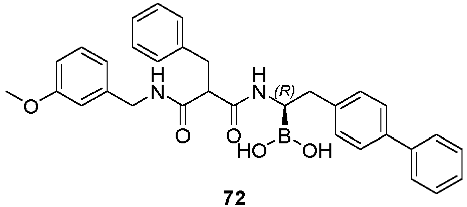

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-benzylmalonate, and fragment BB1 was replaced by fragment BB4 to obtain the title compound.

LC-MS m/z: 519.2 [M-17]⁺.

### Example 73: ((1R)-2-(benzofuran-3-yl)-1-(2-methyl-3-oxo-3-((3-(trifluoromethoxy)benzyl)amino)propionami do)ethyl)boric acid

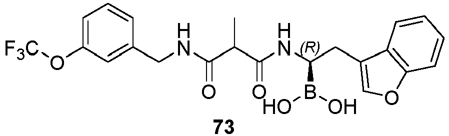

The implementation method was the same as in method I, where (6-phenylpyridin-2-yl)methanamine hydrochloride was replaced by the corresponding 3-trifluoromethoxybenzylamine hydrochloride as a starting material, and fragment BB1 was replaced by fragment BB3 to obtain the title compound.

LC-MS m/z: 461.1 [M-17]+.

### Example 74: ((1R)-2-([1,1'-biphenyl]-4-yl)-1-(2-methyl-3-oxo-3-((3-(trifluoromethoxy)benzyl)amino)propana mido)ethyl)boronic acid

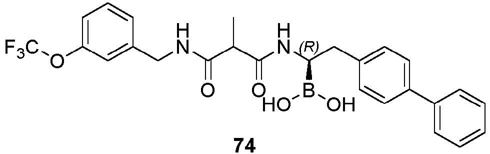

The implementation method was the same as in method I, where (6-phenylpyridin-2-yl)methanamine hydrochloride was replaced by the corresponding 3-trifluoromethoxybenzylamine hydrochloride as a starting material, and fragment BB1 was replaced by fragment BB4 to obtain the title compound.

LC-MS m/z: 497.1 [M-17]⁺.

### Example 75: ((1R)-2-(benzofuran-3-yl)-1-(2-((3-methoxybenzyl)carbamoyl)-3-methylbutanamido)ethyl)boric acid

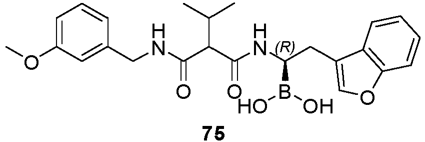

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-isopropylmalonate, and fragment BB1 was replaced by fragment BB3 to obtain the title compound.

LC-MS m/z: 435.2 [M-17]+.

### Example 76: ((1R)-2-([1,1'-biphenyl]-4-yl)-1-(2-((3-methoxybenzyl)carbamoyl)-3-methylbutanamido)ethyl)bo ric acid

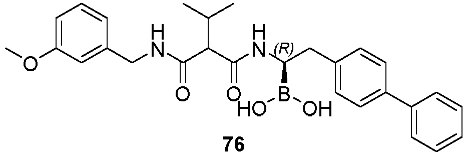

The implementation method was the same as in method I, where (6-phenylpyridin-2-yl)methanamine hydrochloride was replaced by the corresponding 3-methoxybenzylamine as a starting material, monoethyl 2-methylmalonate was replaced by the corresponding monoethyl 2-isopropylmalonate, and fragment BB1 was replaced by fragment BB4 to obtain the title compound.

LC-MS m/z: 471.2 [M-17]⁺.

### Example 77: ((1R)-2-(benzofuran-3-yl)-1-(2-methyl-3-oxo-3-((3-(trifluoromethyl)benzyl)amino)propionamido )ethyl)boric acid

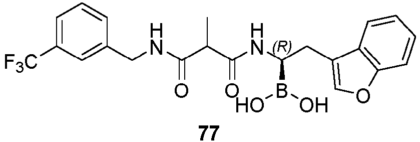

The implementation method was the same as in method I, where (6-phenylpyridin-2-yl)methanamine hydrochloride was replaced by the corresponding 3-trifluoromethylbenzylamine hydrochloride as a starting material, and fragment BB1 was replaced by fragment BB3 to obtain the title compound.

LC-MS m/z: 445.1 [M-17]+.

### Example 78: ((1R)-2-([1,1'-biphenyl]-4-yl)-1-(2-methyl-3-oxo-3-((3-(trifluoromethyl)benzyl)amino)propiona mido)ethyl)boric acid

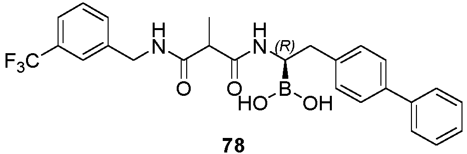

The implementation method was the same as in method I, where (6-phenylpyridin-2-yl)methanamine hydrochloride was replaced by the corresponding 3-trifluoromethylbenzylamine hydrochloride as a starting material, and fragment BB1 was replaced by fragment BB4 to obtain the title compound.

LC-MS m/z: 481.1 [M-17]+.

### Example 79: (R)-(1-(2,2-dimethyl-3-oxo-3-(phenylamino)propionamido)-2-phenylethyl)boric acid

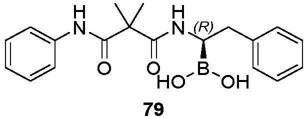

The implementation method was the same as in method I, where (6-phenylpyridin-2-yl)methanamine hydrochloride was replaced by the corresponding aniline as a starting material, and monoethyl methylmalonate was replaced by the monoethyl dimethylmalonate to obtain the title compound.

LC-MS m/z: 337.0 [M-17]⁺.

¹H NMR (400 MHz, DMSO-d6) δ 9.43 (s, 1H), 8.52 (s, 1H), 7.57 (d, *J* = 7.8 Hz, 2H), 7.27 (t, *J* = 7.9 Hz, 2H), 7.15 - 7.03 (m, 6H), 2.80 (dd, *J =* 12.9, 6.3 Hz, 1H), 2.73 (d, *J =* 2.9 Hz, 1H), 2.62 (dd, *J* = 12.9, 6.2 Hz, 1H), 1.38 (d, *J* = 8.7 Hz, 6H).

### Example 80: ((1R)-1-(2-(cyclopropylmethyl)-3-((3-methoxybenzyl)amino)-3-oxopropionamido)-2-(p-tolyl)eth yl)boric acid

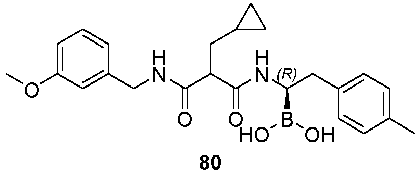

The implementation method was the same as in method III, where fragment BB1 was replaced by fragment BB2 to obtain the title compound.

LC-MS m/z: 421.2 [M-17]+.

### Example 81: ((1R)-2-(benzofuran-3-yl)-1-(2-(cyclopropylmethyl)-3-((3-methoxybenzyl)amino)-3-oxopropiona mido)ethyl)boric acid

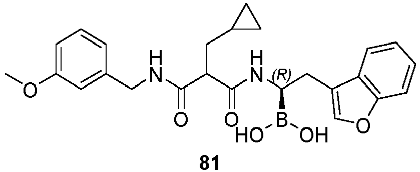

The implementation method was the same as in method III, where fragment BB1 was replaced by fragment BB3 to obtain the title compound.

LC-MS m/z: 447.2 [M-17]⁺.

### Example 82: ((1R)-1-(2-(cyclopropylmethyl)-3-((3-methoxybenzyl)amino)-3-oxopropionamido)-3-methylbuty l)boric acid

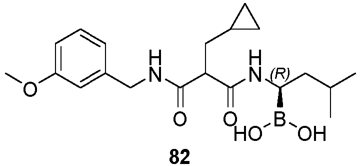

The implementation method was the same as in method III, where fragment BB1 was replaced by fragment BB6 to obtain the title compound.

LC-MS m/z: 373.2 [M-17]+.

### Example 83: ((1R)-1-(2-(cyclohexylmethyl)-3-((3-methoxybenzyl)amino)-3-oxopropionamido)-2-(p-tolyl)ethy l)boric acid

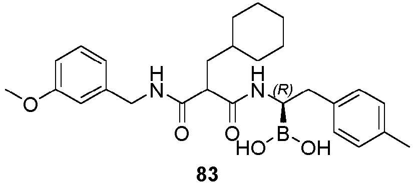

The implementation method was the same as in method III, where bromomethylcyclopropane was replaced by the corresponding bromomethylcyclohexane as a starting material, and fragment BB1 was replaced by fragment BB2 to obtain the title compound.

LC-MS m/z: 463.2 [M-17]⁺.

### Example 84: ((1R)-2-(benzofuran-3-yl)-1-(2-(cyclohexylmethyl)-3-((3-methoxybenzyl)amino)-3-oxopropiona mido)ethyl)boroic acid

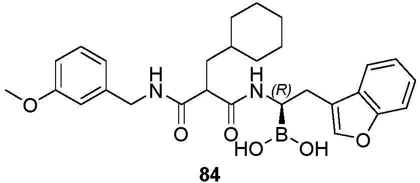

The implementation method was the same as in method III, where bromomethylcyclopropane was replaced by the corresponding bromomethylcyclohexane as a starting material, and fragment BB1 was replaced by fragment BB3 to obtain the title compound.

LC-MS m/z: 489.1[M-17]+.

### Example 85: ((1R)-1-(2-(cyclohexylmethyl)-3-((3-methoxybenzyl)amino)-3-oxopropionamido)-3-methylbutyl )boric acid

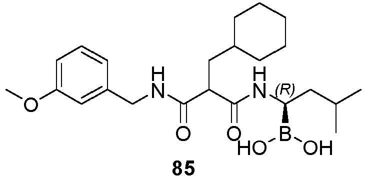

The implementation method was the same as in method III, where bromomethylcyclopropane was replaced by the corresponding bromomethylcyclohexane as a starting material, and fragment BB1 was replaced by fragment BB6 to obtain the title compound.

LC-MS m/z: 415.2[M-17]⁺.

### Example 86: ((1R)-1-(2-methyl-3-oxo-3-((3-(trifluoromethoxy)benzyl)amino)propionamido)-2-(p-tolyl)ethyl)b oric acid

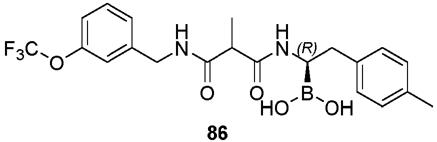

The implementation method was the same as in method I, where (6-phenylpyridin-2-yl)methanamine hydrochloride was replaced by the corresponding 3-trifluoromethoxybenzylamine hydrochloride as a starting material, and fragment BB1 was replaced by fragment BB2 to obtain the title compound.

LC-MS m/z: 435.1 [M-17]+.

### Example 87: ((1R)-1-(2-((3-methoxybenzyl)carbamoyl)-3-methylbutanamido)-2-(p-tolyl)ethyl)boric acid

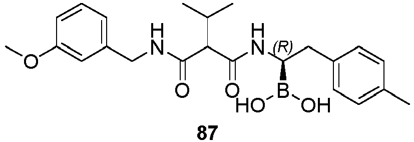

The implementation method was the same as in method III, where bromomethylcyclopropane was replaced by the corresponding 2-iodopropane as a starting material, and fragment BB1 was replaced by fragment BB2 to obtain the title compound.

LC-MS m/z: 409.2 [M-17]+.

### Example 88: ((1R)-1-(2-((2,4-dichlorobenzyl)carbamoyl)-3-methylbutanamido)-2-(p-tolyl)ethyl)boric acid

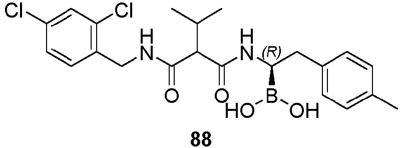

The implementation method was the same as in method III, where 3-methoxybenzylamine was replaced by the corresponding 2,4-dichlorobenzylamine as a starting material, bromomethylcyclopropane was replaced by the corresponding 2-iodopropane as a starting material, and fragment BB1 was replaced by fragment BB2 to obtain the title compound.

LC-MS m/z: 447.1 [M-17]+.

### Example 89: ((1R)-2-(benzofuran-3-yl)-1-(2-((2,4-dichlorobenzyl)carbamoyl)-3-methylbutanamido)ethyl)bori c acid

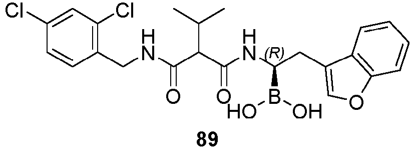

The implementation method was the same as in method III, where 3-methoxybenzylamine was replaced by the corresponding 2,4-dichlorobenzylamine as a starting material, bromomethylcyclopropane was replaced by the corresponding 2-iodopropane as a starting material, and fragment BB1 was replaced by fragment BB3 to obtain the title compound.

LC-MS m/z: 473.1 [M-17]+.

### Example 90: ((1R)-1-(3-(((1H-indol-5-yl)methyl)amino)-2-benzyl-3-oxopropionamido)-2-(p-tolyl)ethyl)boric acid

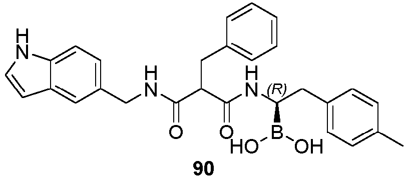

The implementation method was the same as in method I, where (6-phenylpyridin-2-yl)methanamine hydrochloride was replaced by the corresponding 5-(aminomethyl)indole as a starting material, monoethyl 2-methylmalonate was replaced by the corresponding monoethyl 2-benzylmalonate, and fragment BB1 was replaced by fragment BB2 to obtain the title compound.

LC-MS m/z: 466.2 [M-17]+

### Example 91: ((1R)-1-(3-(((1H-indol-5-yl)methyl)amino)-2-benzyl-3-oxopropionamido)-2-(benzofuran-3-yl)et hyl)boric acid

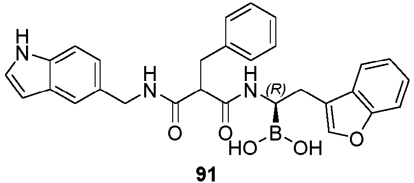

The implementation method was the same as in method I, where (6-phenylpyridin-2-yl)methanamine hydrochloride was replaced by the corresponding 5-(aminomethyl)indole as a starting material, monoethyl 2-methylmalonate was replaced by the corresponding monoethyl 2-benzylmalonate, and fragment BB1 was replaced by fragment BB3 to obtain the title compound.

LC-MS m/z: 492.1 [M-17]+

### Example 92: ((1R)-1-(2-methyl-3-oxo-3-((3-(trifluoromethyl)benzyl)amino)propionamido)-2-(p-tolyl)ethyl)bo ric acid

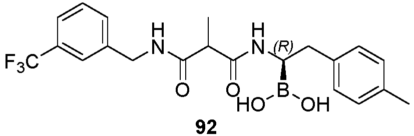

The implementation method was the same as in method I, where (6-phenylpyridin-2-yl)methanamine hydrochloride was replaced by the corresponding 3-trifluoromethylbenzylamine hydrochloride as a starting material, and fragment BB1 was replaced by fragment BB2 to obtain the title compound.

LC-MS m/z: 419.1 [M-17]+.

### Example 93: ((R)-1-((S)-2-fluoro-2-methyl-3-oxo-3-(((6-phenylpyridin-2-yl)methyl)amino)propionamido)-2-( p-tolyl)ethyl)boric acid and ((R)-1-((R)-2-fluoro-2-methyl-3-oxo-3-(((6-phenylpyridin-2-yl)methyl)amino)propionamido)-2-(p-tolyl)ethyl)boric acid

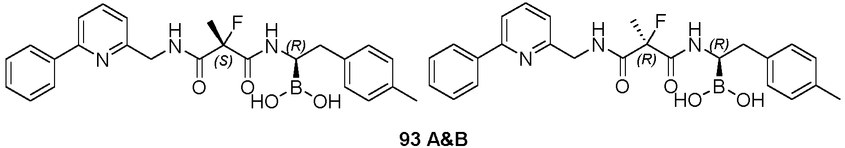

Implementation Method: The racemic starting material ((1*R*)-1-(2-fluoro-2-methyl-3-oxo-3-(((6-phenylpyridin-2-yl)methyl)amino)propionamido)-2-(p-t olyl)ethyl)boric acid (10 mg) prepared in Example 56 was separated by chiral high performance liquid chromatography (HPLC) (analytical column: CHIRALPAK AD-H; mobile phase and gradient: 3% isopropanol in n-hexane (0-20 min), 3%-5% isopropanol in n-hexane (20 min-20.1 min); 5% isopropanol in n-hexane (20.1 min-80 min); 5%-3% isopropanol in n-hexane (80 min-81 min); and 3% isopropanol in n-hexane (81 min-85 min); a flow rate of 2 mL/min) to give the title two fractions: isomer 93A (3.5 mg, 35%, LC-MS m/z: 446.2 [M-17]+) with a retention time of 56.59 min under the above conditions, and isomer 93B (3.9 mg, 39%, LC-MS m/z: 446.2 [M-17]+) with a retention time of 69.85 min under the above conditions.

### Example 94: ((R)-1-((R)-2-methyl-3-oxo-3-((3-(trifluoromethoxy)benzyl)amino)propionamido)-2-(p-tolyl)ethy l)boric acid and ((R)-1-((S)-2-methyl-3-oxo-3-((3-(trifluoromethoxy)benzyl)amino)propionamido)-2-(p-tolyl)ethy l)boric acid

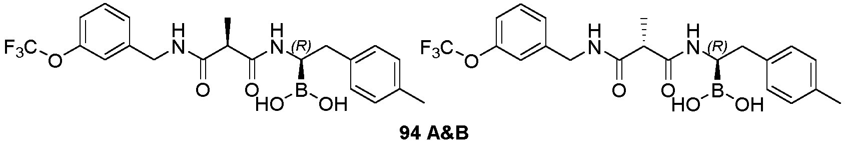

Implementation method: The racemic starting material ((1*R*)-1-(2-methyl-3-oxo-3-((3-(trifluoromethoxy)benzyl)amino)propionamido)-2-(p-tolyl)ethyl)b oric acid (50 mg) prepared in Example 86 was separated by chiral HPLC (analytical column: CHIRALPAK AD-H; mobile phase and gradient: 4% isopropanol in n-hexane (0-50 min), a flow rate of 1.3 mL/min) to obtain the title two fractions: isomer 94A (8.5 mg, 17%, LC-MS m/z: 435.1 [M-17]+) with a retention time of 9.59 min under the above conditions, and isomer 94B (13 mg, 26%, LC-MS m/z: 435.1 [M-17]+) with a retention time of 13.5 min under the above conditions.

### Example 95 (Implementation Method 4): ((1R)-1-(2-fluoro-3-oxo-3-((3-(trifluoromethoxy)benzyl)amino)propionamido)-2-phenylethyl)bor ic acid

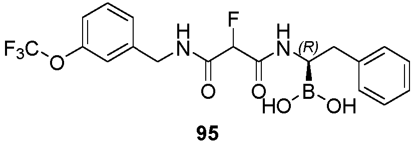

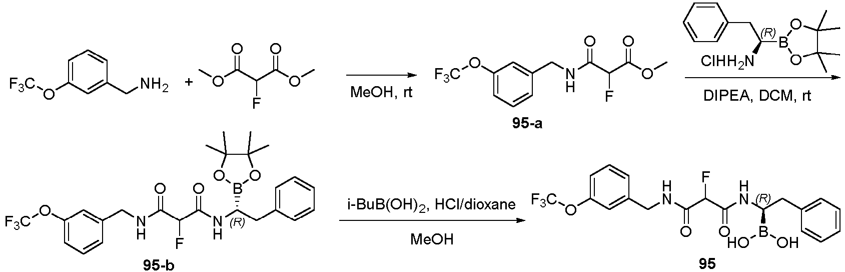

### Implementation method 4: Implementation steps:

### Step 1: Methyl 2-fluoro-3-oxo-3-((3-(trifluoromethoxy)benzyl)amino)propionate

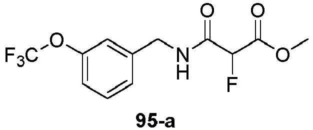

To a solution of dimethyl 2-fluoromalonate (785 mg, 5.23 mmol) in methanol (15 mL) was added 3-trifluoromethoxybenzylamine (1.00 g, 5.23 mmol). The reaction mixture was stirred and reacted at room temperature for 16 h. After completion of reaction, the reaction solution was concentrated under reduced pressure, and the resulting crude product was separated and purified by column chromatography (an eluent of 15% ethyl acetate in petroleum ether) to obtain a colorless liquid (680 mg, 42%).

### LC-MS m/z: 310.0 [M+1]+.

### Step 2: 2-Fluoro-N¹-((R)-2-phenyl-1-(4,4,5,5-tetramethyl-1,3,2-dioxaborolan-2-yl)ethyl)-N³-(3-(trifluoro methoxy)benzyl)malonamide

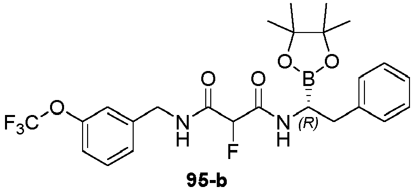

To a solution of methyl 2-fluoro-3-oxo-3-((3-(trifluoromethoxy)benzyl)amino)propanoate (100 mg, 0.32 mmol) and (*R*)-2-phenyl-1-(4,4,5,5-tetramethyl-1,3,2-dioxaborolan-2-yl)ethan-1-amine hydrochloride (91 mg, 0.33 mmol) (fragment BB1) in dichloromethane (15 mL) was added diisopropylethylamine (125 mg, 0.97 mmol). The reaction mixture was stirred at room temperature for 12 h of reaction. After completion of reaction, the reaction solution was diluted with water (20 mL) and extracted with dichloromethane (20 mL×2). The combined organic layers were washed with a saturated aqueous solution of sodium chloride (20 mL), dried over anhydrous sodium sulfate, filtered and concentrated to obtain a brown solid (120 mg, 71%).

LC-MS m/z: 525.1 [M+1]⁺.

### Step 3: ((1R)-1-(2-fluoro-3-oxo-3-((3-(trifluoromethoxy)benzyl)amino)propionamido)-2-phenylethyl)bor ic acid

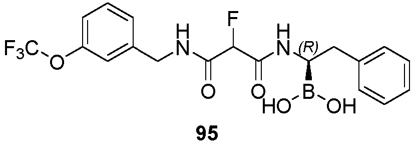

2-Fluoro-*N*¹-((R)-2-phenyl-1-(4,4,5,5-tetramethyl-1,3,2-dioxaborolan-2-yl)ethyl)-*N*³-(3-(trifluoromethoxy)benzyl)propanediamide (120 mg, 0.23 mmol) and isobutyl boric acid (70 mg, 0.69 mmol) were dissolved in methanol (3 mL). This reaction solution was added with a solution of hydrogen chloride in methanol (0.29 mL, 4 M, 1.14 mmol), and stirred at room temperature for 3 h of reaction. After completion of reaction, the reaction solution was washed with petroleum ether (2 mL×2), and the methanol layer was concentrated. The crude product was isolated and purified by preparative thin-layer chromatography (a chromatography eluent of 9% methanol in dichloromethane) to obtain a white solid (55 mg, 54%).

LC-MS m/z: 425.1 [M-17]⁺.

### Example 96: ((1R)-1-(2-fluoro-3-oxo-3-((3-(trifluoromethoxy)benzyl)amino)propionamido)-2-(p-tolyl)ethyl)b oric acid

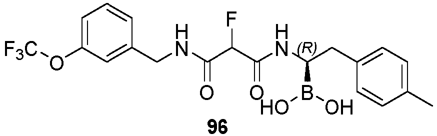

The implement method was the same as method IV, where the fragment BB1 was replaced by the fragment BB2 to obtain the title compound.

LC-MS m/z: 439.1 [M-17]+.

### Example 97: ((1R)-2-(Benzofuran-3-yl)-1-(2-fluoro-3-oxo-3-((3-(trifluoromethoxy)benzyl)amino)propionamid o)ethyl)boric acid

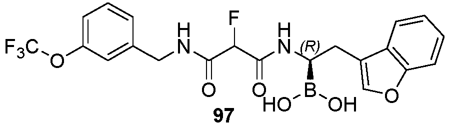

The implement method was the same as method IV, where the fragment BB1 was replaced by the fragment BB3 to obtain the title compound.

LC-MS m/z: 465.0 [M-17]+.

### Example 98: (R)-(1-(2,2-difluoro-3-oxo-3-((3-(trifluoromethoxy)benzyl)amino)propionamido)-2-(p-tolyl)ethyl )boric acid

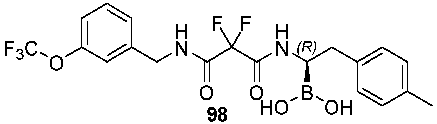

The implement method was the same as method IV, where dimethyl 2-fluoromalonate was replaced by diethyl 2,2-difluoromalonate,and the fragment BB1 was replaced by the fragment BB2 to obtain the title compound.

LC-MS m/z: 457.1 [M-17]⁺.

### Example 99: ((1R)-2-(benzofuran-3-yl)-1-(2-fluoro-3-oxo-3-((3-(trifluoromethoxy)benzyl)amino)propionamid o)ethyl)boric acid

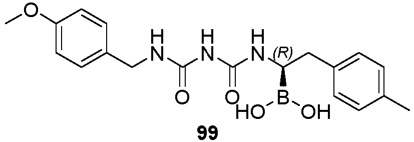

The implement method was the same as that in example 7, where benzylurea was replaced by 1-(4-methoxybenzyl)urea, and the fragment BB1 was replaced by the fragment BB2 to obtain the title compound.

LC-MS m/z: 368.1 [M-17]+.

### Example 100: ((1R)-1-(2-fluoro-3-((3-methoxyphenylethyl)amino)-3-oxopropionamido)-2-(p-tolyl)ethyl)boroni c acid

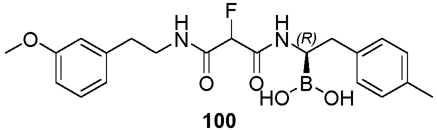

The implement method was the same as method IV, where 3-trifluoromethoxybenzylamine was replaced by 3-methoxyphenylethylamine, and the fragment BB1 was replaced by the fragment BB2 to obtain the title compound.

LC-MS m/z: 399.2 [M-17]+.

### Example 101: ((1R)-2-(Benzofuran-3-yl)-1-(2-fluoro-3-((3-methoxyphenylethyl)amino)-3-oxopropionamido)et hyl)boric acid

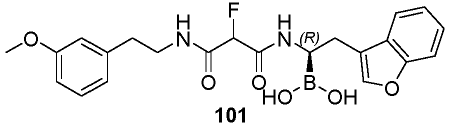

The implement method was the same as method IV, where 3-trifluoromethoxybenzylamine was replaced by 3-methoxyphenylethylamine, and the fragment BB1 was replaced by the fragment BB3 to obtain the title compound.

LC-MS m/z: 425.1 [M-17]+.

### Example 102: ((1R)-1-(2-fluoro-3-((3-methoxybenzyl)amino)-3-oxopropionamido)-2-phenylethyl)boric acid

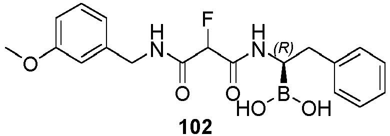

The implement method was the same as method IV, where 3-trifluoromethoxybenzylamine was replaced by 3-methoxybenzylamine to obtain the title compound.

LC-MS m/z: 371.1 [M-17]+.

### Example 103: ((1R)-1-(2-fluoro-3-((3-methoxybenzyl)amino)-3-oxopropionamido)-2-(p-tolyl)ethyl)boric acid

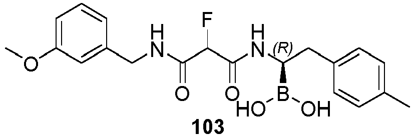

The implement method was the same as method IV, where 3-trifluoromethoxybenzylamine was replaced by 3-methoxybenzylamine, and the fragment BB1 was replaced by the fragment BB2 to obtain the title compound.

LC-MS m/z: 385.1 [M-17]+.

### Example 104: ((1R)-2-(benzofuran-3-yl)-1-(2-fluoro-3-((3-methoxybenzyl)amino)-3-oxopropionamido)ethyl)bo ric acid

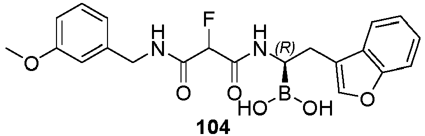

The implement method was the same as method IV, where 3-trifluoromethoxybenzylamine was replaced by 3-methoxybenzylamine, and the fragment BB1 was replaced by the fragment BB3 to obtain the title compound.

LC-MS m/z: 411.1 [M-17]+.

### Part II: Measurement of proteasome inhibitory activity

### Cell Treatment

In the present disclosure, the fluorescent peptide substrate Ac-Ala-Asn-Trp-AMC (abbreviated as Ac-ANW-AMC) and the Trypsin-Like kit containing a fluorescent peptide substrate Suc-Leu-Leu-Val-Tyr-AMC (abbreviated as Suc-LLVY-AMC) (Promega, Item No. G8660) were used to detect the activities of the β5i subunit andβ5c subunit of the 20S proteasome, respectively.

The proteasome tested in the present disclosure was derived from Raji cells, the fluorescent substrate Ac-ANW-AMC was purchased from Roche company, and other reagents were purchased from Sigma company. The experimental system was divided into two parts of cell lysis system and enzyme reaction system. The cell lysis system had a volume of 100 µL, including 7×10⁵ Raji cells and 100 µL of lysate. The enzyme reaction system had a volume of 50 µL, including 25 µL of proteasome, 25 µL of substrate buffer solution. The starting concentration of the compound (inhibitor) was 1 µM. It was diluted in a 3-fold serial dilution to a total of 8 concentrations, that is, the administration concentration can be 1 µM, 0.333 µM, 0.111 µM......, respectively, and the specific experimental process was described below.

### Cell Culture

Raji cells (1×10⁶ cells) cryopreserved in liquid nitrogen were thawed rapidly in a water bath at 37°C. The cell pellet was transferred to a 15 mL centrifuge tube, added with 9 mL of fresh medium (Gibco RPMI1640 added with 10% fetal bovine serum and 1% double antibiotics), centrifuged at 120 g for 5 min, inoculated to 25 mL cell culture flasks, and passaged once every 2-3 days.

### Compound Preparation

The compound was weighed and dissolved in DMSO to a concentration of 10 mM. The compound was then sequentially diluted to concentrations of 200 µM, 66.67 µM, 22.22 µM, 7.41 µM, 2.47 µM, 0.82 µM, 0.27 µM, and 0.091 µM with fresh medium. Raji cells in a good growth status were inoculated into a 48-well cell culture plate at 7×10⁵ cells/mL.

### Treatment of cells

Raji cells in a good growth status were taken and counted. They were then inoculated to a 48-well cell culture plate at 7×10⁵ cells/mL. Each 5 µL of the various concentrations of diluted compounds above were taken and added to the culture plate inoculated with cells, and the lowest concentration of compound was 0 µM. The drug bortezomib (Velcade) and ONX-0914 were used as the positive control compounds. All cells were incubated at 37°C for 4 h.

### Proteasome Isolation

The compound-treated cells were sequentially transferred into 1.5 mL centrifuge tubes, centrifuged at 10000 rpm for 2 min, and the supernatants were removed. The cell pellet was resuspended in 1 mL of PBS, centrifuged at 10000 rpm for 2 min, and the supernatant was removed. Cells were resuspended by adding 100 µL of cell lysate (50 mM Tris of pH 7.5, 150 mM NaCl, 5 mM MgCl₂, 5 mM ATP, 1 mM DTT, 0.01% NP-40, 1 mM Digitonin, and Cocktail), placed in an ice bath for 30 min, and centrifuged at 13000 rpm for 10 min.

### Substrate Preparation

2 mg of fluorescent peptide substrate AC-ANW-AMC was dissolved in 424.7 µL of DMSO to obtain an 8 mM stock solution, and stored at -20°C. The stock solution was diluted 100-fold to a final concentration of 8 µM for use in the reaction system. The fluorescent peptide substrate Suc-LLVY-AMC was prepared according to the instructions of the Promega kit.

### Reaction System Preparation

Each 20 µL of the prepared proteasome solution above was added to a 96-well white luciferase microplate and added with 25 µL of AC-ANW-AM substrate buffer solution (50 mM Tris of pH 7.5, 150 mM NaCl, 5 mM MgCl₂, 5 mM ATP, 80 uM Ac-ANW-AMC and 1 mM DTT) and 25 µL of Suc- LLVY-AMC substrate buffer solution for 10 min of reaction at 37°C in the dark. Fluorescence values were read at 345 nm/445 nm by using a fluorence microplate reader.

### Data Processing

The data were exported and the inhibition rate in percentage was calculated according to the following formula: Inhibition rate= (maximum signal value-compound signal value)/ (maximum signal value - blank signal value) ×100%.

Where the maximum signal was obtained from cell lysates treated with DMSO only, and the blank signal was obtained from wells containing only lysates.

### Measurement of inhibitory activity of cell strain

### Cell Preparation

The cells used in this experiment were MM.1S cell strain and the detection reagent was CellTiter-Glo (Promega). The experimental system had a volume of 190 µL, containing 100 µL of cell suspension, 50 µL of compound, and 40 µL of detection solution. The experimental process was described below. MM.1S cells in the logarithmic growth phase and in a good status were digested into individual cells by trypsin, centrifuged to remove the supernatant, and counted. Cells were diluted and 100 µL of cell suspension per well was added to a 96-well enzyme-coated plate at 1×10⁵ cells per well. The wells containing only RPMI1640 complete medium were set as blank control. The 96-well plate was transferred to a 37°C incubator containing 5% CO₂ for 24 h of incubation.

### Compound Treatment

The compound was accurately weighed and dissolved in DMSO to a concentration of 10⁻² M. 10 µL of compound was pipetted to 190 µL of DMSO to given a 5×10⁻⁴ M compound. 6 µL of the compound (5×10⁻⁵ M) was pipetted to a new 96-well microplate containing 194 µL of RPMI1640 complete medium and diluted in a 3-fold gradient dilution. 50 µL of the diluted compound was transferred to the 96-well microplate containing the cells such that the final compound concentrations were 5×10⁻⁷ M, 1.67×10⁻⁷ M, 5.55×10⁻⁸ M, 1.85×10⁻⁸ M, 6.17×10⁻⁹ M, 2.06×10⁻⁹ M, 6.86×10⁻¹⁰ M, 2.29×10⁻¹⁰ M, and 0 M. For the 0 M, no compound was added, and the same concentration of DMSO was added. The marketed drug bortezomib was used as a positive control. The 96-well microplate added with compounds was placed in the 37°C incubator for 72 h of incubation.

### Cell Assay

At the predetermined detection time, the 96-well microplate to be detected was placed at room temperature, added with 40 µL of detection reagent (CellTiter-Glo) per well, and covered with a dark lid or piece of aluminium foil. The contents were mixed on an orbit oscillator for 10 min for complete cell lysis, with a vibration speed of 150 rpm. Luminescence values were detected on a multifunctional microplate reader (BioTek Synergy H1 Hybrid Multi-Mode Reade).

### Data Processing

The data were exported and the percentage inhibition was calculated according to the following formula: Inhibition rate= (maximum signal value-compound signal value)/ (maximum signal value - blank signal value) ×100%,
wherein maximum signal was obtained from alone DMSO-treated cells, and blank signal was obtained from wells containing only culture medium.

The activities of the compounds in the examples provided by the present disclosure are shown in the table below.

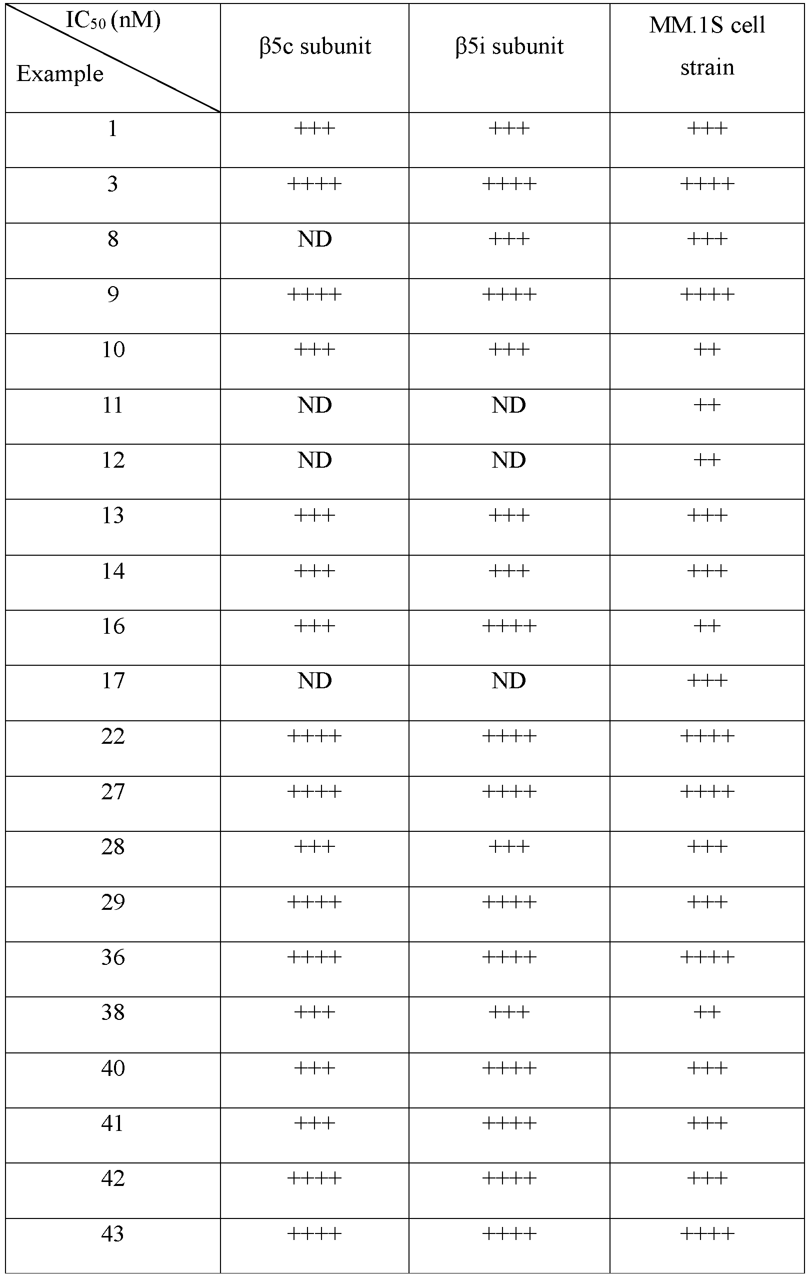

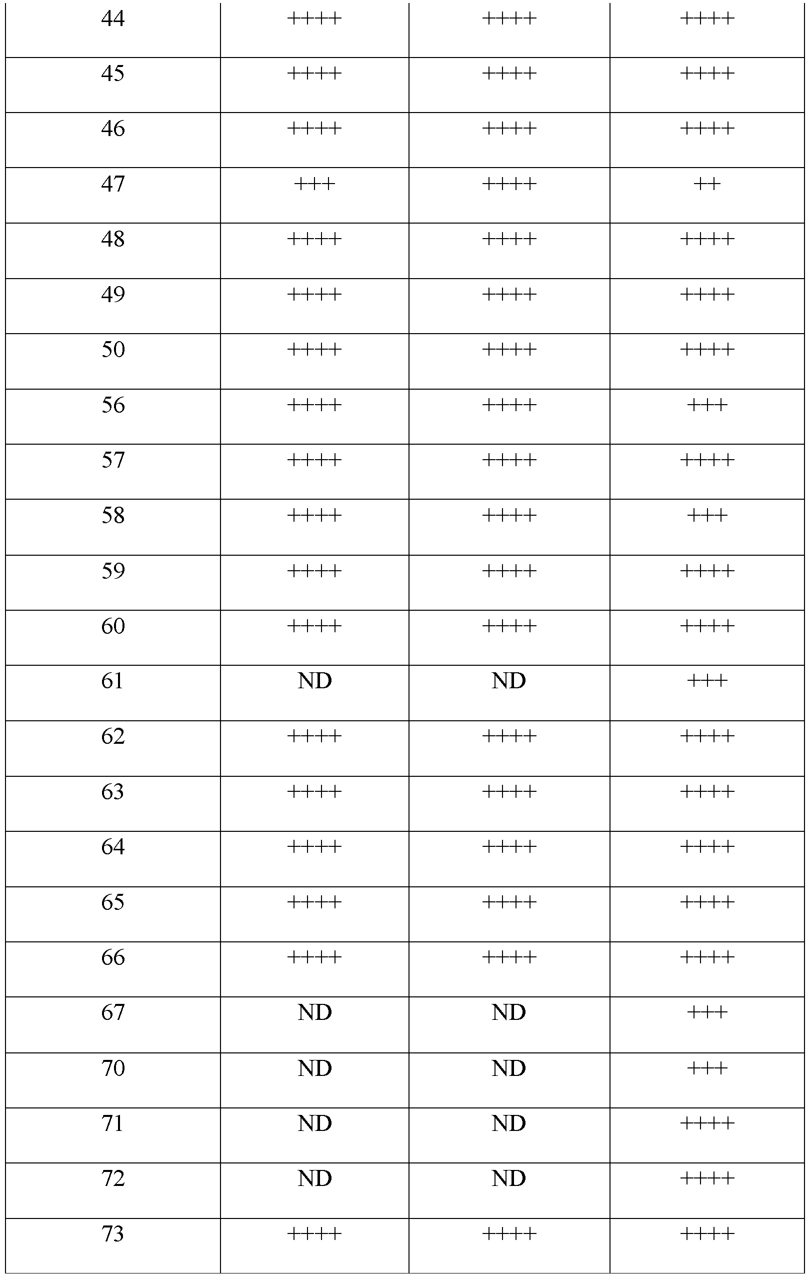

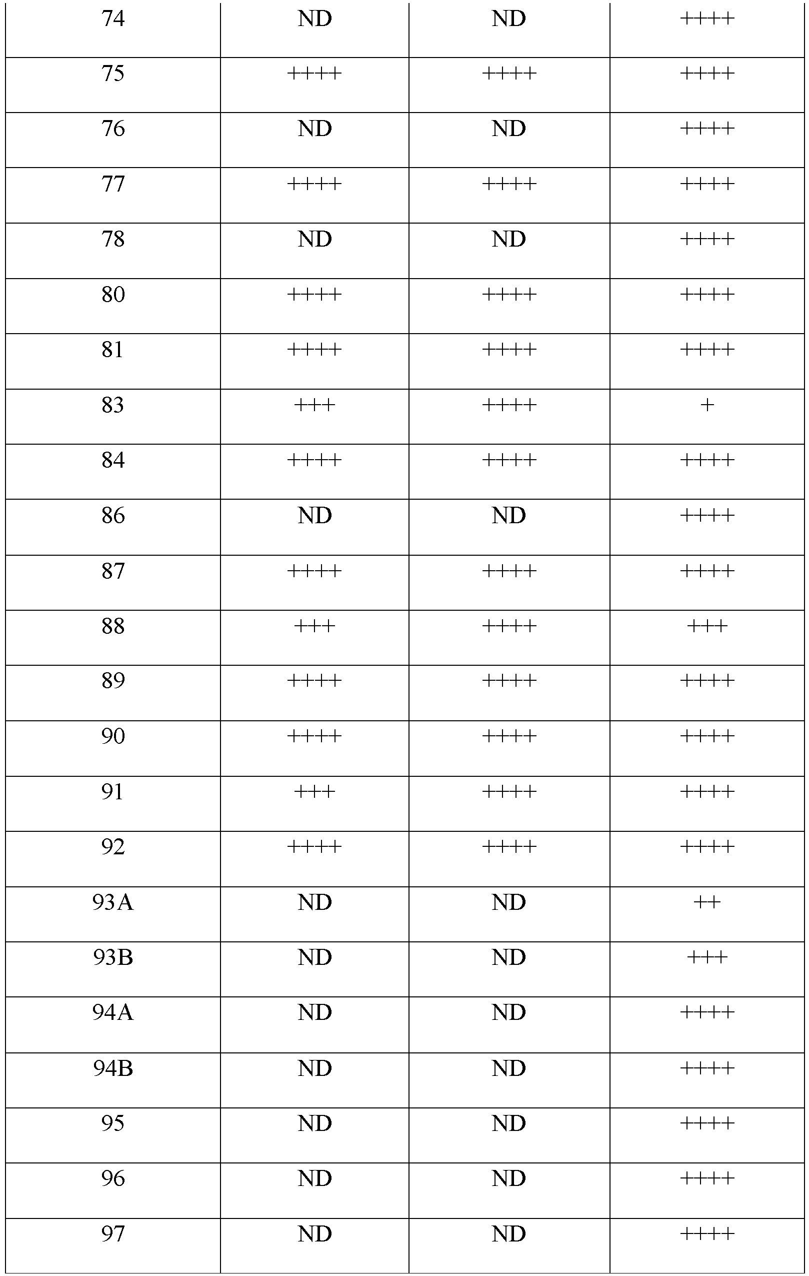

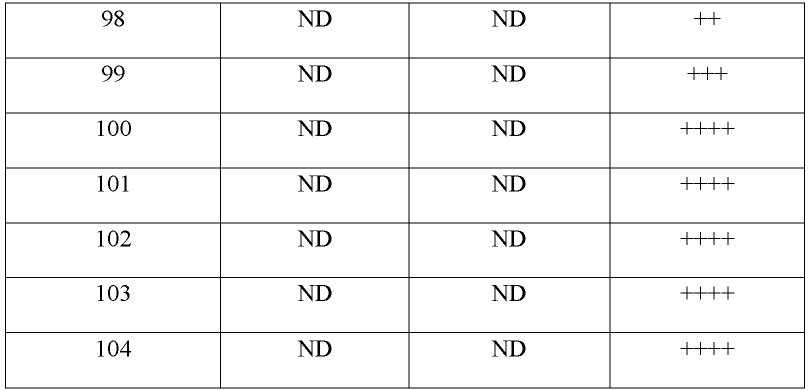

wherein + indicates an IC₅₀ value greater than 500 nM, ++ indicates an IC₅₀ value greater than 200 nM and less than or equal to 500 nM, +++ indicates an IC₅₀ value greater than 50 nM and less than or equal to 200 nM, ++++ indicates an IC₅₀ value less than or equal to 50 nM, and ND represents not determined.

### Part III: Blood concentration measurement of orally-administrated mice

### Implementation Method

The C57BL/6JNifdc mice used in this experiment were provided by Zhejiang Vital River Laboratory Animal Technology Co., Ltd. The compounds were dissolved in DMSO/Solutol/Saline (v/v/v = 10/10/80) as a stock solution (3 mg/mL). The stock solution was administrated orally to 6 C57BL/6JNifdc mice at a dose of 30 mg/kg. Blood was collected from the orbital venous plexus at 0.5 h, 1 h, 1.5 h, and 2 h after oral administration.

### Plasma Processing

Approximately 200 µL of blood samples were collected into EDTAK2 Blood Collection tubes and then immediately centrifuged at 10,000 rpm for 5 min. 66 µL of plasma supernatant was added with198 µL of acetonitrile (Energy), the resulting mixture was shaken on a vortex for 5 min, and then immediately centrifuged at 13000 rpm for 8 min. 200 µL of supernatant was taken for blood compound concentration measurement.

### Standard Sample Preparation

The compound was accurately weighed and dissolved in acetonitrile to a concentration of 100 µg/mL. 50 µL of compound was pipetted and added to 4.95 mL of acetonitrile/water (v/v = 3/1) solution to obtain 1000 ng/mL mother liquid. 650 µL of the mother liquid was taken and sequentially diluted with a solution of acetonitrile/water (v/v = 3/1) in a 2-fold gradient to obtain standard samples with various concentrations of 500 ng/mL, 250 ng/mL, 125 ng/mL, 62.5 ng/mL, and 31.25 ng/mL.

### Sample Concentration Monitoring

Standard solutions with concentrations of 1000 ng/mL, 500 ng/mL, 250 ng/mL, 125 ng/mL, 62.5 ng/mL, and 31.25 ng/mL were measured by using LC-MS (LC-MS 2020 + LC-2030 Plus, Shimadzu) SIM mode to obtain a calibration curve. The treated blood samples were measured by this method and then subjected to quantitative analysis by using the calibration curve to obtain compound concentration data of each treated blood sample.

### Data Processing

The data were exported and the average blood compound concentration at each sampling time was calculated.

Blood compound concentration=compound concentration in treated blood sample×4/molecular weight of the compound. The average blood compound concentration is the average of the compound concentrations in the blood at each sampling time.

The compounds of the present disclosure provided a good blood compound concentration after oral administration, indicating that it can be well absorbed orally:

| Compound | Average blood compound concentration (µM) | | | |
|---|---|---|---|---|
| | 0.5 h | 1.0 h | 1.5 h | 2.0 h |
| Compound in Example 59 | 23 | 20 | 22 | 16 |
| Compound in Example 63 | 23 | 24 | 15 | 16 |
| Bortezomib | 2.0 | 1.3 | 1.0 | 0.9 |

As can be seen from the above results, the compounds of the present disclosure can achieve good blood concentrations and can be used as oral drugs. In this respect, they are significantly superior to the control compound bortezomib.

The present disclosure has been described in detail by way of illustration and example for purposes of clarity and understanding. It will be apparent to those skilled in the art that changes and modifications may be implemented within the scope of the appended claims of the present application. Therefore, it should be understood that the above description is intended to be illustrative and not restrictive. Therefore, the scope of the present disclosure should not be determined by the above description, but is limited by the claims and equivalents of the claims.

## Claims

1. A compound represented by formula (I) or a pharmaceutically acceptable salt thereof: 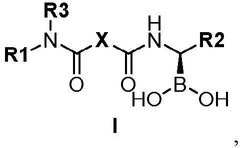 wherein,
X is selected from the group consisting of -C₁₋₃ alkyl-, -C₃₋₆ cycloalkyl-, -vinyl-, -ethynyl-, -NH-, -NH-NH-, and -heterocyclyl-, and X is optionally substituted with a substituent, wherein the substituent is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen;
R1 and R3 are each independently selected from the group consisting of hydrogen, C₁₋₁₀ alkyl, C₃₋₆ cycloalkyl, aryl, heteroaryl, benzyl, heterocyclyl, and bridged ring group, and the C₁₋₁₀ alkyl, C₃₋₆ cycloalkyl, aryl, heteroaryl, benzyl, heterocyclyl, and bridged ring group are optionally substituted with a substituent which is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen; and
R2 is selected from the group consisting of C1-10 alkyl, benzyl, biphenylmethyl and heterocyclyl, and the C1-10 alkyl, benzyl, biphenylmethyl and heterocyclyl are optionally substituted with a substituent which is selected from the group consisting of deuterium, C₁₋₄ alkyl, C₁₋₁₀ alkoxy, C₃₋₆ cycloalkyl, heterocyclyl, aryl, heteroaryl, aryloxy, cyano, hydroxyl, mercapto, amino, and halogen.

2. The compound represented by formula (I) or the pharmaceutically acceptable salt thereof according to claim 1, wherein the compound represented by formula (I) has a structure represented by formula (II): 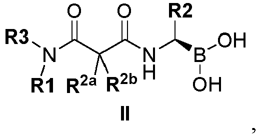
wherein, R1, R2, and R3 have the definitions as in claim 1; and
R^{2a} and R^{2b} are each independently selected from the group consisting of hydrogen, deuterium, C₁₋₁₀ alkyl, benzyl, isopropyl, cyclopropyl methyl, cyclobutyl methyl, cyclopentyl methyl, cyclohexyl methyl, heterocyclyl methyl, and halogen, or R^{2a} and R^{2b}, together with the carbon atom to which they are attached, form a 3- to 6- membered carbon ring.

3. The compound represented by formula (I) or the pharmaceutically acceptable salt thereof according to claim 1, wherein the compound represented by formula (I) has a structure represented by formula (III): 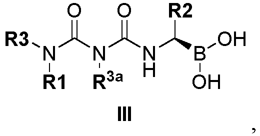
wherein, R1, R2, and R3 have the definitions as in claim 1; and
R^{3a} is selected from the group consisting of hydrogen, C₁₋₁₀ alkyl, benzyl, isopropyl, cyclopropyl methyl, cyclobutyl methyl, cyclopentyl methyl, cyclohexyl methyl, and heterocyclyl methyl.

4. The compound represented by formula (I) or the pharmaceutically acceptable salt thereof according to claim 1, wherein the compound represented by formula (I) has a structure represented by formula (IV): 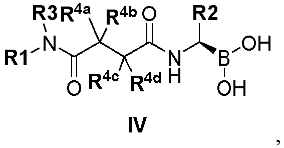
wherein, R1, R2, and R3 have the definitions as in claim 1; and
R^{4a}, R^{4b}, R^{4c} and R^{4d} are each independently selected from the group consisting of hydrogen, deuterium, C₁₋₁₀ alkyl, halogen, hydroxyl, and amino, wherein the amino is optionally substituted with C₁₋₄ alkyl or C₃₋₆ cycloalkyl.

5. The compound represented by formula (I) or the pharmaceutically acceptable salt thereof according to claim 1, wherein the compound represented by formula (I) has a structure represented by formula (V): 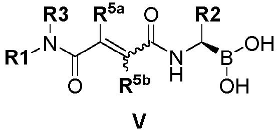
wherein, R1, R2, and R3 have the definitions as in claim 1; and
R^{5a} and R^{5b} are each independently selected from the group consisting of hydrogen, deuterium, C₁₋₄ alkyl, halogen and hydroxyl, and R^{5a} and R^{5b} are in either cis- or trans-substituted form to each other.

6. The compound represented by formula (I) or the pharmaceutically acceptable salt thereof according to claim 1, wherein the compound represented by formula (I) has a structure represented by formula (VI): 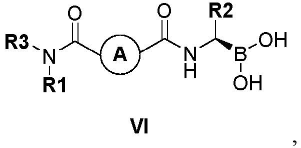
wherein R1, R2, and R3 have the definitions as in claim 1; and
ring A is selected from the group consisting of -C₃₋₆ cycloalkyl- and -heterocyclyl-, wherein ring A is optionally substituted with a substituent which is selected from the group consisting of C₁₋₄ alkyl, C₁₋₁₀ alkoxy, C₃₋₆ cycloalkyl, cyano, hydroxyl and halogen.

7. The compound or the pharmaceutically acceptable salt thereof according to any one of claims 1 to 6, wherein R1 and R3 are each selected from the group consisting of hydrogen,

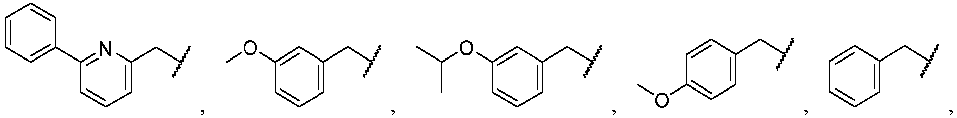

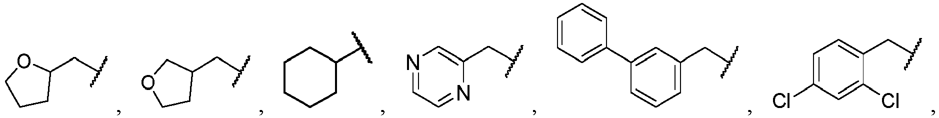

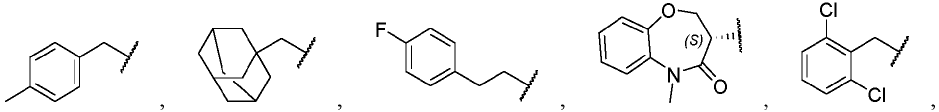

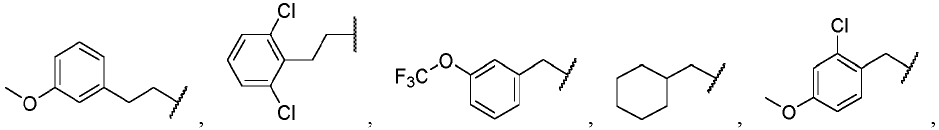

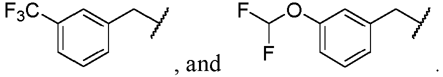

8. The compound or the pharmaceutically acceptable salt thereof according to any one of claims 1 to 7, wherein R2 is selected from the group consisting of:

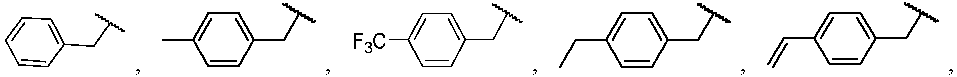

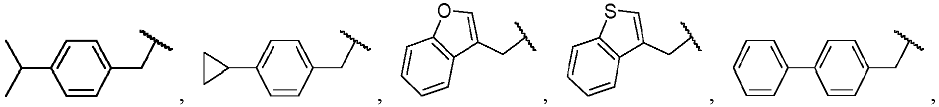

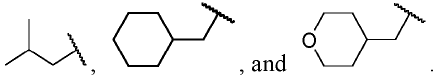

9. The compound or the pharmaceutically acceptable salt thereof according to any one of claims 1 to 8, wherein the compound is selected from the group consisting of:

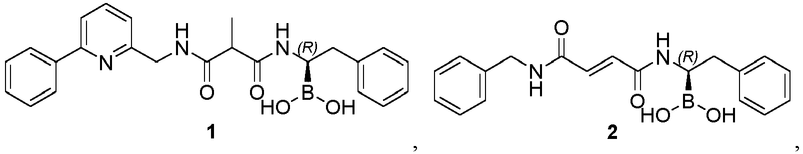

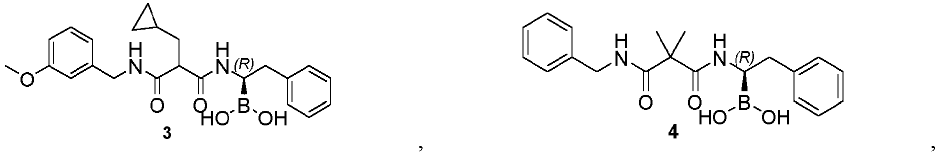

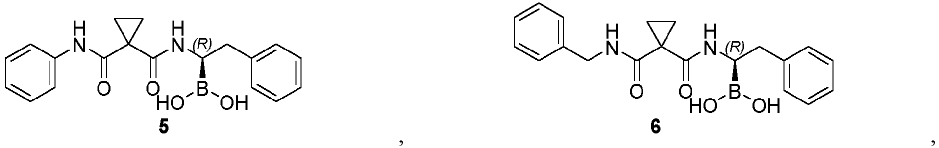

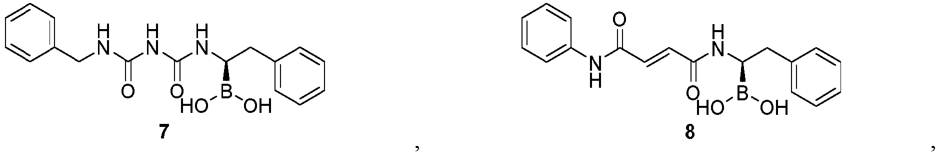

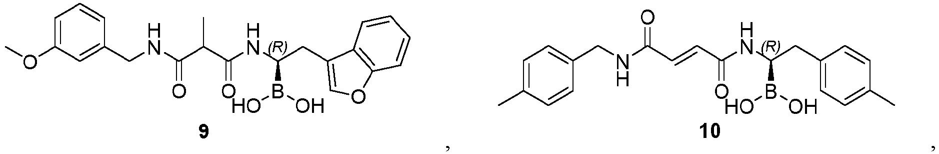

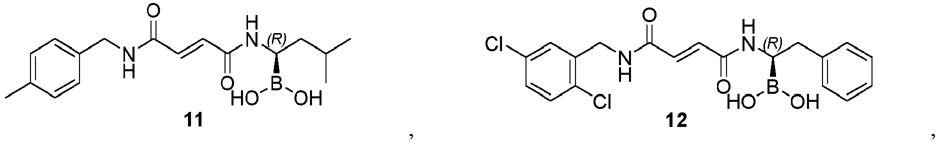

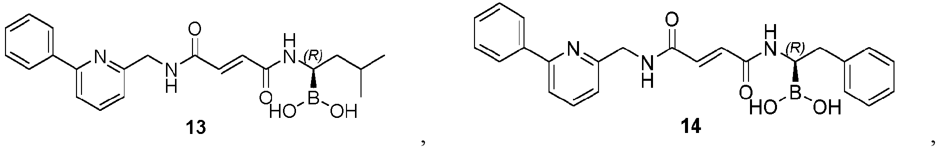

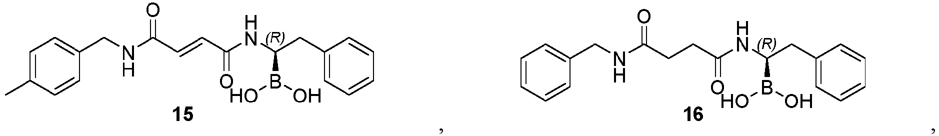

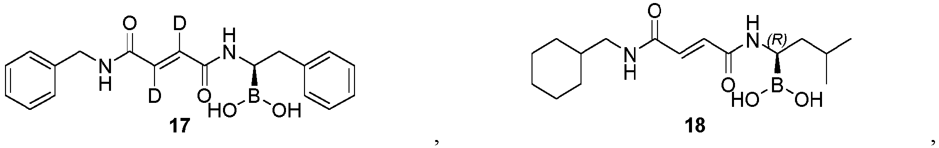

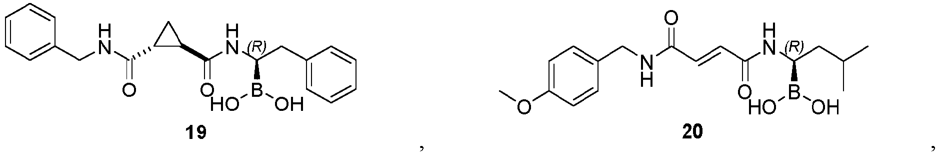

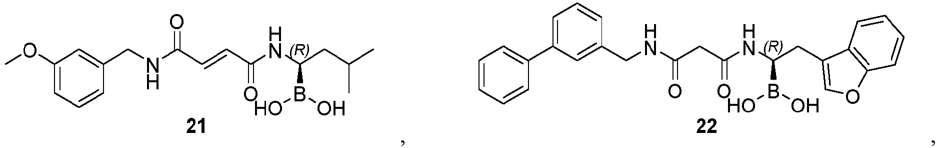

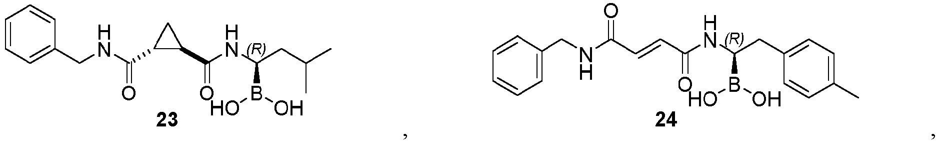

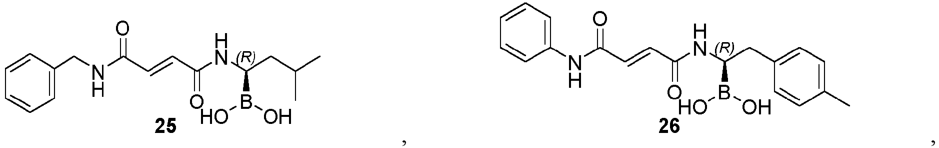

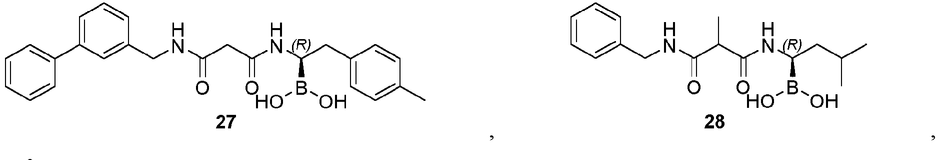

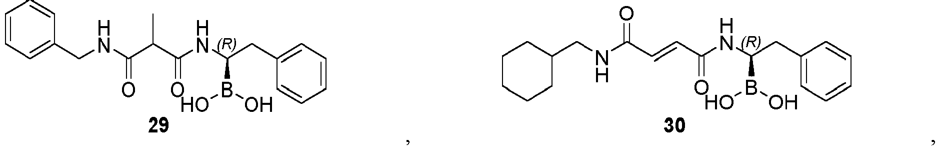

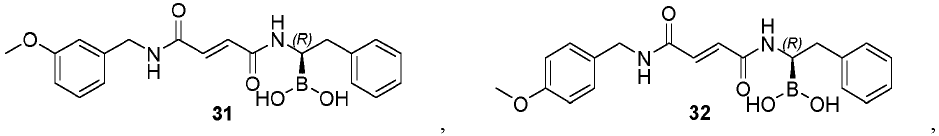

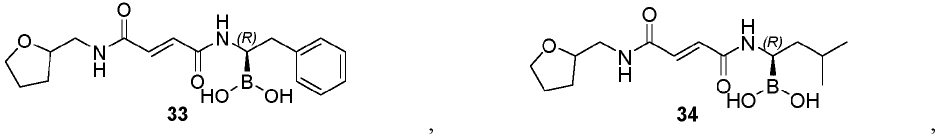

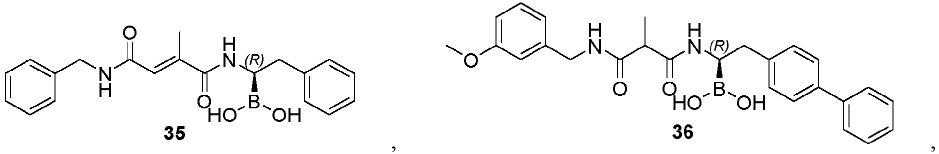

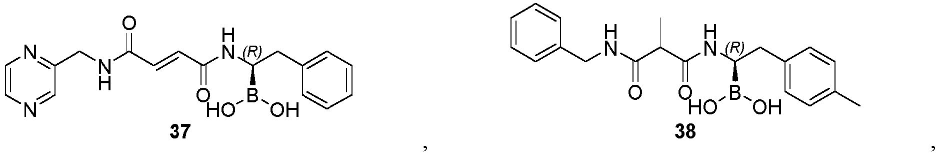

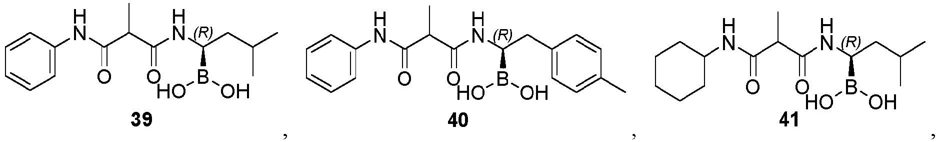

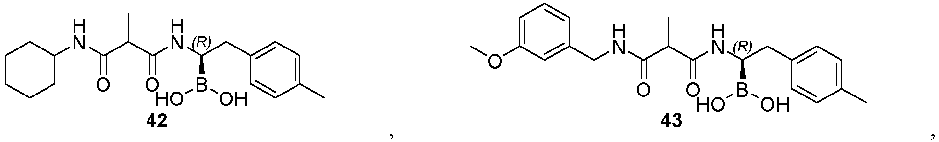

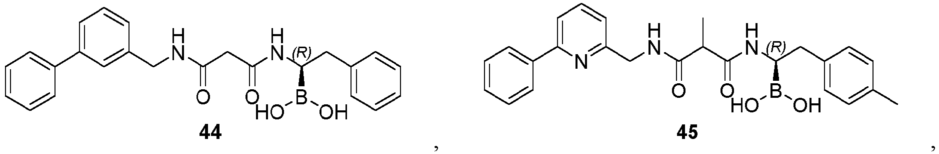

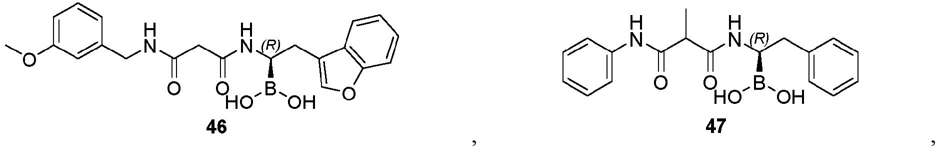

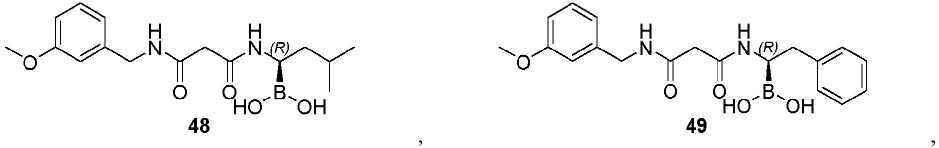

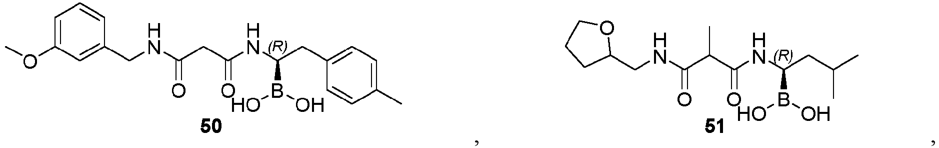

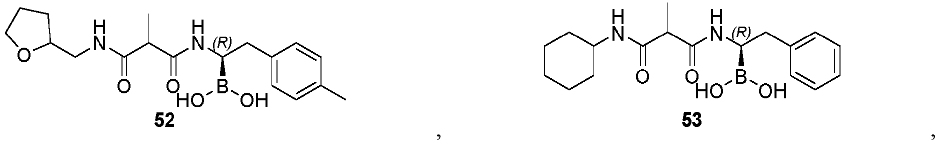

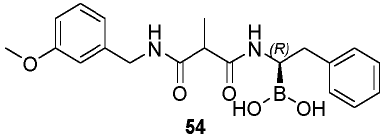

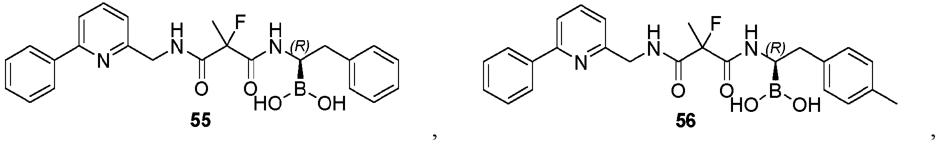

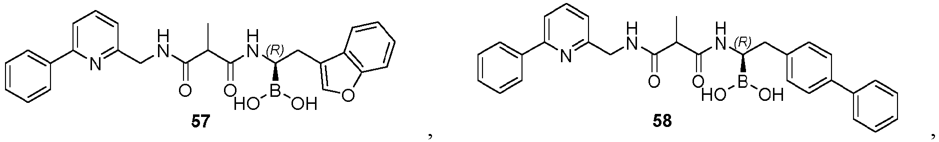

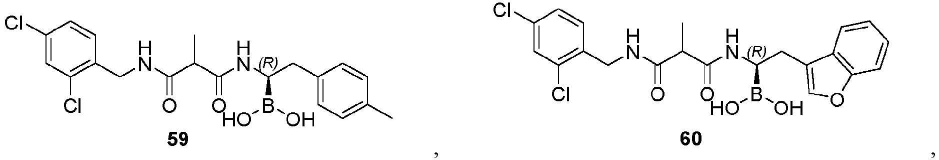

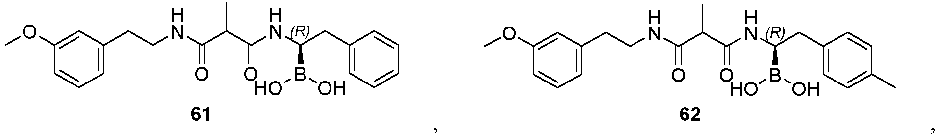

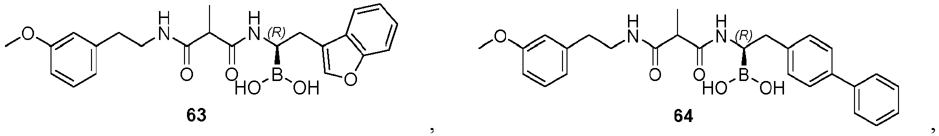

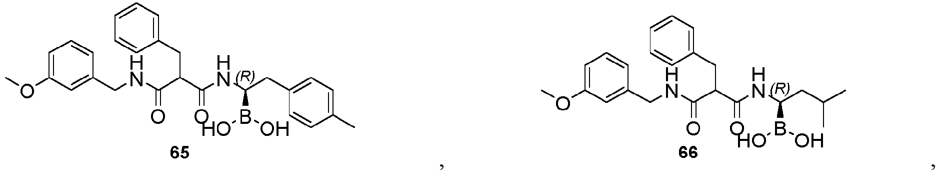

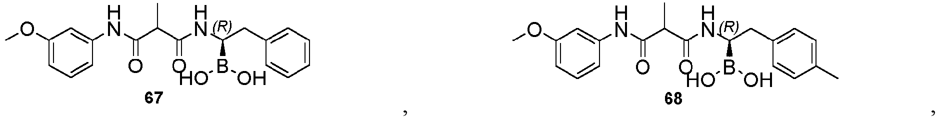

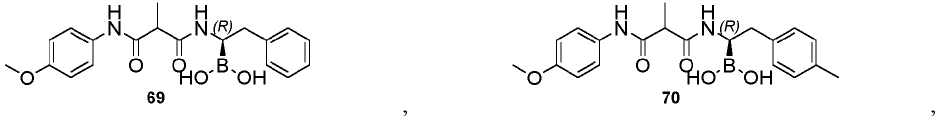

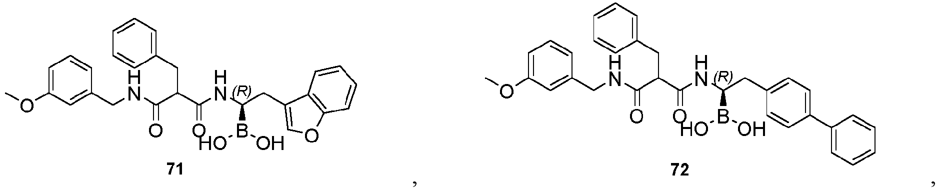

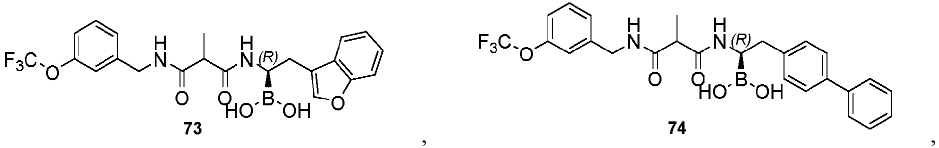

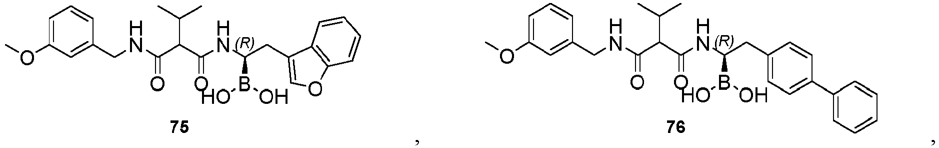

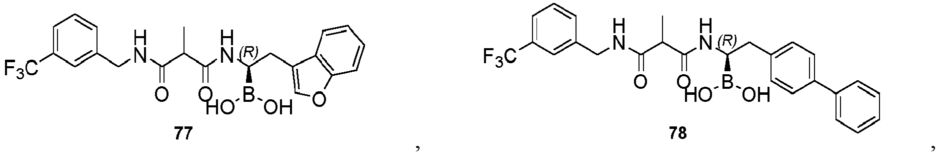

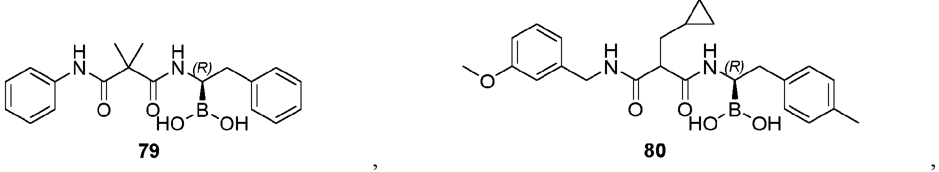

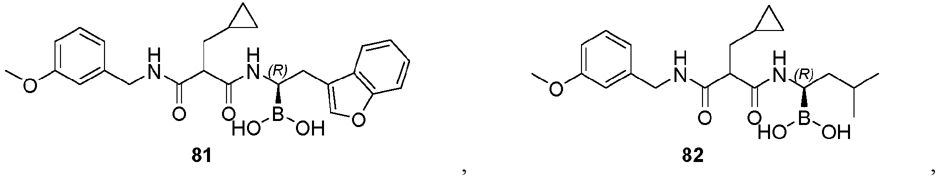

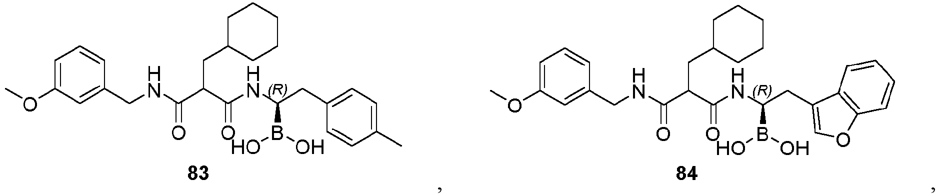

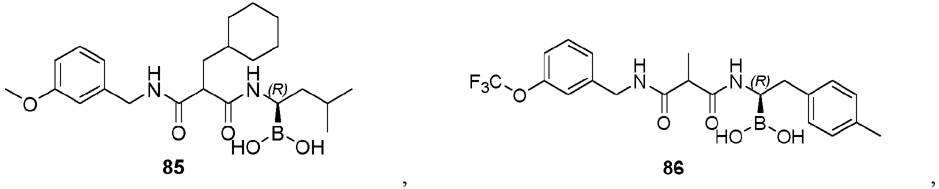

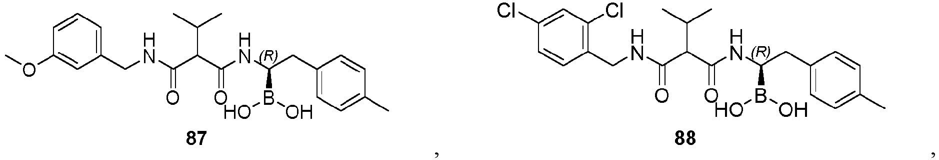

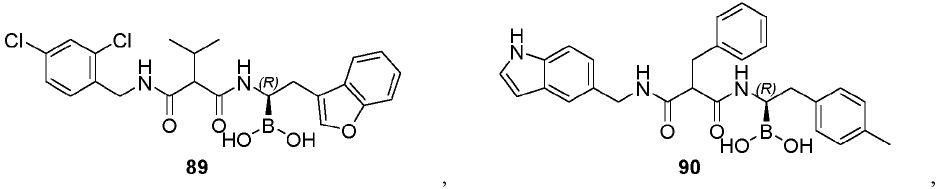

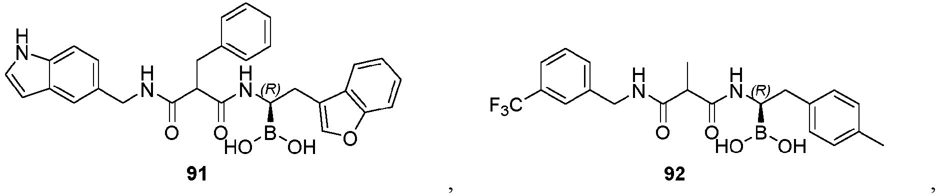

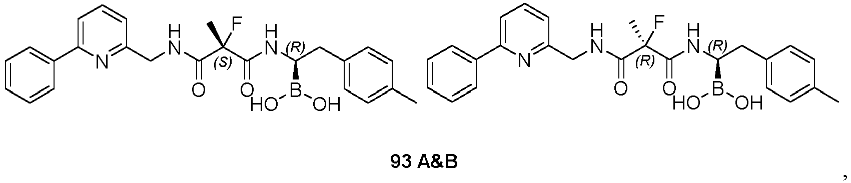

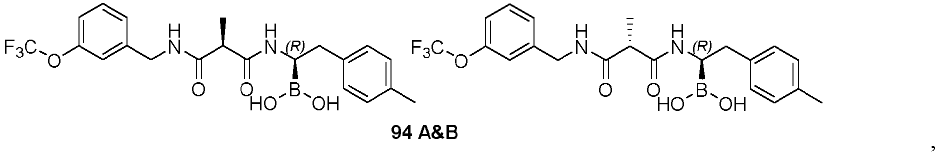

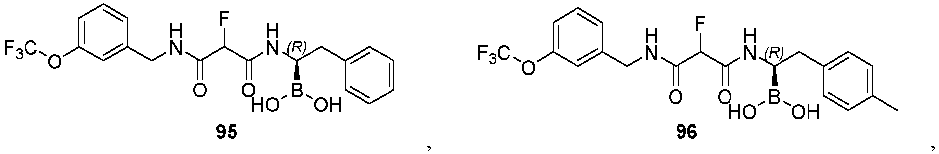

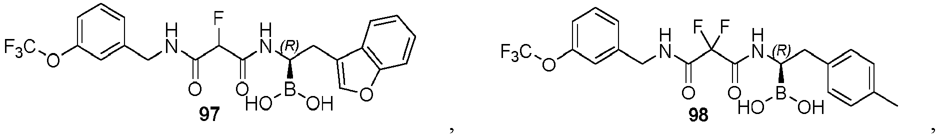

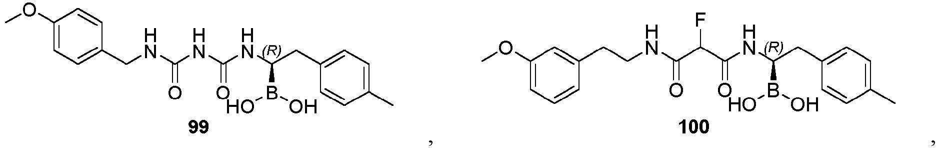

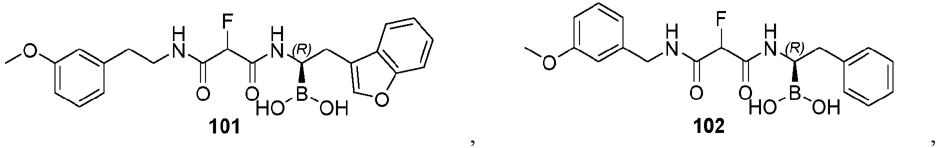

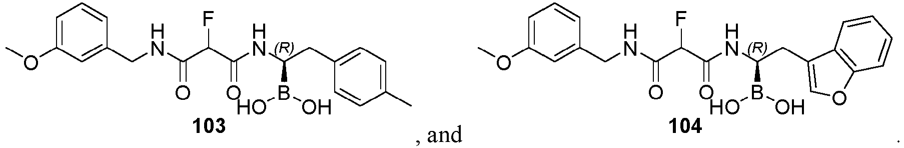

10. A pharmaceutical composition comprising the compound or the pharmaceutically acceptable salt thereof according to any one of claims 1 to 9 and an optional pharmaceutically acceptable carrier.

11. Use of the compound or the pharmaceutically acceptable salt thereof according to any one of claims 1 to 9 in the manufacture of a medicament as a proteasome inhibitor.

12. Use of the compound or the pharmaceutically acceptable salt thereof according to any one of claims 1 to 9 in the manufacture of a medicament for the treatment or prevention of a disease related to proteasome.

13. The use according to claim 12, wherein the disease is selected from the group consisting of a tumor and an autoimmune disease.

14. The use according to claim 12, wherein the disease is multiple myeloma.

15. The use according to claim 12, wherein the disease is selected from the group consisting of systemic lupus erythematosus (SLE) and inflammatory bowel disease (IBD).
